# EUROPEAN PATENT APPLICATION

(11) **EP 4 733 393 A1**
(43) Date of publication of application: **29.04.2026**
(21) Application number: 24825977.2
(22) Date of filing: 20.06.2024
(51) Int. Cl.: C12N 7/01, A61K 35/763, A61K 48/00, A61P 35/00, A61P 37/04, C07K 16/28, C12N 15/13

(54) **IMMUNOSTIMULATORY HERPES VIRUS**

(30) Priority: 20.06.2023 JP 2023101127
(71) Applicant: Todo, Tomoki, Koto-ku Tokyo 135-0034 (JP)
(72) Inventor: Todo, Tomoki, Koto-ku Tokyo 135-0034 (JP)
(74) Representative: Hoffmann Eitle
(86) International application number: PCT/JP2024/022411
(87) International publication number: WO 2024/262576

(57) **Abstract**

An oncolytic herpes simplex virus having a gene encoding an immune checkpoint inhibitor is provided. The herpes simplex virus preferably has the following characteristics (a) to (c): (a) the ICP6 gene is deleted or inactivated, (b) the γ34.5 gene is deleted or inactivated, and (c) the α47 gene is deleted or inactivated. The expressed immune checkpoint inhibitor remains localized to a tumor site and does not leak into bloodstream, thus avoiding systemic side effects.

## Description

### Technical Field

The present invention relates to an oncolytic herpes virus. More specifically, the present invention relates to a function-added oncolytic herpes virus that enhances therapeutic effect of viral therapy.

### Background Art

Researches are advancing on cancer therapies using viruses, which comprise infecting cancer cells with viruses and destroying the cancer cells in conjunction with virus replication to treat cancers. Unlike existing treatment methods, this approach has the characteristic that, in addition to the direct cytolytic effect accompanying viral replication, cancer cell-specific immunity is induced during the process of destroying cancer cells, systemically exerting a therapeutic effect via the immune system.

The inventor of the present invention invented a third-generation oncolytic herpes simplex virus type 1 (HSV-1) G47Δ having triple mutations, conducted clinical development thereof, and successfully achieved practical application thereof (Patent document 1 and Non-patent document 1). The inventor of the present invention is further advancing development of function-added G47Δ variants as next-generation oncolytic HSV-1, which consist of the basic G47Δ backbone incorporated with various exogenous genes, such as a swelling-suppressive oncolytic virus incorporated with a gene encoding the vascular endothelial growth factor (VEGF) antagonist (Patent document 2). The inventor of the present invention also reported on an oncolytic virus incorporated with a gene that expresses interleukin-12 (IL-12) as a fusion polypeptide where the light chain and heavy chain are linked with an elastin motif (Non-patent document 2).

Many cancer cells evade attack of the immune system by utilizing the immunosuppressive function of immune checkpoint molecules. Immune checkpoint inhibitor therapies aim to achieve anticancer effects by blocking the transmission of immunosuppressive signals mediated by immune checkpoint molecules, thereby releasing the suppression of activation of immune cells, including T cells, and increasing the patients' immune activity. As immune checkpoint inhibitors, nivolumab (trade name Opdivo), a PD-1 (Programmed Death-1) inhibitory antibody; atezolizumab (trade name Tecentriq), a PD-L1 (Programmed Death-1 Ligand 1) inhibitory antibody; tremelimumab and ipilimumab, CTLA-4 (Cytotoxic T-lymphocyte-associated antigen 4) inhibitor antibodies etc. have been used for the treatment of specific cancers.

### Prior Art References

### Patent documents

Patent document 1: Japanese Patent No. 4212897
Patent document 2: WO2019/189643

### Non-patent document

Non-patent document 1: Todo, T. et al., Nat Commun 13, 4119 (2022), https://doi.org/10.1038/s41467-022-31262-y105, 841-846
Non-patent document 2: Fukuhara, H. et al., Commun Med (Lond) 3, 40 (2023), https://www.nature.com/articles/s43856-023-00270-4

### Summary of the Invention

### Problem to be solved by the invention

Immune checkpoint inhibitor antibodies can shift the entire immune system into an attack mode, but therapeutic effects are not achieved unless the immune system recognizes the cancer as non-self. Furthermore, it is known that systemic administration of immune checkpoint inhibitor antibodies can cause severe side effects, such as autoimmune diseases, with relatively high frequency.

### Means for solving the problem

The inventor of the present invention lately developed a function-added third-generation oncolytic HSV-1 that expresses an immune checkpoint inhibitor antibody by incorporating a gene encoding an immune checkpoint antibody into the basic backbone of G47Δ. Virus therapies using HSV-1 are characterized by inducing anticancer immunity by causing the immune system to recognize cancer cells as non-self. Therefore, combining HSV-1 with an immune checkpoint inhibitor antibody is expected to significantly enhance therapeutic efficacy.

Furthermore, the oncolytic HSV-1 of the present invention expresses immune checkpoint inhibitor antibodies each time the virus replicates in and infects into a tumor. Consequently, the immune checkpoint inhibitor antibodies are expressed and accumulate only locally within the tumor and this can allow enhanced therapeutic efficacy of the viral therapy without inducing systemic side effects.

The present invention provides the following.
[1] An oncolytic herpes simplex virus having a gene encoding an immune checkpoint inhibitor.
[2] The virus according to 1, wherein the herpes simplex virus has the following characteristics (a) to (c):
   (a) the ICP6 gene is deleted or inactivated,
   (b) the γ34.5 gene is deleted or inactivated, and
   (c) the α47 gene is deleted or inactivated.
[3] The virus according to 1 or 2, wherein the gene encoding an immune checkpoint inhibitor is inserted at a site where the ICP6 gene is deleted or inactivated.
[4] The virus according to any one of 1 to 3, wherein the immune checkpoint inhibitor is an immune checkpoint inhibitor antibody or a fragment thereof.
[5] The virus according to any one of 1 to 4, wherein the immune checkpoint inhibitor is an anti-PD-1 antibody, an anti-PD-L1 antibody, or an anti-CTLA-4 antibody, or a fragment thereof.
[6] A pharmaceutical composition containing the virus according to any one of 1 to 5.
[7] The pharmaceutical composition according to 6, which is for local injection.
[8] The pharmaceutical composition according to 6 or 7, being a formulation designed to suppress blood concentration of the immune checkpoint inhibitor.
[9] A combination of the virus according to any one of 1 to 5 and at least one type of oncolytic herpes simplex virus having a gene encoding an immune checkpoint inhibitor different from that of the foregoing virus.
[10] A combination of the virus according to any one of 1 to 5 and at least one of an oncolytic herpes simplex virus having a gene encoding soluble B7-1 and an oncolytic herpes simplex virus having a gene encoding IL-12.

### Effects of the Invention

The oncolytic herpes simplex virus provided by the present invention, which has a gene encoding an immune checkpoint inhibitor, can express immune checkpoint inhibitor antibodies in conjunction with viral replication, when it is administered to tumor cells.

The oncolytic herpes simplex virus can also provide the following effects.
- The expressed antibodies have the same function as commercially available immune checkpoint inhibitor formulations for systemic administration.
- Administration of a single type of the virus produces the effect of combined use of systemic administration of immune checkpoint inhibitor antibodies and viral therapy.
- Compared with non-expressing viruses, it exhibits enhanced therapeutic effects on both administered tumor and distant non-administered tumors.
- The expressed immune checkpoint inhibitor antibodies remain localized to the tumor site and scarcely leak into the bloodstream, thereby avoiding systemic side effects.
- Administration of a combination of multiple types of viruses expressing different immune checkpoint inhibitor antibodies (administration as a mixture or sequential administration) is expected to further enhance therapeutic efficacy.
- Combination of the virus with a virus with soluble B7-1 expression and/or a virus with IL-12 expression is expected to further enhance the therapeutic efficacy.

### Brief Description of the Drawings

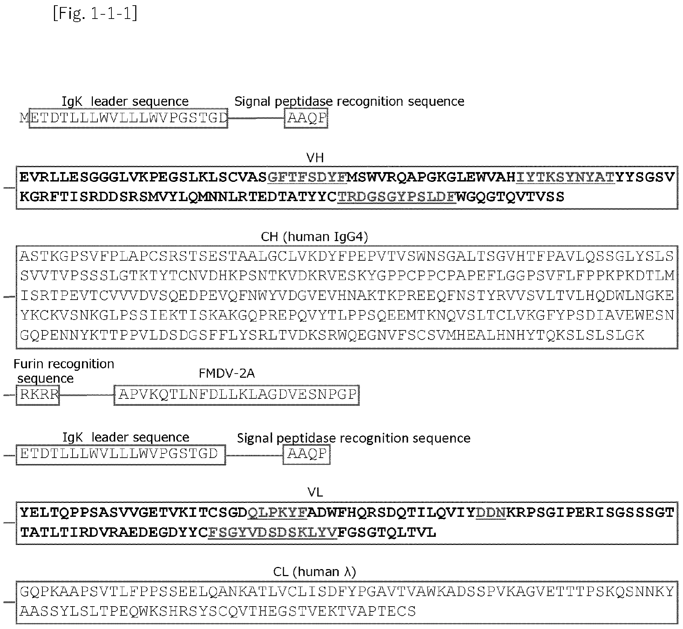

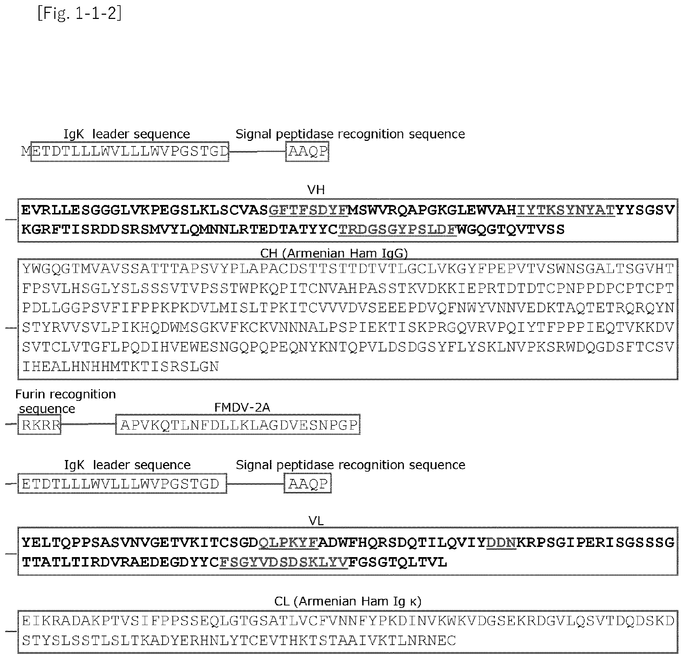

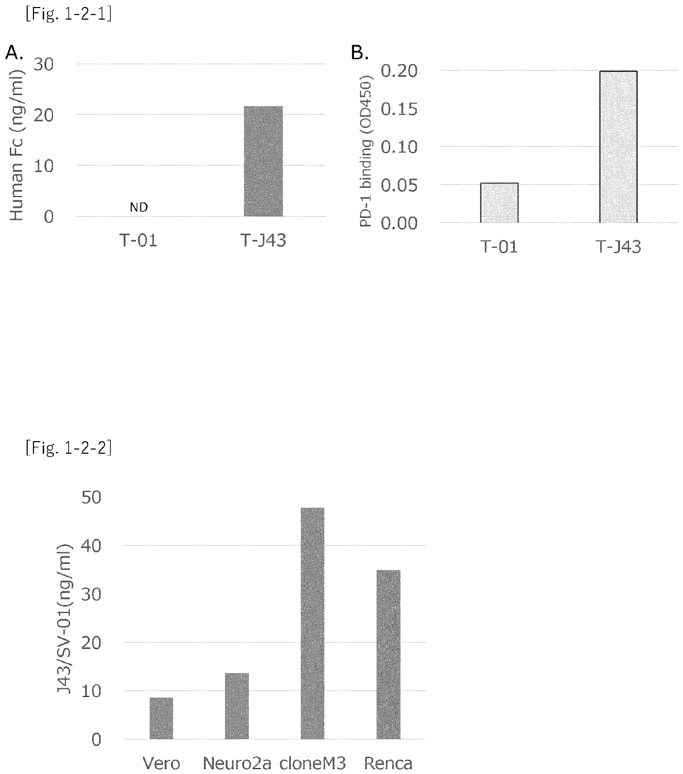

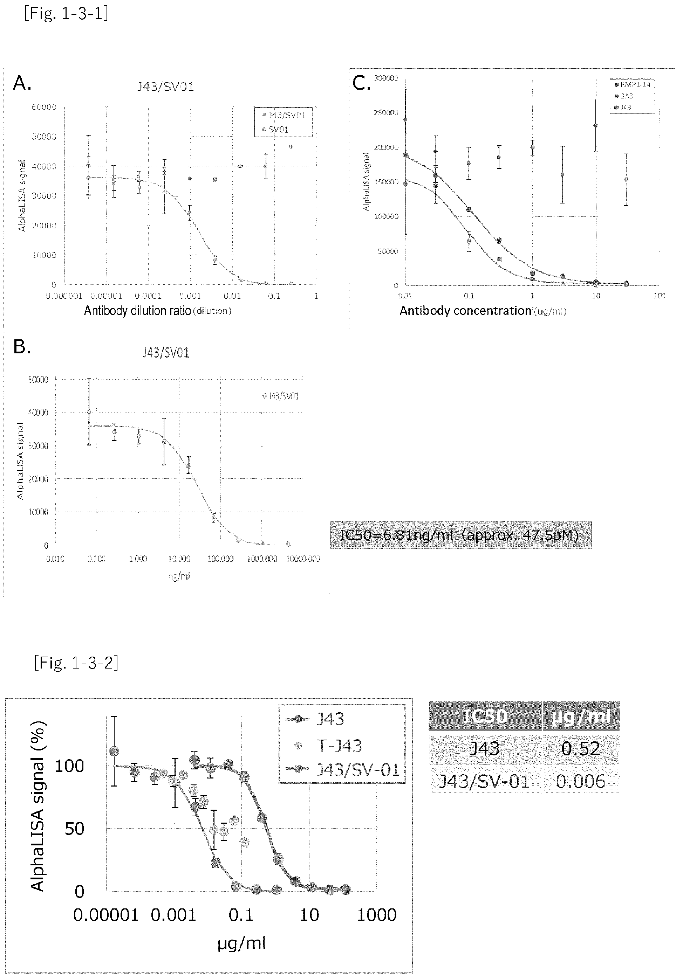

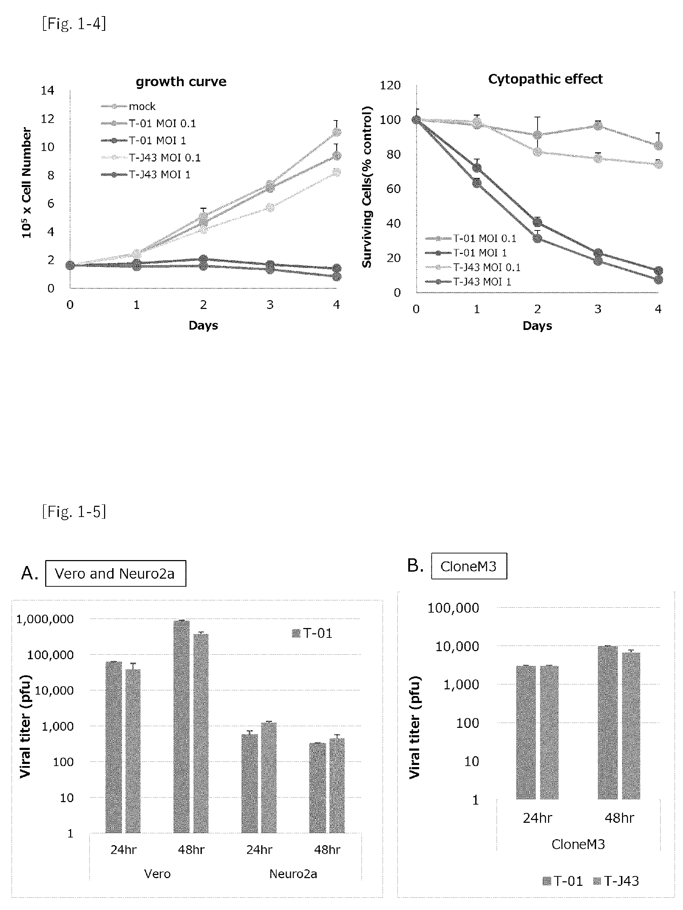

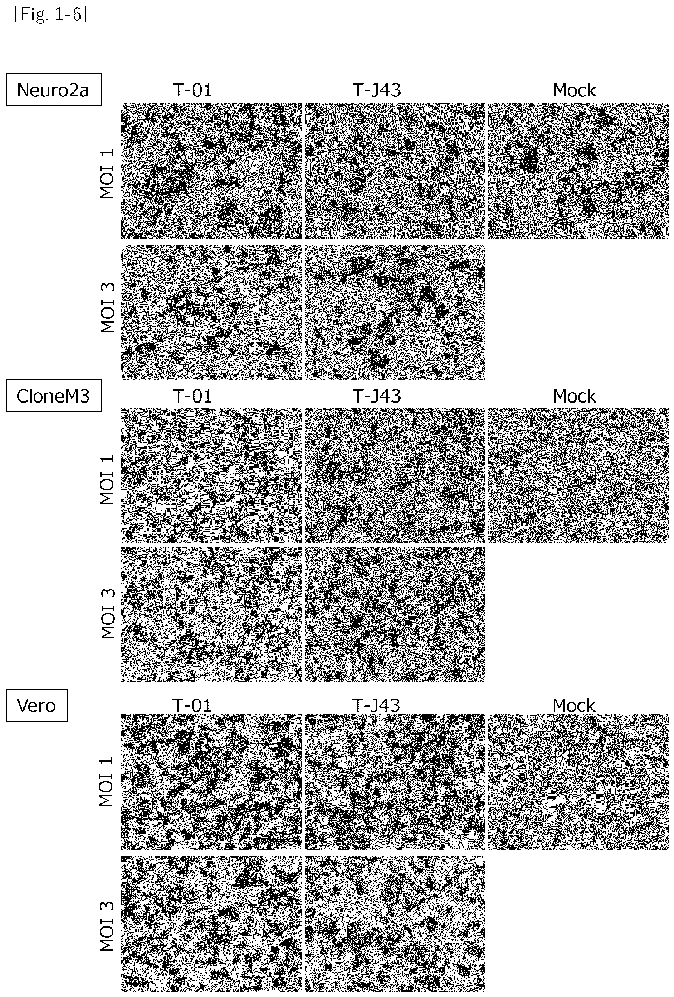

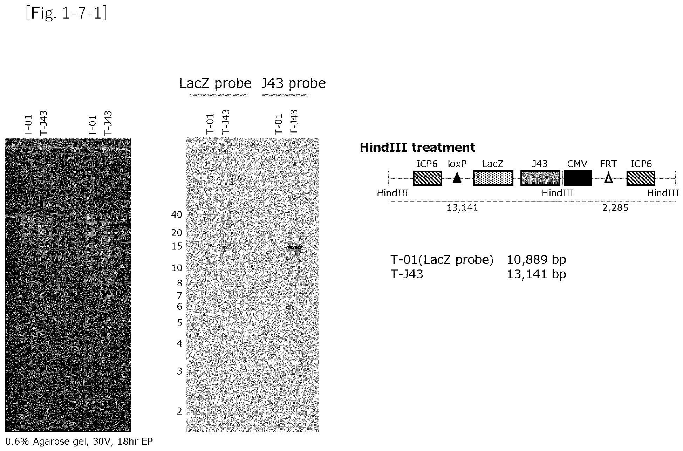

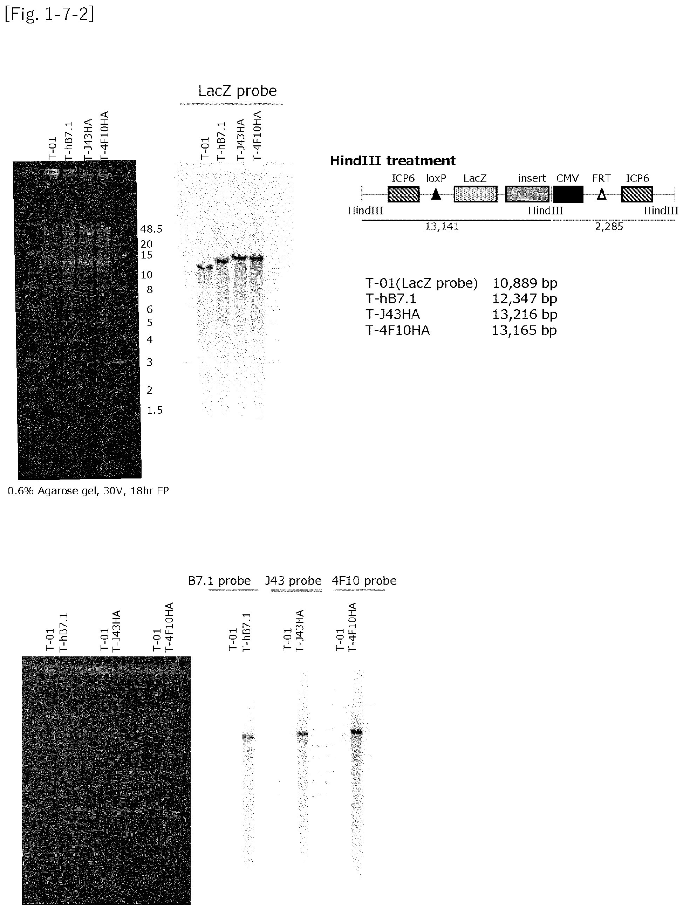

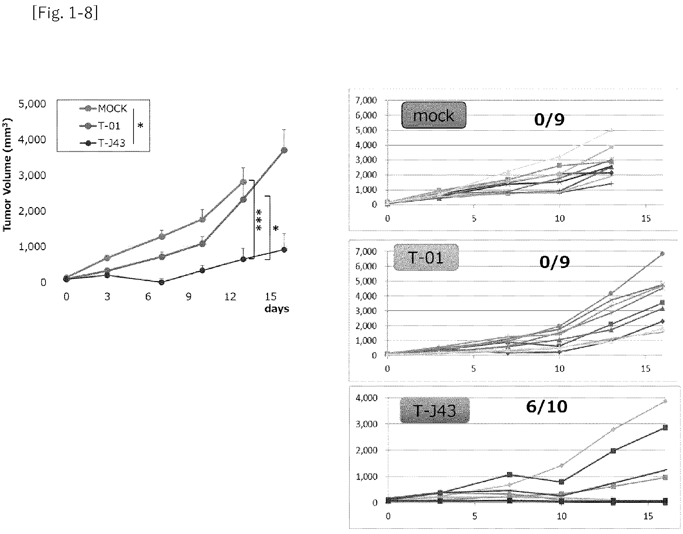

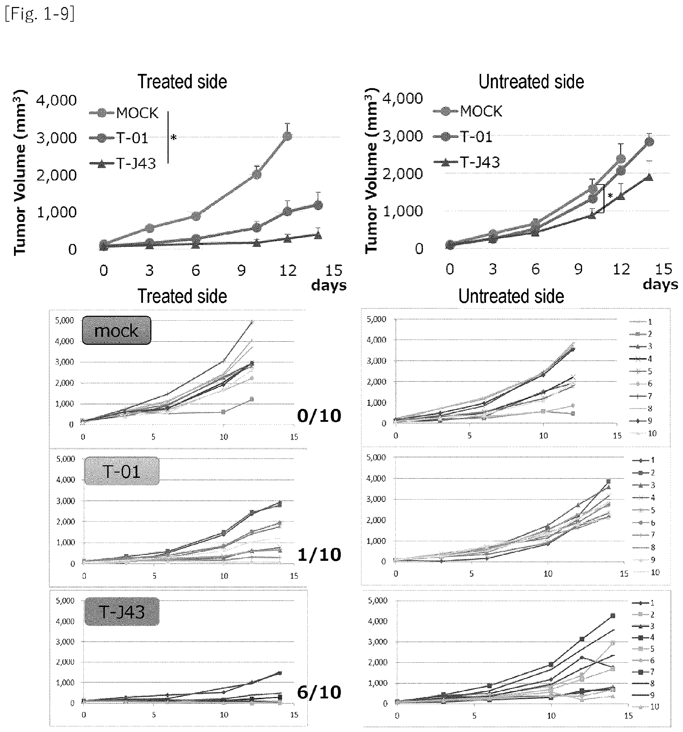

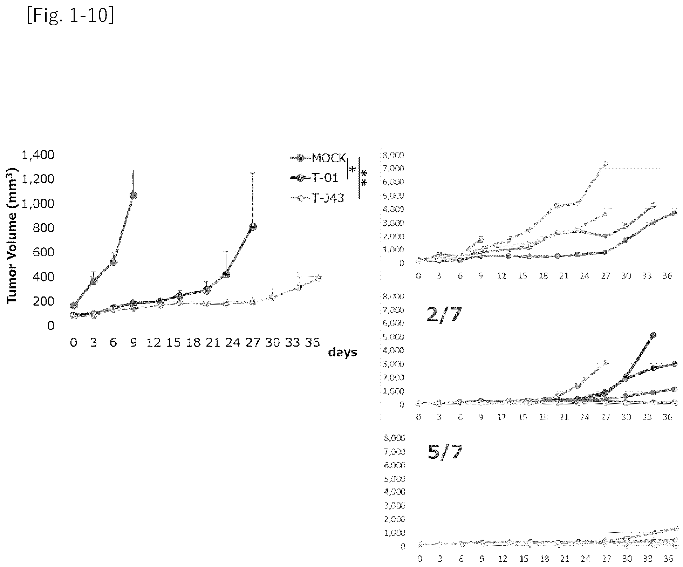

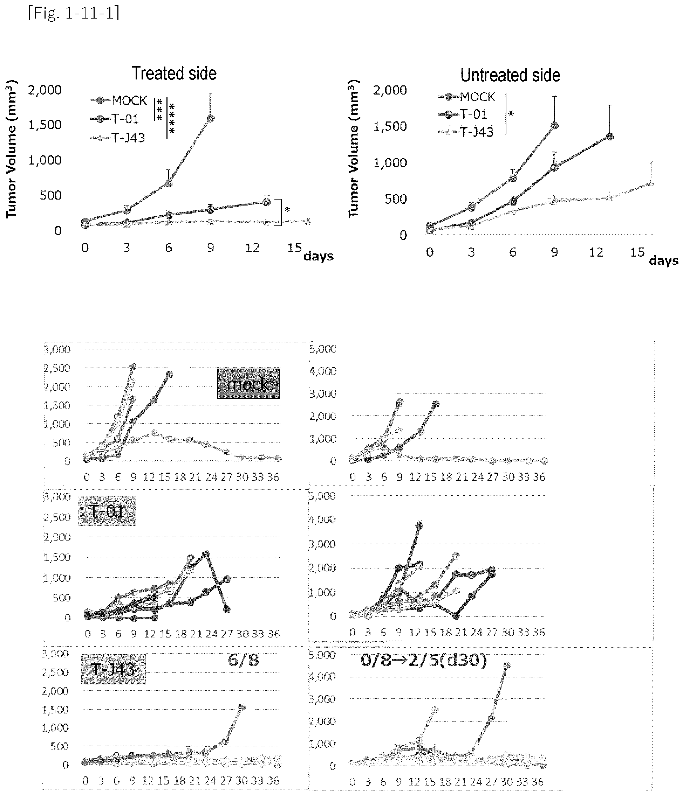

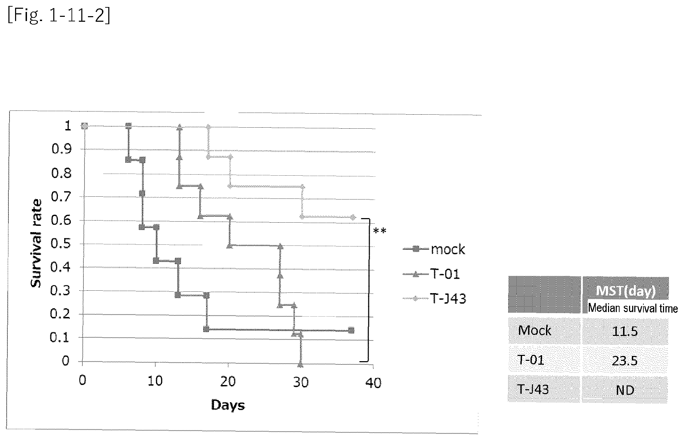

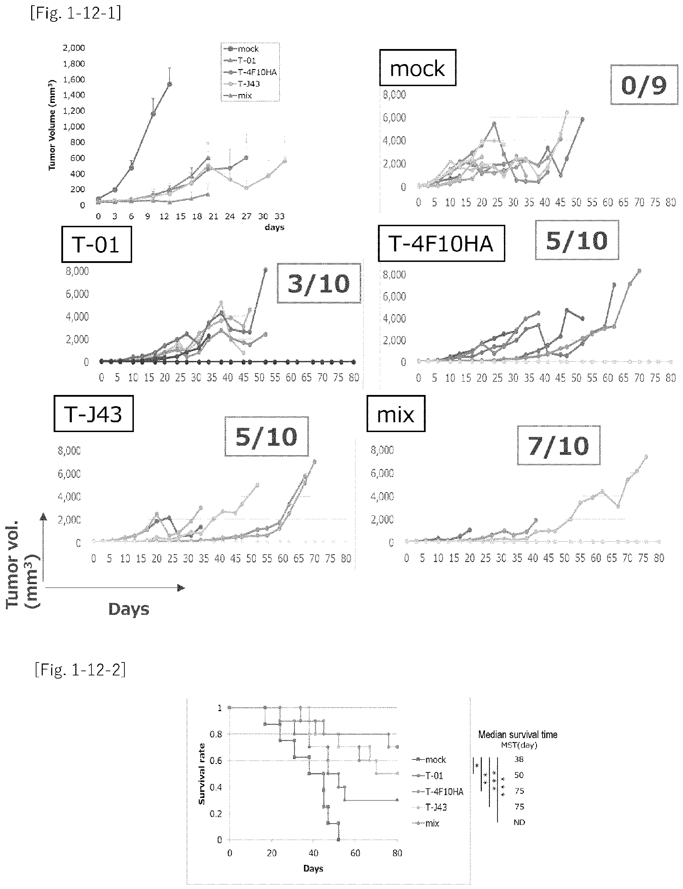

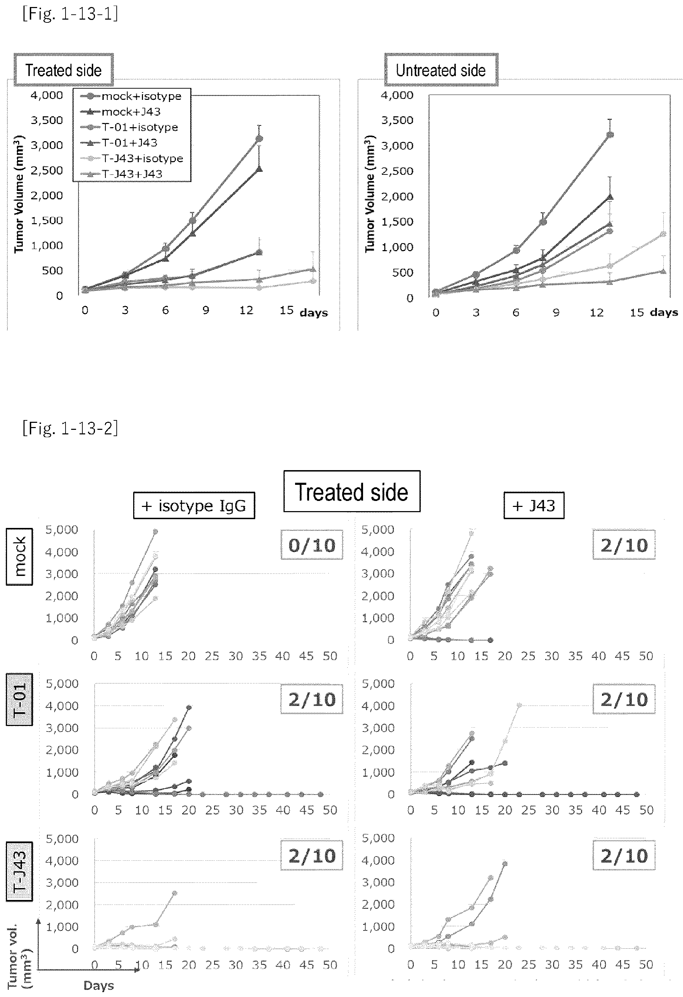

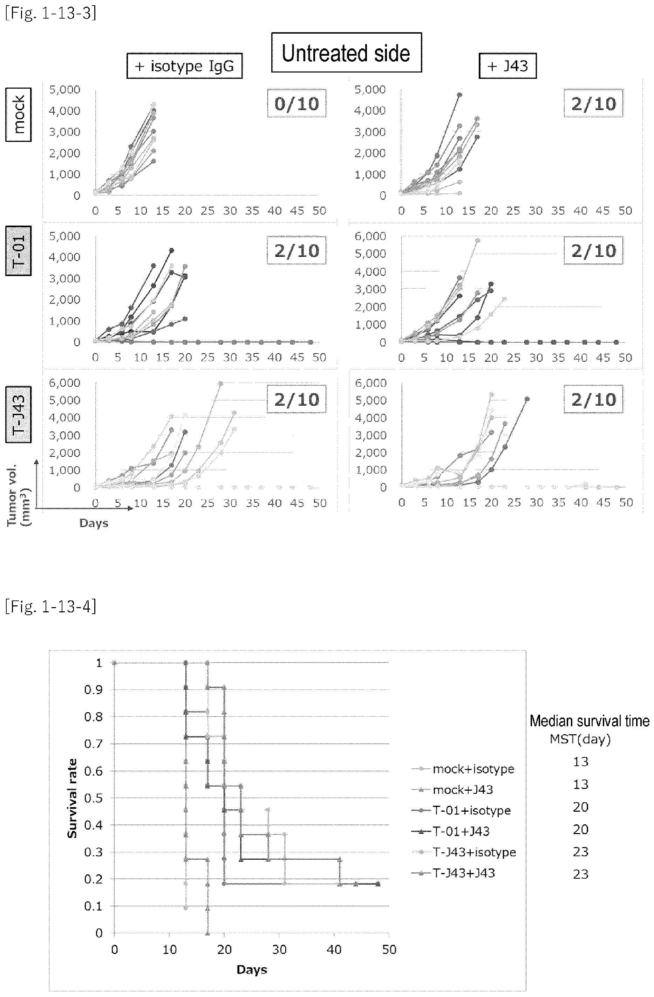

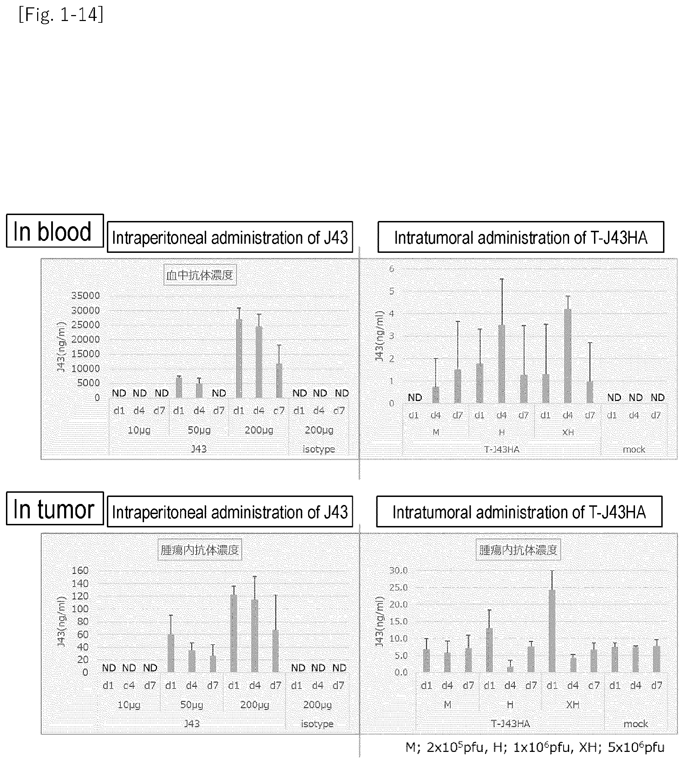

[Fig. 1-1-1] Amino acid sequence for expressing anti-mouse PD-1 antibody (derived from J43). Similar to nivolumab, the constant region of the H (heavy) chain was derived from human IgG4. The constant region of the L (light) chain was derived from human λ chain. The underlined portions are the hypervariable regions predicted by IMGT/DomainGapAlign.
[Fig. 1-1-2] Amino acid sequence for expressing anti-mouse PD-1 antibody (derived from J43). The constant region of the H chain was derived from Armenian Ham IgG, and the constant region of the L chain was derived from the Armenian Ham Ig κ chain.

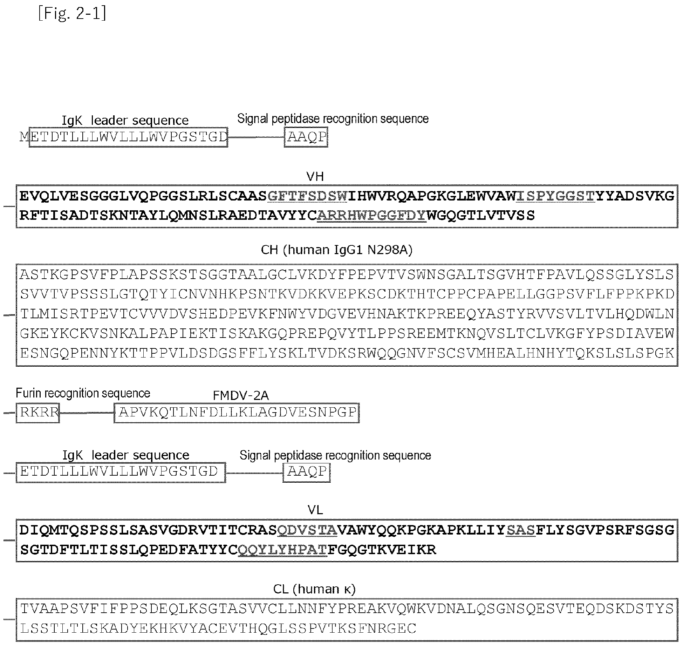

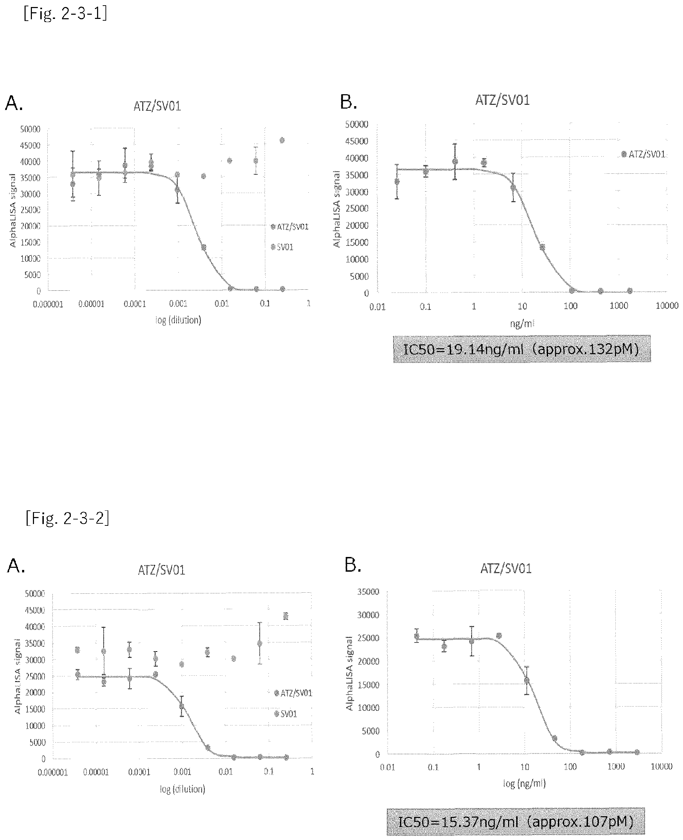

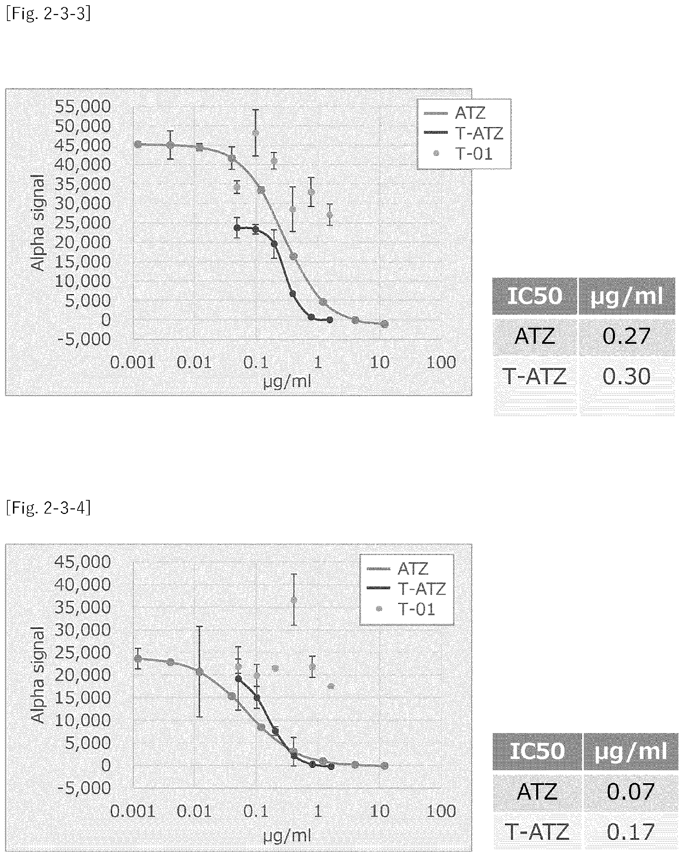

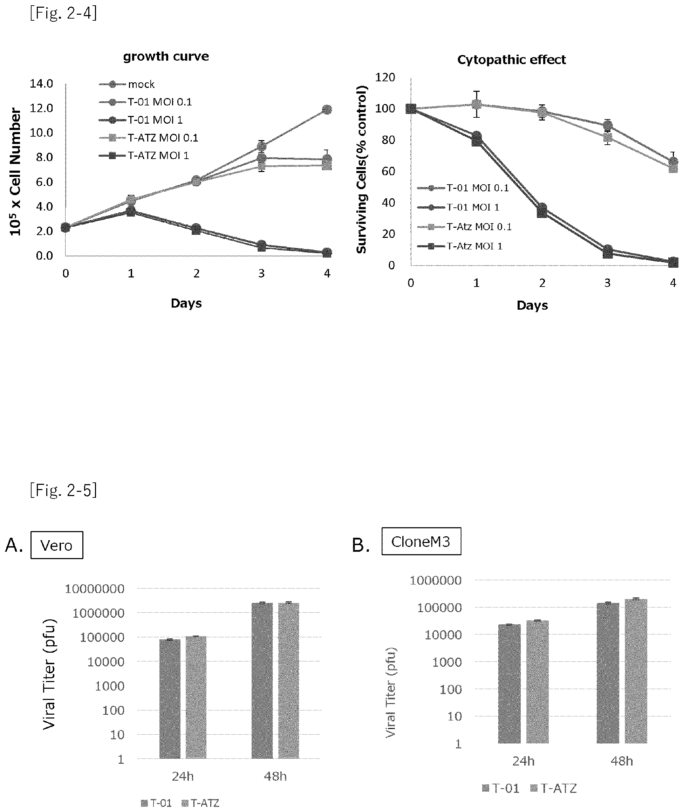

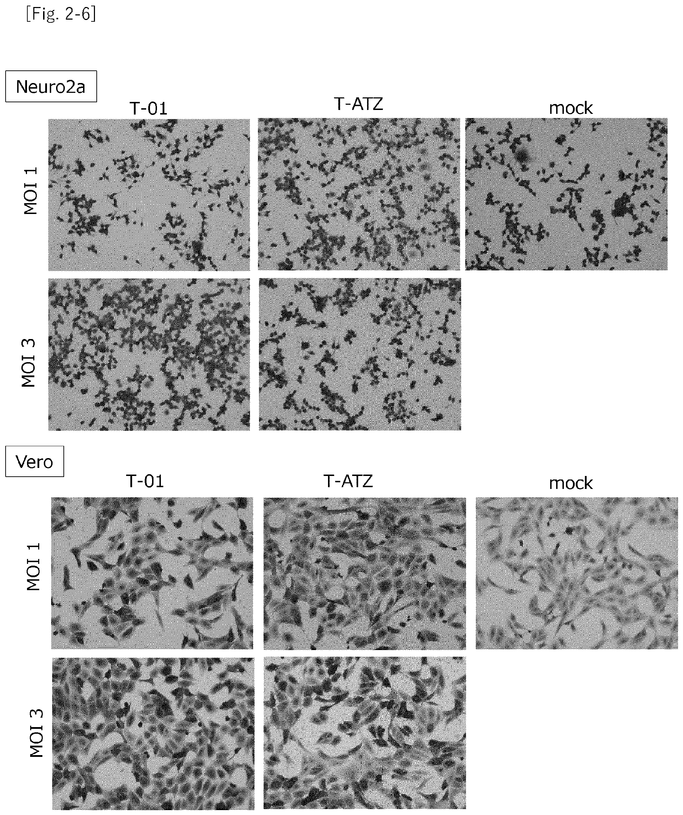

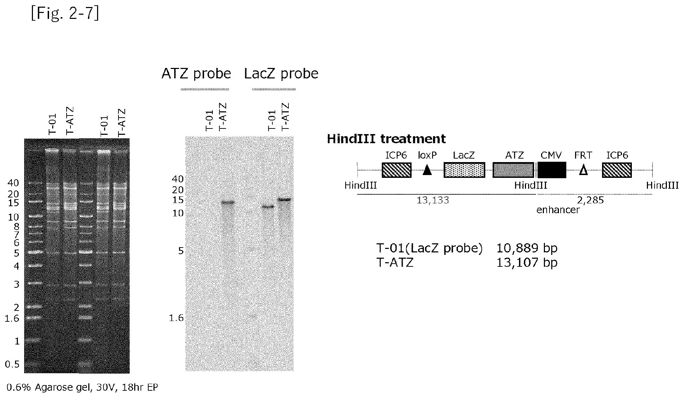

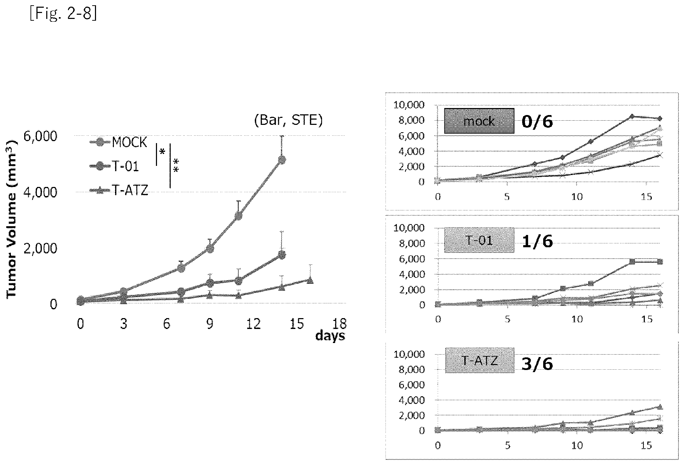

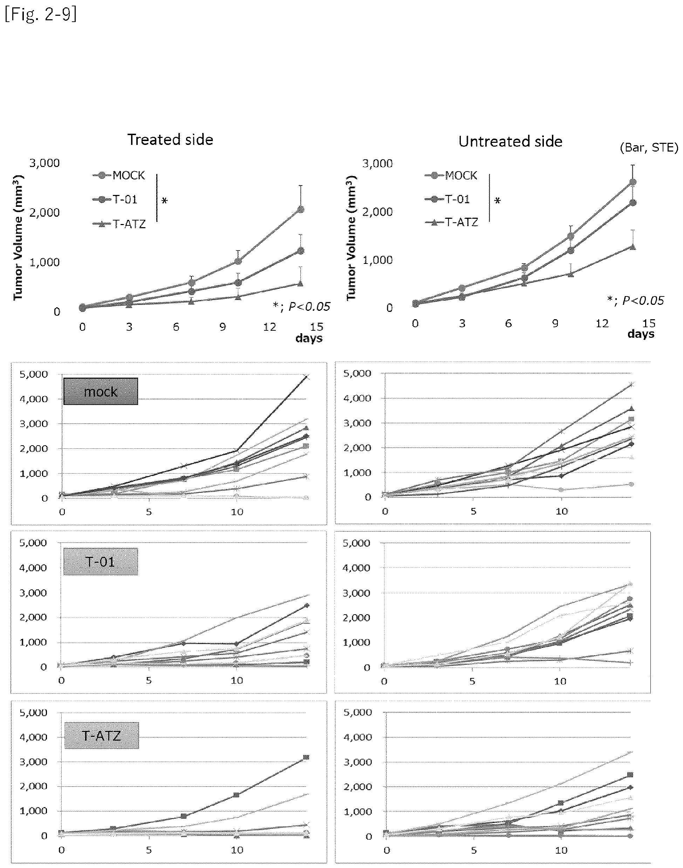

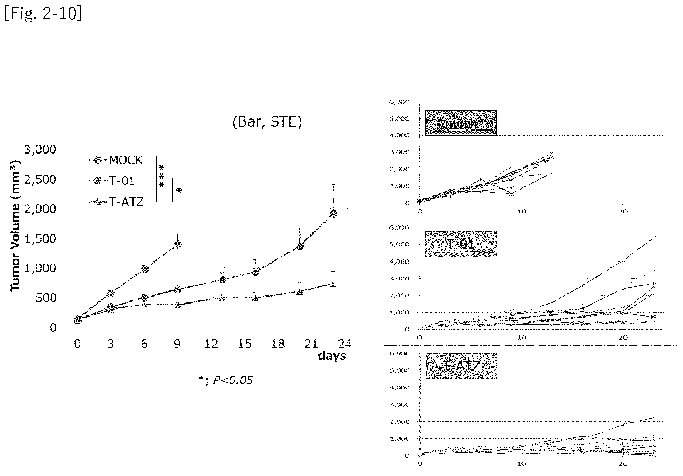

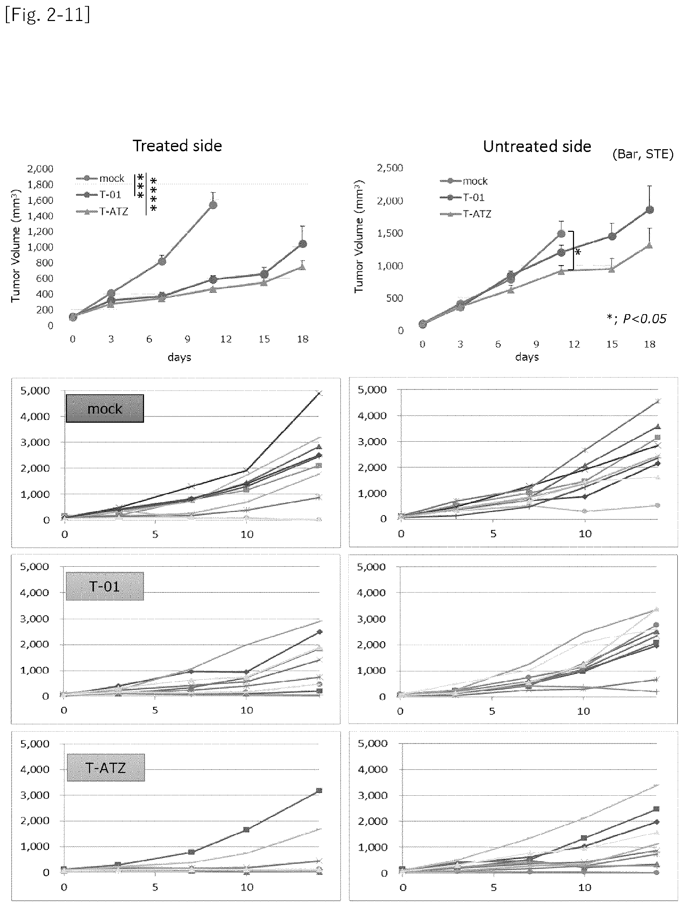

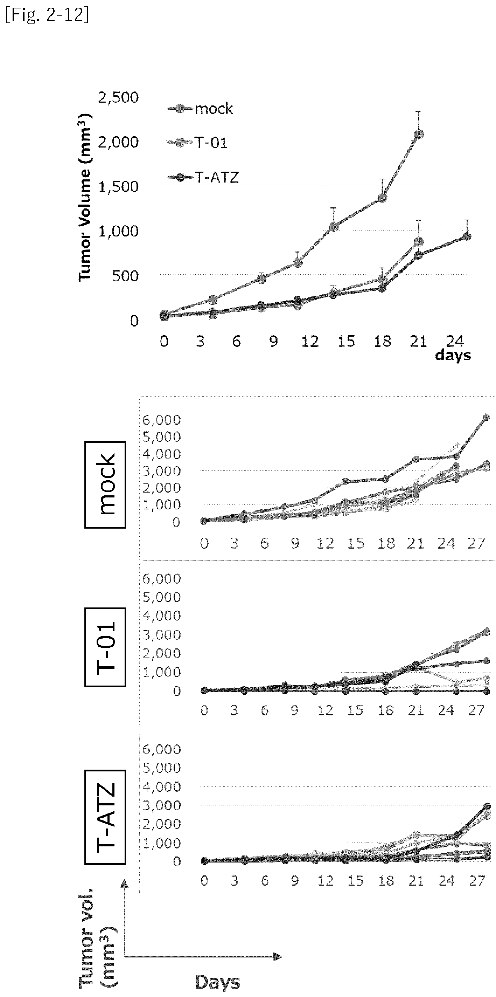

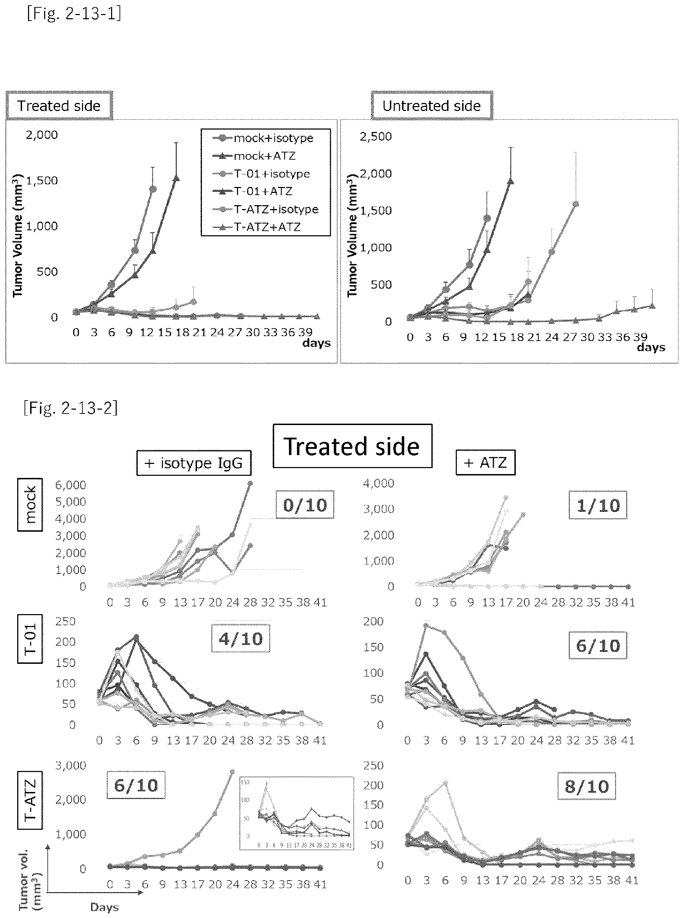

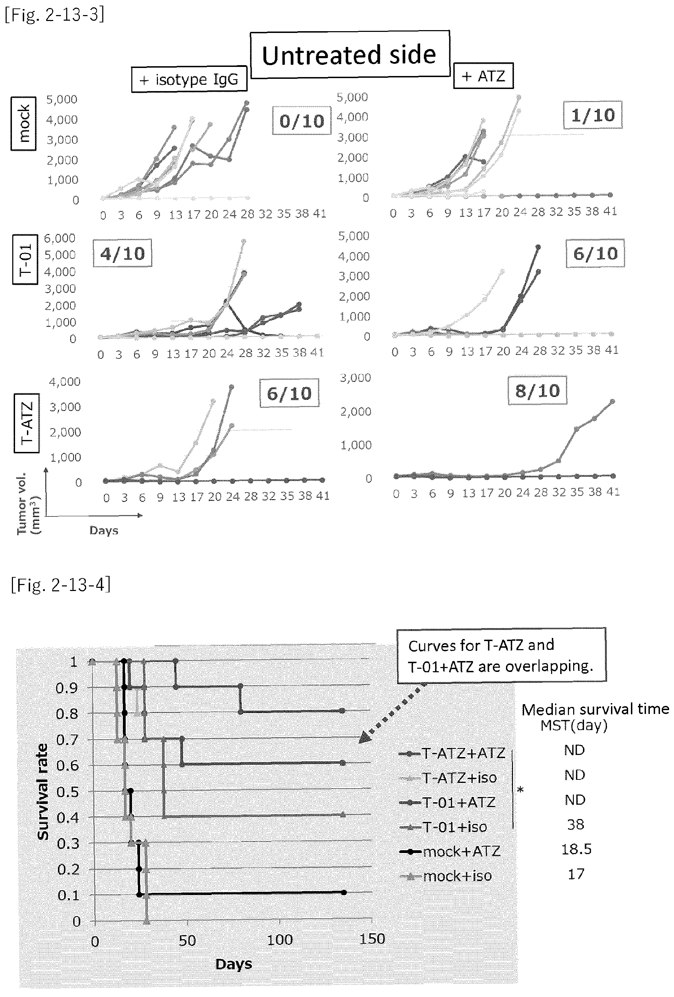

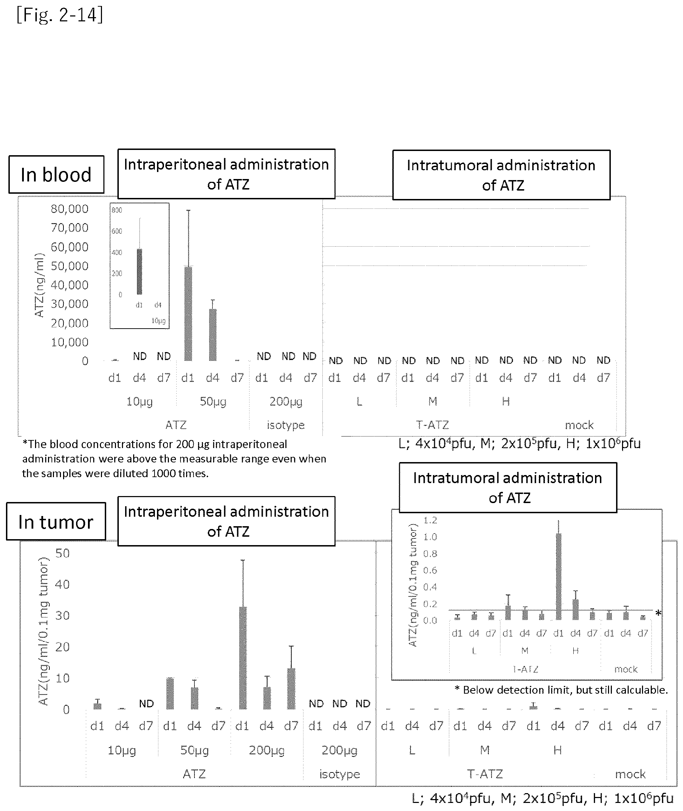

[Fig. 1-2-1] Comparison of anti-PD-1 antibody expression levels. T-J43 and T-01 were infected into HEK293T cells at MOI=1. After 48 hours, the supernatants were collected, concentrated by ultrafiltration (Amicon Ultra 30K), and then subjected to A) ELISA for human Fc (anti-human Fc antibody was immobilized, and detection was performed with HRP-anti-human Fc antibody) and B) PD-1-binding ELISA (mouse PD-1 was immobilized, and detection was performed with HRP-anti-human Fc antibody).

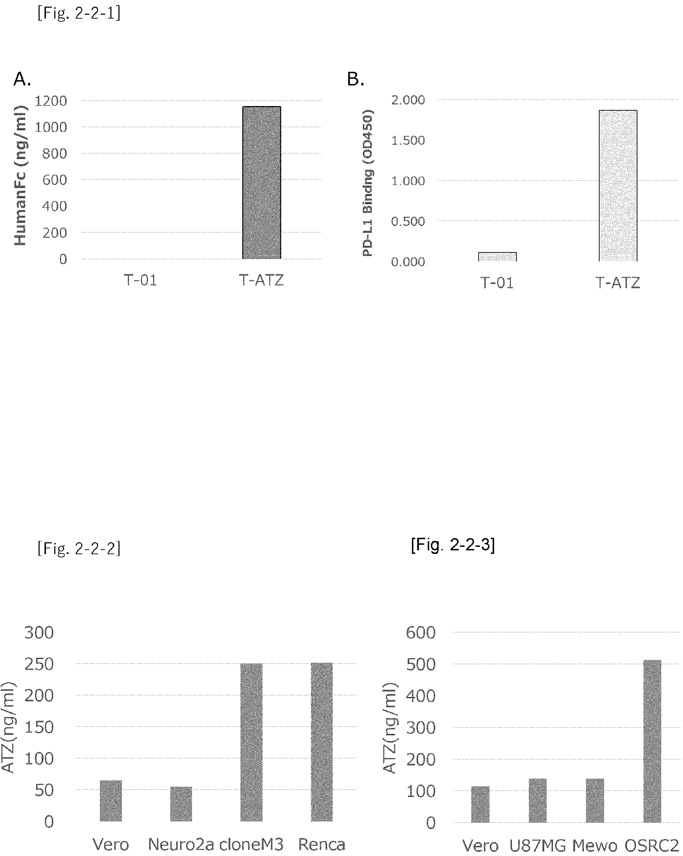

[Fig. 1-2-2] Comparison of anti-mouse PD-1 antibody expression levels in cells of various cell lines. PD-1-binding ELISA (mouse PD-1 was immobilized, and detection was performed with HRP-human Fc antibody) was performed. J43/SV01 was used as the standard (pre-quantified with human IgG).

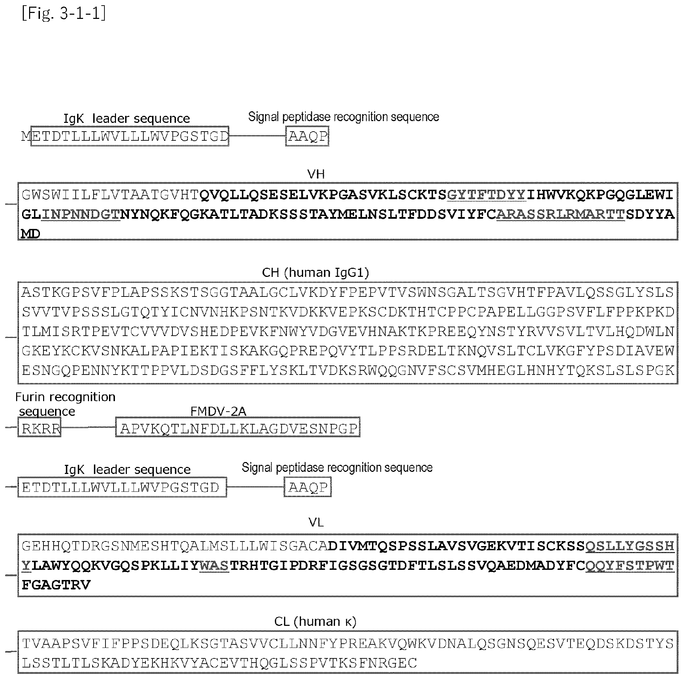

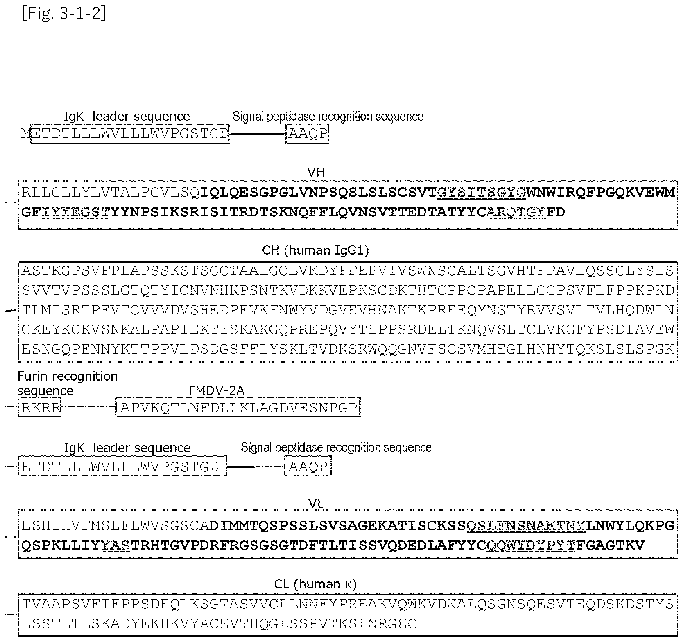

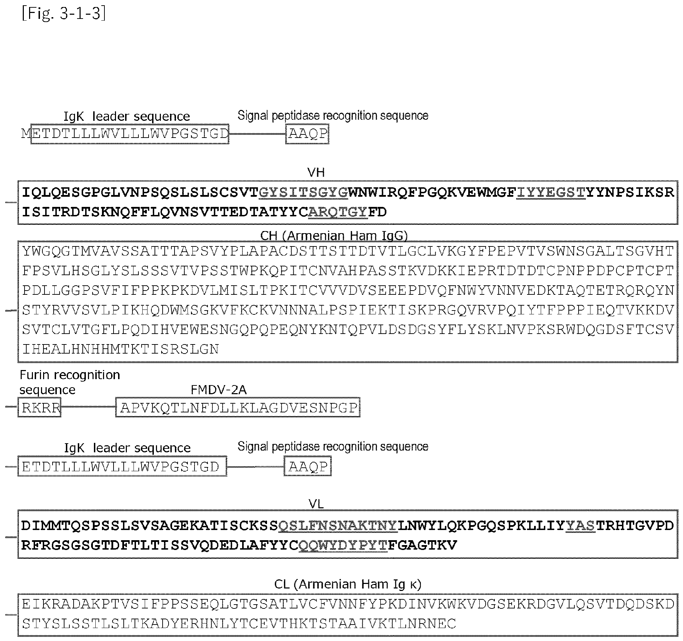

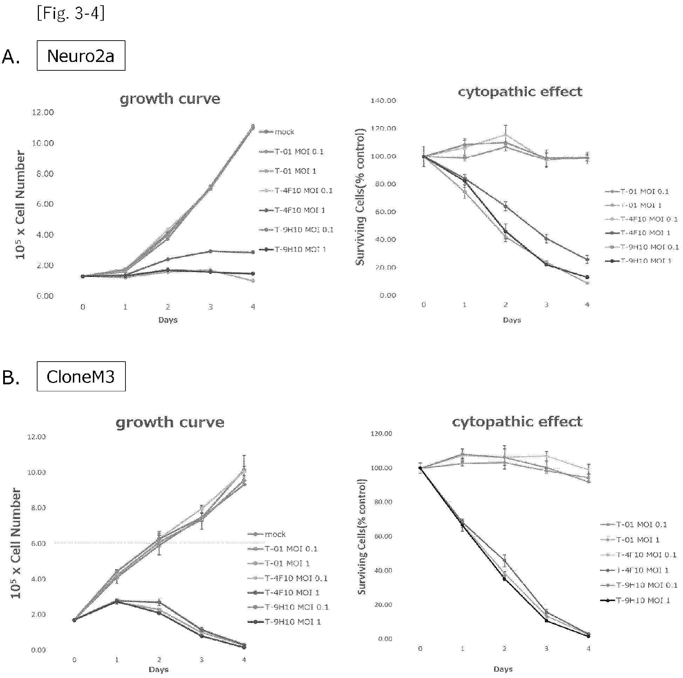

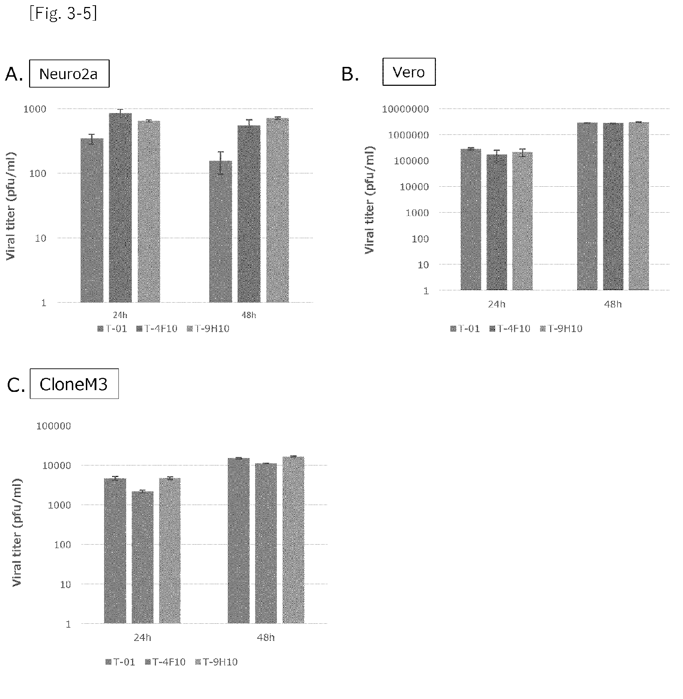

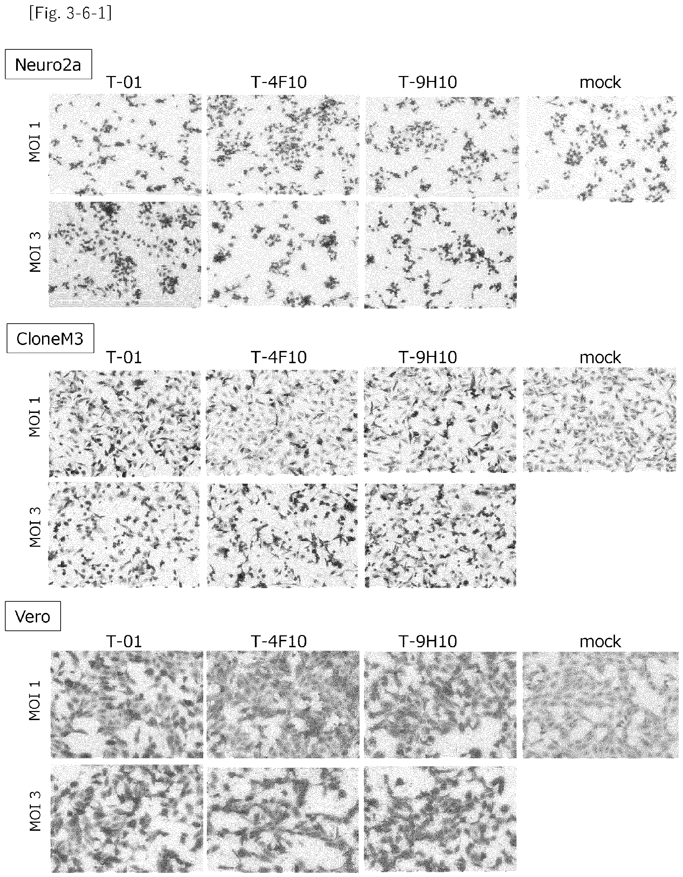

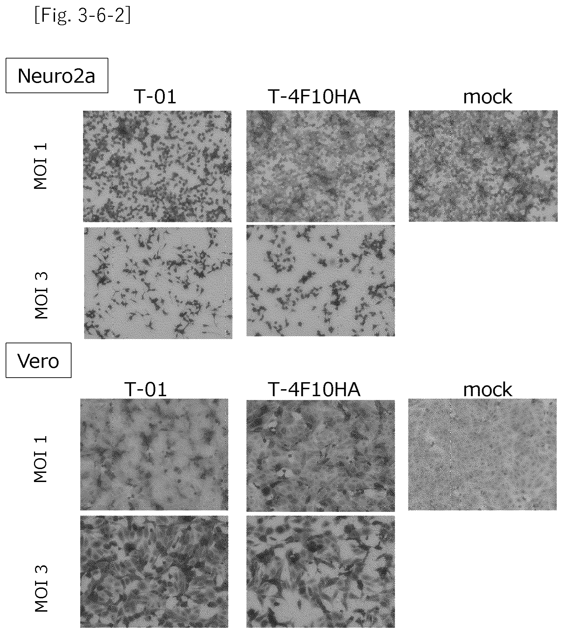

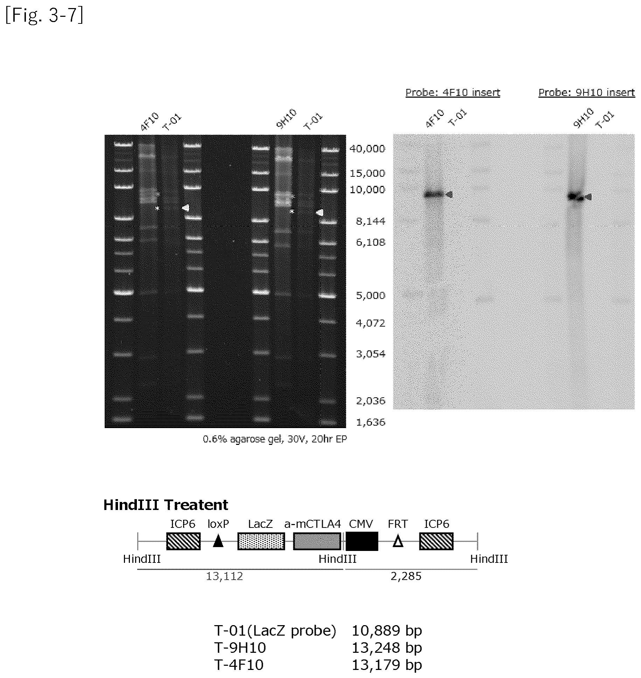

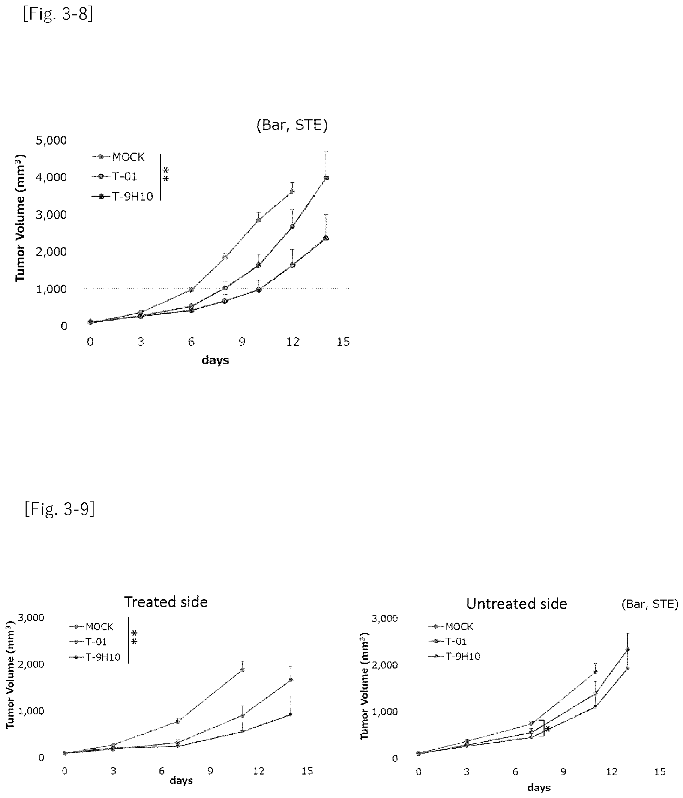

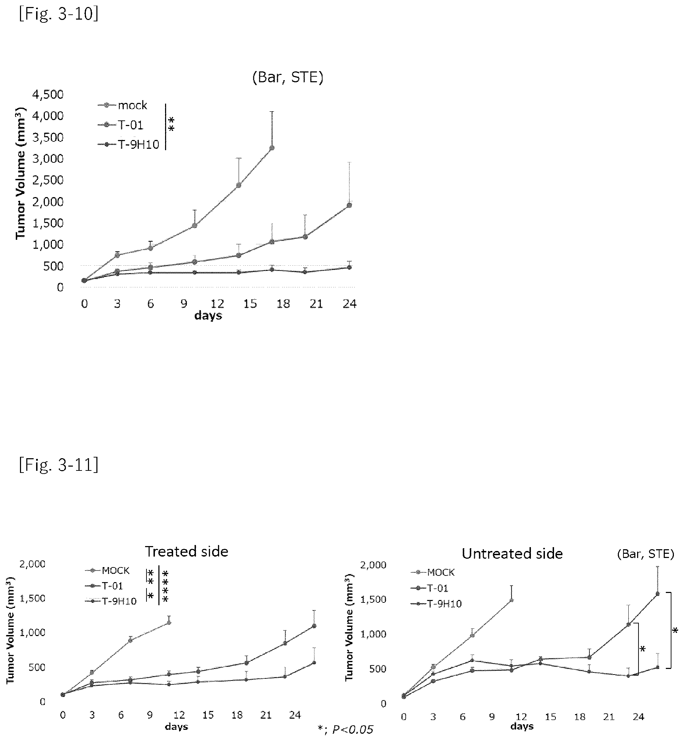

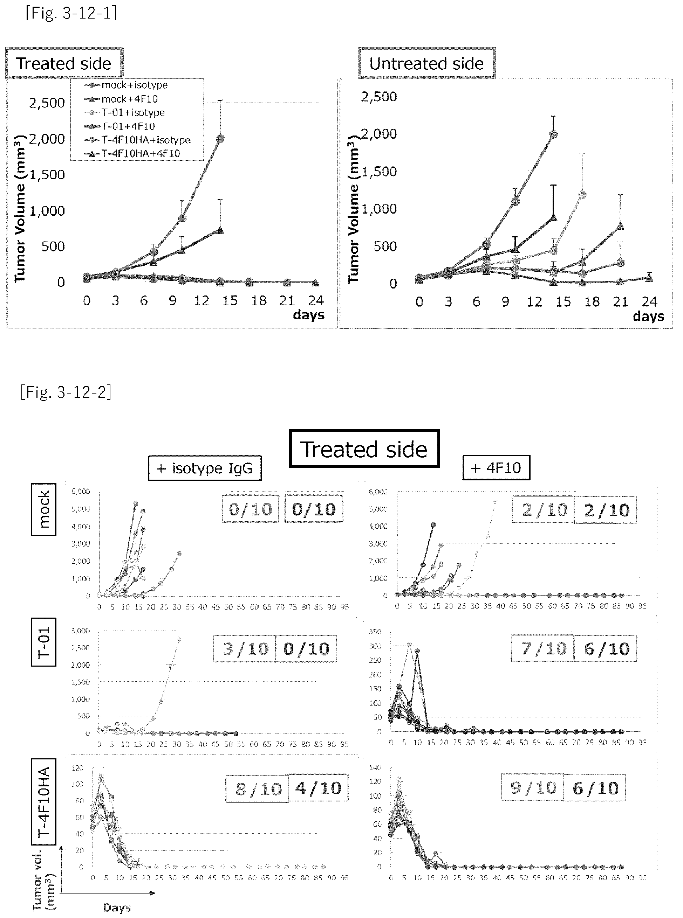

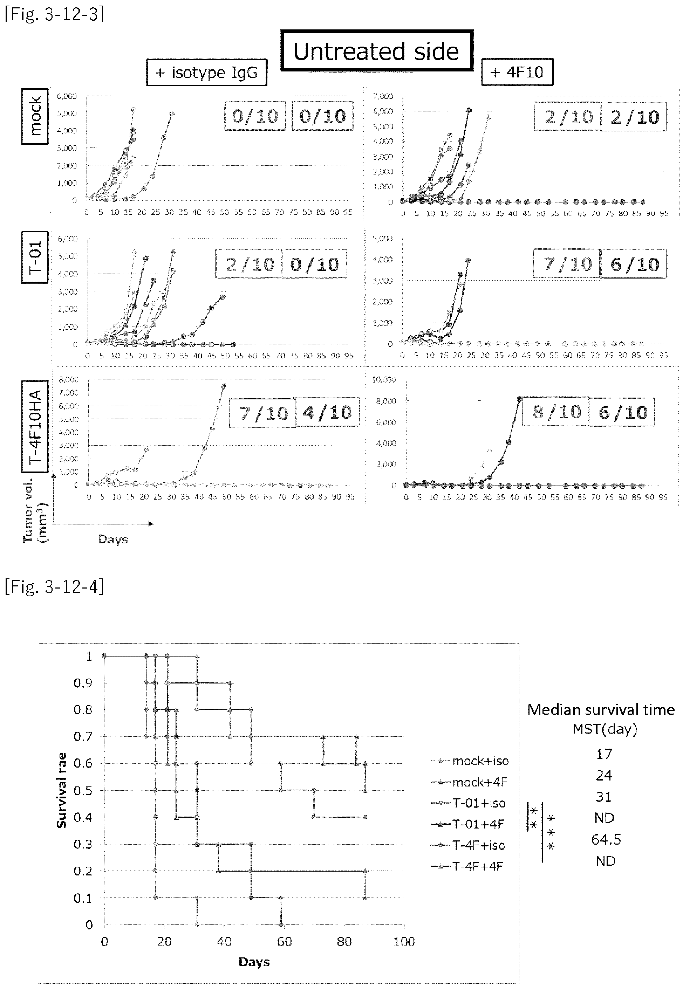

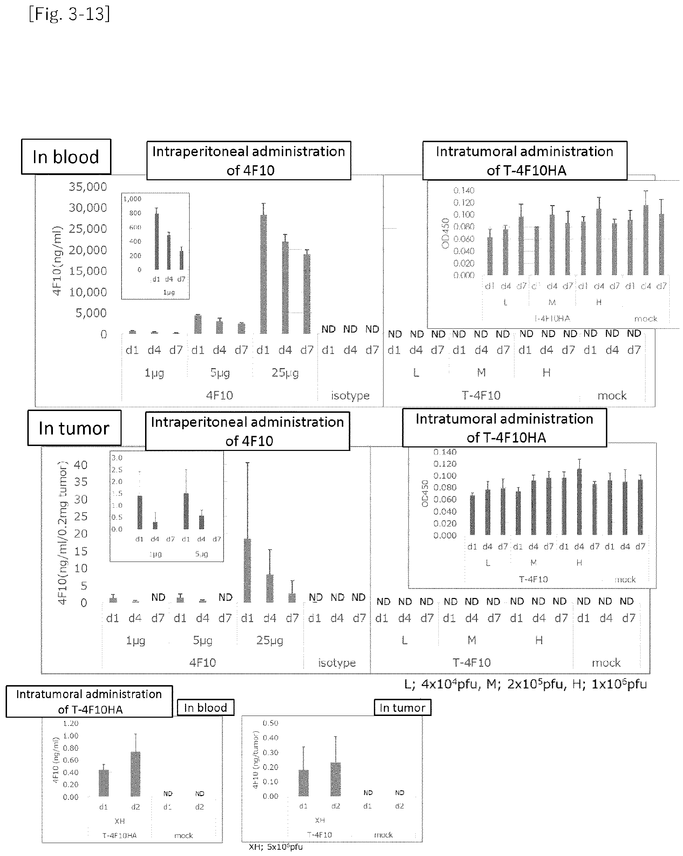

[Fig. 1-3-1] Results of PD-1/PD-L1 binding inhibitory activity assay. A) Transfection culture supernatants obtained with J43/SV-01 and SV-01 (empty vector). B) The IC₅₀ value of J43/SV-01 used in the experiment of A) calculated in terms of the concentration as human Fc was 6.81 ng/ml. C) Commercially available anti-mouse PD-1 antibody (RMP1-14, J43) and isotype control antibody (2A3).

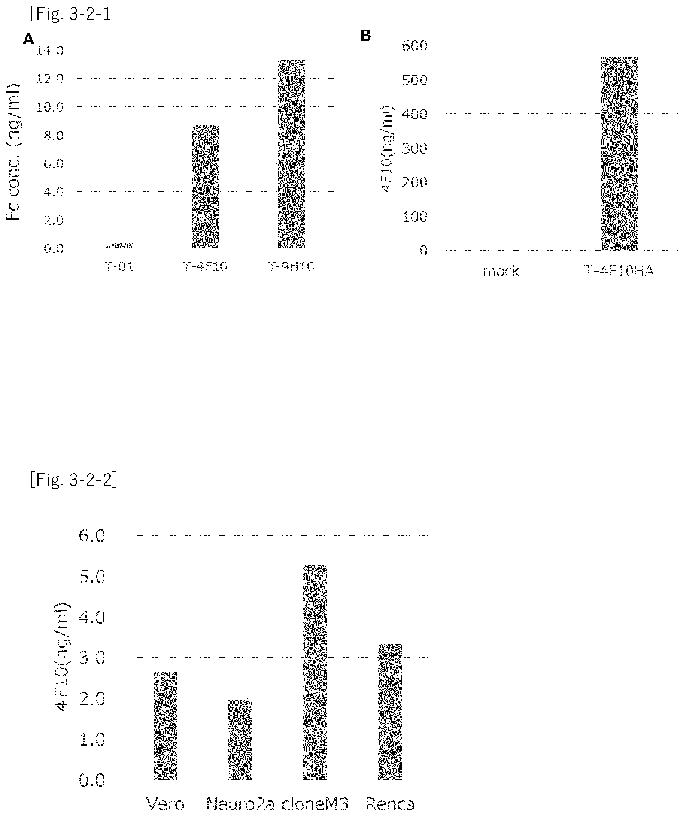

[Fig. 1-3-2] Results of PD-1/PD-L1 binding inhibitory activity assay.
[Fig. 1-4] Cytotoxicity assay.
[Fig. 1-5] Replication assay.
[Fig. 1-6] Infection efficiency of the prepared viruses (X-gal staining).

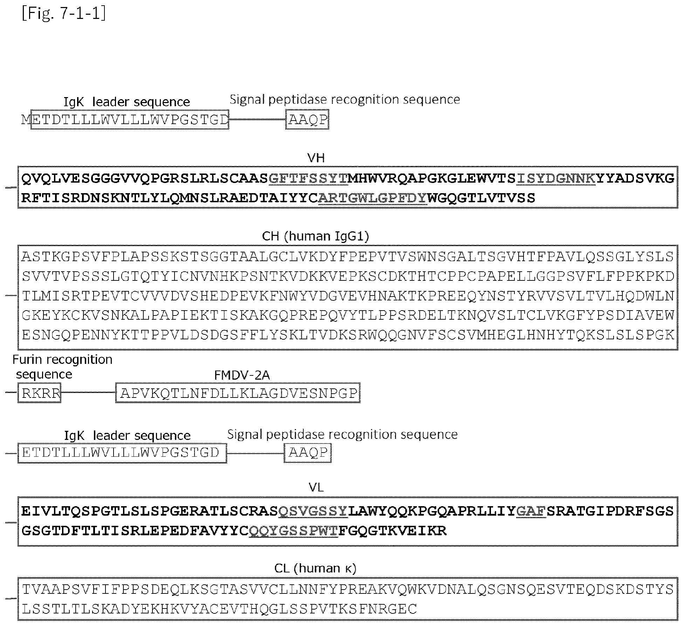

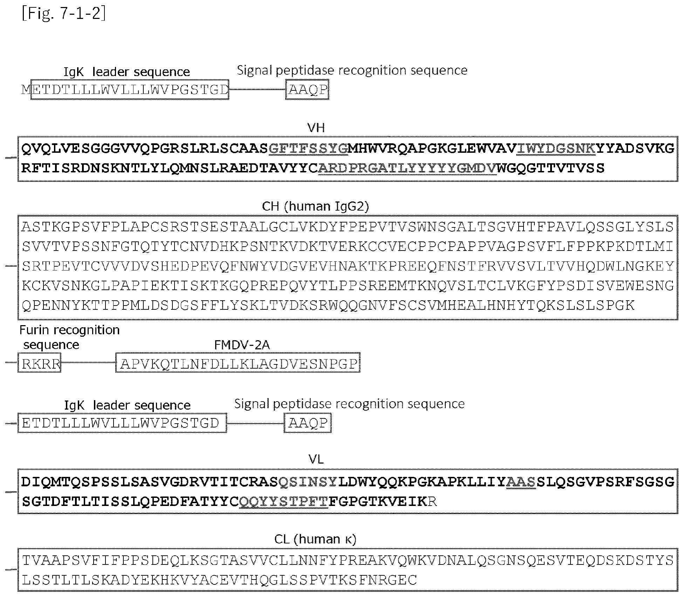

[Fig. 1-7-1] Structural confirmation of the prepared viruses by Southern blotting.

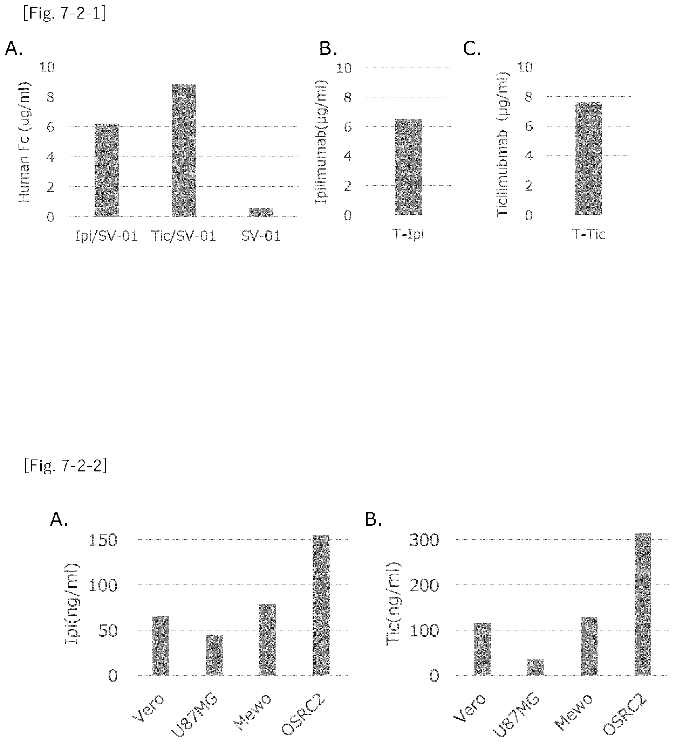

[Fig. 1-7-2] Structural confirmation of the prepared viruses by Southern blotting.
[Fig. 1-8] Comparison of antitumor effects of HSV-1 with anti-mouse PD-1 antibody expression in Neuro2a/A/J mouse unilateral subcutaneous tumor model. *, P<0.05 (two-way analysis of variance (two-way ANOVA) and Bonferroni comparison test). On day 13, significant differences were observed between mock and T-J43 (***, P<0.001) and T-01 and T-J43 (*, P<0.05) (one-way analysis of variance (one-way ANOVA) and Tukey's comparison test). Bar=SEM. The numbers of mice in which tumor suppression effect was observed (defined as tumor size of 200 mm³ or smaller) on day 16 are shown.
[Fig. 1-9] Comparison of antitumor effects of HSV-1 with anti-mouse PD-1 antibody expression in Neuro2a/A/J mouse bilateral subcutaneous tumor model. *, P<0.05 (two-way ANOVA and Tukey's comparison test). Untreated side: On day 10, a significant difference was observed only between mock and T-J43 (one-way ANOVA and Tukey's comparison test). Bar=SEM. The numbers of mice in which tumor suppression effect was observed (defined as tumor size of 200 mm³ or smaller) on day 14 are shown.
[Fig. 1-10] Antitumor effects in Clone M3/DBA/2 mouse unilateral subcutaneous tumor model. *, P<0.05; **, P<0.01 (two-way ANOVA and Bonferroni comparison test). Treated side; On day 13, a significant difference was observed only between T-01 and T-J43 (t-test). Bar=SEM. The ratios of mice that showed tumor disappearance (defined as tumor size of 200 mm³ or smaller) on day 27 are shown.
[Fig. 1-11-1] Antitumor effects in Clone M3/DBA/2 mouse bilateral subcutaneous tumor model. *, P < 0.05; ***, P < 0.001; ****, P<0.0001 (two-way ANOVA and Bonferroni comparison test). The ratios of mice that showed tumor disappearance (defined as tumor size of 200 mm³ or smaller) on day 13 are shown.
[Fig. 1-11-2] Antitumor effects in Clone M3/DBA/2 mouse bilateral subcutaneous tumor model. Survival curves obtained by observation ended upon death or when the maximum tumor diameter reached 20 mm on either side. The observation ended on day 38. T-01 vs T-J43, P=0.0069; log-rank test.
[Fig. 1-12] Comparison of antitumor effects of HSV-1 with anti-mouse PD-1 antibody (T-J43) expression and HSV-1 with anti-mouse CTLA-4 antibody (T-4F10HA) expression in Renca/Balb/c mouse unilateral subcutaneous tumor model. Bar=SEM. The ratios of mice that showed tumor disappearance (defined as tumor size of 50 mm³ or smaller) on day 80 are shown.
[Fig. 1-12-2] Kaplan-Meier survival curves. Survival curves obtained by observation ended upon death or when the maximum tumor diameter reached 24 mm. The observation ended on day 80. *, P<0.05; **, P<0.01; ***, P<0.001; log-rank test.
[Fig. 1-13-1] Comparison of antitumor effects of combination of T-01 and J43 antibody and T-J43 alone in Neuro2a/A/J mouse bilateral subcutaneous tumor model. P<0.05, T-01+J43 vs T-J43+J43 (2-way ANOVA and Bonferroni multiple comparison test)
[Fig. 1-13-2] Comparison of antitumor effects of combination of T-01 and J43 antibody and T-J43 alone in Neuro2a/A/J mouse bilateral subcutaneous tumor model (treated side). The ratios of mice that showed tumor disappearance (defined as tumor size of 50 mm³ or smaller) on day 48 are shown.
[Fig. 1-13-3] Comparison of antitumor effects of combination of T-01 and J43 antibody and T-J43 alone in Neuro2a/A/J mouse bilateral subcutaneous tumor model (untreated side). The ratios of mice that showed tumor disappearance (defined as tumor size of 50 mm³ or smaller) on day 48 are shown.
[Fig. 1-13-4] Kaplan-Meier survival curves. Survival curves obtained by observation ended upon death or when the maximum tumor diameter reached 20 mm on either side. The observation ended on day 48.
[Fig. 1-14] Comparison of antibody concentrations in blood and tumor observed after intraperitoneal administration of J43 and intratumoral administration of T-J43HA in Neuro2a/A/J unilateral subcutaneous tumor model.
[Fig. 2-1] Amino acid sequence for expressing anti-human and mouse PD-L1 antibodies (derived from atezolizumab). The constant region of the H chain was derived from human IgG1. The constant region of the L chain was derived from the human κ chain. The underlined portions are hypervariable regions predicted by IMGT/DomainGapAlign.
[Fig. 2-2-1] Comparison of anti-PD-L1 antibody expression levels. T-ATZ and T-01 were infected into HEK293T cells at MOI=1. After 48 hours, the supernatants were collected, concentrated by ultrafiltration (Amicon Ultra 30K), and then subjected to A) ELISA for human Fc (human Fc antibody was immobilized, and detection was performed with HRP-human Fc antibody) and B) PD-1-binding ELISA (mouse PD-1 was immobilized, and detection was performed with HRP-human Fc antibody).
[Fig. 2-2-2] Comparison of anti-mouse PD-L1 antibody expression levels in cells of various cell lines. PD-L1-binding ELISA (mouse PD-1 was immobilized, and detection was performed with HRP-human Fc antibody) was performed. Atezolizumab (ATZ) was used as the standard.
[Fig. 2-2-3] Comparison of anti-human PD-L1 antibody expression levels in cells of various cell lines. PD-L1-binding ELISA (human PD-1 was immobilized, and detection was performed with HRP-human Fc antibody) was performed. ATZ was used as the standard.
[Fig. 2-3-1] Results of PD-1/PD-L1 inhibitory activity assay. A) Transfection culture supernatants obtained with ATZ/SV-01 and SV-01 (empty vector). B) The IC₅₀ value of ATZ/SV-01 used in the experiment of A) calculated in terms of the concentration as human Fc was 19.14 ng/ml.
[Fig. 2-3-2] Results of PD-1/PD-L1 inhibitory activity assay. A) Transfection culture supernatants obtained with ATZ/SV-01 and SV-01 (empty vector). B) The IC₅₀ value of ATZ/SV-01 used in the experiment of A) calculated in terms of the concentration as human Fc was 15.37 ng/ml.

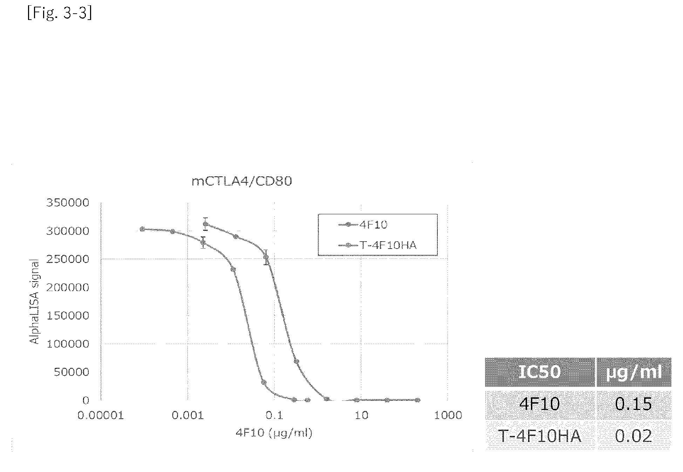

[Fig. 2-3-3] Results of mouse PD-1/PD-L1 inhibitory activity assay.
[Fig. 2-3-4] Results of human PD-1/PD-L1 inhibitory activity assay.
[Fig. 2-4] Cytotoxicity assay.
[Fig. 2-5] Replication assay.
[Fig. 2-6] Infection efficiency of the prepared viruses (X-gal staining).
[Fig. 2-7] Structural confirmation of the prepared viruses by Southern blotting.
[Fig. 2-8] Comparison of the antitumor effects of HSV-1 with anti-mouse PD-L1 antibody expression in Neuro2a/A/J mouse unilateral subcutaneous tumor model. *, P<0.05; **, P<0.01 (two-way ANOVA and Bonferroni comparison test). The numbers of mice in which tumor suppression effect was observed (defined as tumor size of 200 mm³ or smaller) on day 16 are shown.
[Fig. 2-9] Antitumor effects in Neuro2a/A/J mouse bilateral subcutaneous tumor model. *, P < 0.05 (two-way ANOVA and Bonferroni comparison test)
[Fig. 2-10] Antitumor effects in Clone M3/DBA/2 mouse unilateral subcutaneous tumor model. *, P<0.05; ***, P<0.001 (two-way ANOVA and Bonferroni comparison test)
[Fig. 2-11] Antitumor effects in Clone M3/DBA/2 mouse bilateral subcutaneous tumor model. Treated side: ***, P < 0.001; ****, P < 0.0001 (two-way ANOVA and Tukey's comparison test). Untreated side: *, P<0.05; a significant difference was observed only between mock and T-J43 on day 11 (one-way ANOVA and Tukey's comparison test); Bar=SEM.
[Fig. 2-12] Comparison of antitumor effect of HSV-1 with anti-mouse PD-L1 antibody (T-ATZ) expression in Renca/Balb/c mouse unilateral subcutaneous tumor model. The results were substantially equivalent to those obtained with T-01. Bar=SEM.
[Fig. 2-13-1] Comparison of antitumor effects of combination of T-01 and ATZ antibody and T-ATZ alone in Neuro2a/A/J mouse bilateral subcutaneous tumor model
[Fig. 2-13-2] Comparison of antitumor effects of combination of T-01 and ATZ antibody and T-ATZ alone in Neuro2a/A/J mouse bilateral subcutaneous tumor model (treated side). The ratios of mice that showed tumor disappearance (defined as tumor size of 50 mm³ or smaller) on day 87 are shown.
[Fig. 2-13-3] Comparison of antitumor effects of combination of T-01 and ATZ antibody and T-ATZ alone in Neuro2a/A/J mouse bilateral subcutaneous tumor model (untreated side). The ratios of mice that showed tumor disappearance (defined as tumor size of 50 mm³ or smaller) on day 87 are shown.
[Fig. 2-13-4] Kaplan-Meier survival curves. Survival curves obtained by observation ended upon death or when the maximum tumor diameter reached 20 mm. The observation ended on day 135. *, P<0.05; log-rank test.
[Fig. 2-14] Comparison of antibody concentrations in blood and tumor observed after intraperitoneal administration of ATZ and intratumoral administration of T-ATZ in Neuro2a/A/J mouse unilateral subcutaneous tumor model. The blood concentrations for 200 µg ATZ administration group were exceeded the measurable range even at a 1:1000 dilution, and therefore the results are indicated as ND. For the intratumoral administration of T-ATZ, the results were below the line shown in the graph indicating the detection limit, but values could be calculated from the equation of the calibration curve and therefore they are shown for reference.
[Fig. 3-1-1] Amino acid sequence for expressing anti-mouse CTLA4 antibody (derived from 9H10). The constant region of the H chain was derived from human IgG1, and the constant region of the L chain was derived from the human κ chain. The underlined red characters indicate hypervariable regions predicted by IMGT/DomainGapAlign.
[Fig. 3-1-2] Amino acid sequence for expressing anti-mouse CTLA4 antibody (derived from 4F10). The constant region of the H chain was derived from human IgG1, and the constant region of the L chain was derived from the human κ chain. The underlined red characters indicate hypervariable regions predicted by IMGT/DomainGapAlign.
[Fig. 3-1-3] Amino acid sequence for expressing anti-mouse CTLA4 antibody (derived from 4F10). The constant region of the H chain was derived from Armenian Ham IgG, and the constant region of the L chain was derived from the Armenian Ham Ig κ chain.
[Fig. 3-2-1] Comparison of anti-CTLA-4 antibody expression levels.
[Fig. 3-2-2] Comparison of anti-mouse CTLA-4 antibody expression levels in cells of various cell lines. CTLA-4 binding ELISA was performed (mouse CTLA-4 was immobilized, and detection was performed with HRP-labeled anti-Armenian Hamster IgG antibody). 4F10 was used as the standard.
[Fig. 3-3] Results of CTLA-4/CD80 binding inhibitory activity assay.
[Fig. 3-4] Cytotoxicity assay.
[Fig. 3-5] Replication assay.

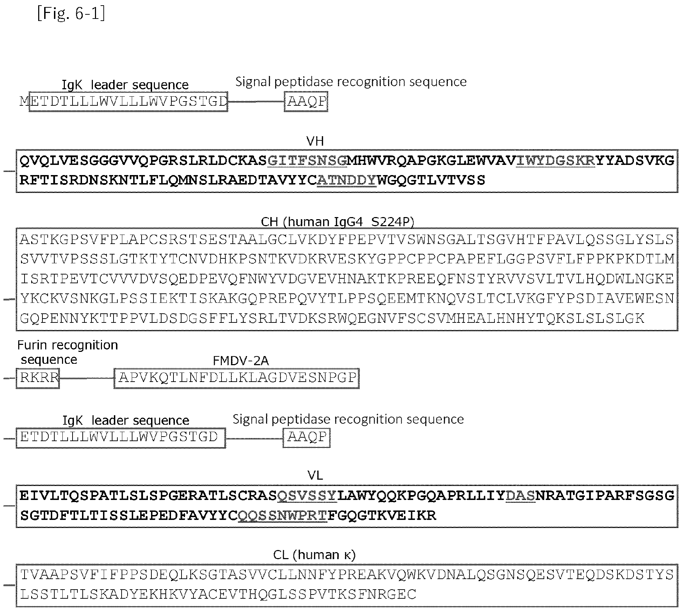

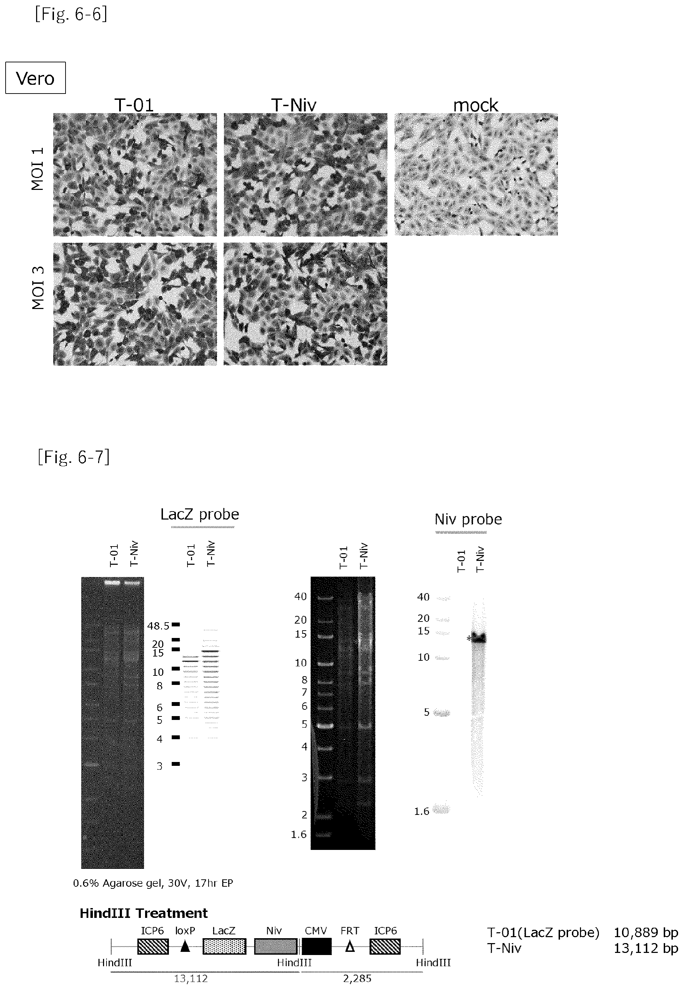

[Fig. 3-6-1] Infection efficiency of the prepared viruses (X-gal staining).

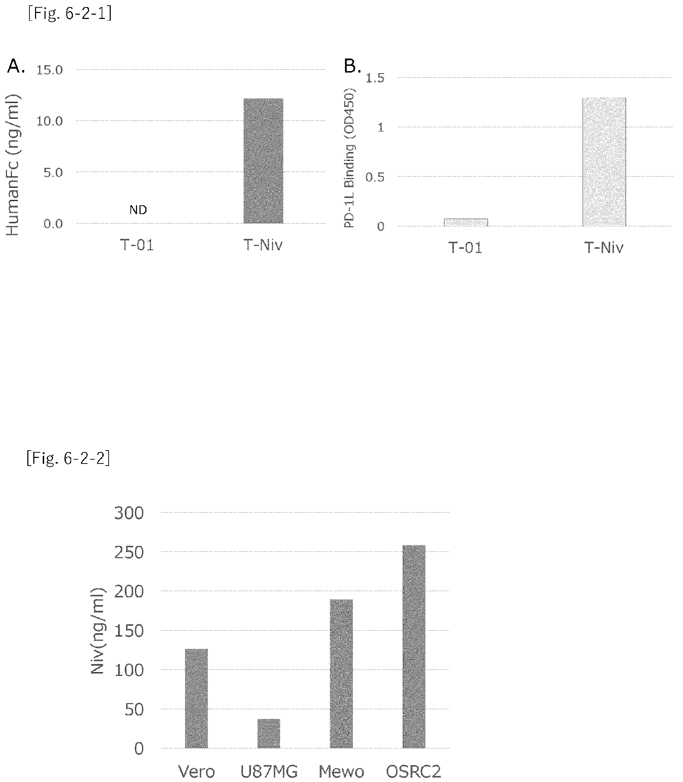

[Fig. 3-6-2] Infection efficiency of the prepared viruses (X-gal staining).
[Fig. 3-7] Structural confirmation of the prepared viruses by Southern blotting.
[Fig. 3-8] Antitumor effects in Neuro2a/A/J mouse unilateral subcutaneous tumor model. **, P<0.01 (two-way ANOVA and Bonferroni comparison test).
[Fig. 3-9] Antitumor effects in Neuro2a/A/J mouse bilateral subcutaneous tumor model. Treated side: **, P < 0.01 (two-way ANOVA and Bonferroni comparison test). Untreated side: *, P < 0.05. A significant difference was observed only between mock and T-9H10 on day 7 (one-way ANOVA and Tukey's comparison test). Bar=SEM.
[Fig. 3-10] Antitumor effects in Clone M3/DBA/2 mouse unilateral subcutaneous tumor model. **, P < 0.01 (two-way ANOVA and Bonferroni comparison test).
[Fig. 3-11] Antitumor effects in Clone M3/DBA/2 mouse bilateral subcutaneous tumor model. Treated side: *, P < 0.05; **, P < 0.01; ****, P < 0.0001 (two-way ANOVA and Bonferroni comparison test). Untreated side: *, P < 0.05. Significant difference was observed only between T-01 and T-9H10 on days 23 and 26 (t-test). Bar = SEM.
[Fig. 3-12-1] Comparison of antitumor effects of combination of T-01 and 4F10 antibody and T-4F10HA alone in Neuro2a/A/J mouse bilateral subcutaneous tumor model.
[Fig. 3-12-2] Comparison of antitumor effects of combination of T-01 and 4F10 antibody and T-4F10HA alone in Neuro2a/A/J mouse bilateral subcutaneous tumor model (treated side). The ratios of mice that showed tumor disappearance on day 42 are shown on the left side, and the ratios of mice that showed tumor disappearance on day 87 are shown on the right side.
[Fig. 3-12-3] Comparison of antitumor effects of combination of T-01 and 4F10 antibody and T-4F10HA alone in Neuro2a/A/J mouse bilateral subcutaneous tumor model (untreated side). The ratios of mice that showed tumor disappearance on day 42 are shown on the left side, and the ratios of mice that showed tumor disappearance on day 87 are shown on the right side.
[Fig. 3-12-4] Kaplan-Meier survival curves. Survival curves obtained by observation ended upon death or when the maximum tumor diameter reached 20 mm. The observation ended on day 38. **, P<0.01; ***, P<0.001; log-rank test (only major significant differences are indicated).
[Fig. 3-13] Comparison of antibody concentrations in blood and tumor observed after intraperitoneal administration of 4F 10 and intratumoral administration of T-4F10HA in Neuro2a/A/J mouse unilateral subcutaneous tumor model.

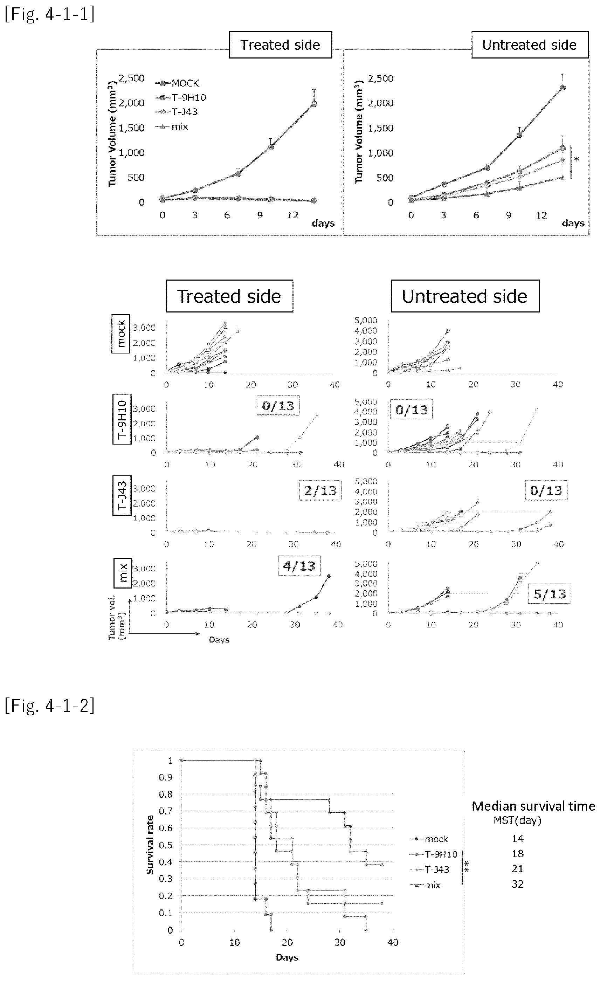

[Fig. 4-1-1] Effect of combination (mix) of HSV-1 with anti-CTLA-4 antibody expression (T-9H10) and HSV-1 with anti-PD-1 antibody (T-J43) expression in Neuro2a/A/J mouse bilateral subcutaneous tumor model. The ratios of mice that showed tumor disappearance (defined as tumor size of 50 mm³ or smaller) on day 48 are shown. Untreated side: Significant difference was observed between T-4F10 and the mix (*, P<0.05; two-way ANOVA and Bonferroni comparison test)
[Fig. 4-1-2] Kaplan-Meier survival curves. Survival curves obtained by observation ended upon death or when the maximum tumor diameter reached 20 mm on either side. The observation ended on day 38. **, P<0.01; log-rank test.

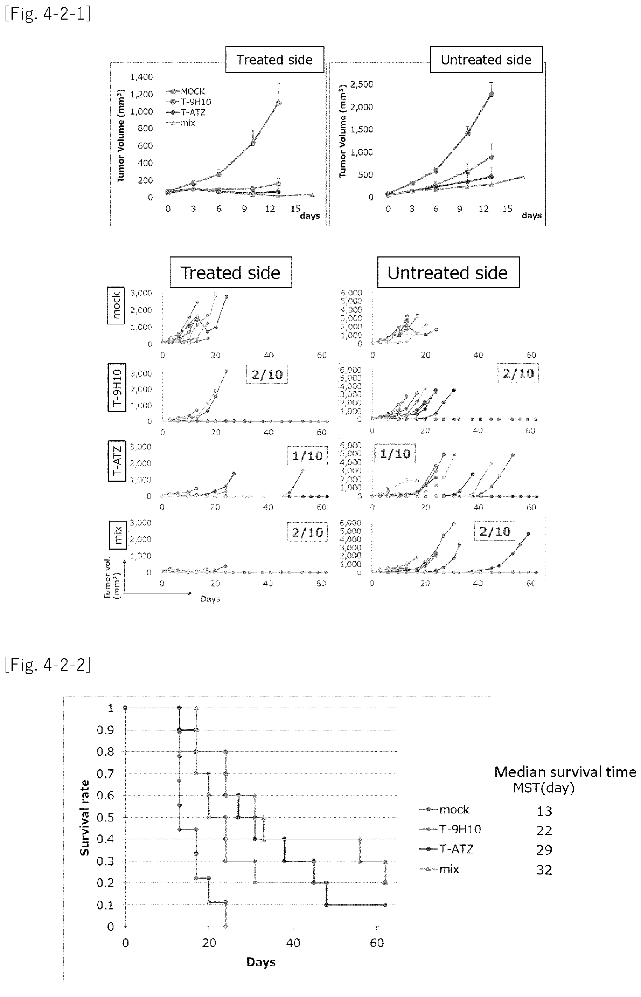

[Fig. 4-2-1] Effect of combination (mix) of HSV-1 with anti-CTLA-4 antibody expression (T-9H10) and HSV-1 with anti-PD-L1 antibody expression (T-ATZ) in Neuro2a/A/J mouse bilateral subcutaneous tumor model. The ratios of mice that showed tumor disappearance (defined as tumor size of 50 mm³ or smaller) on day 62 are shown.
[Fig. 4-2-2] Kaplan-Meier survival curves. Survival curves obtained by observation ended upon death or when the maximum tumor diameter reached 20 mm on either side. The observation ended on day 62.

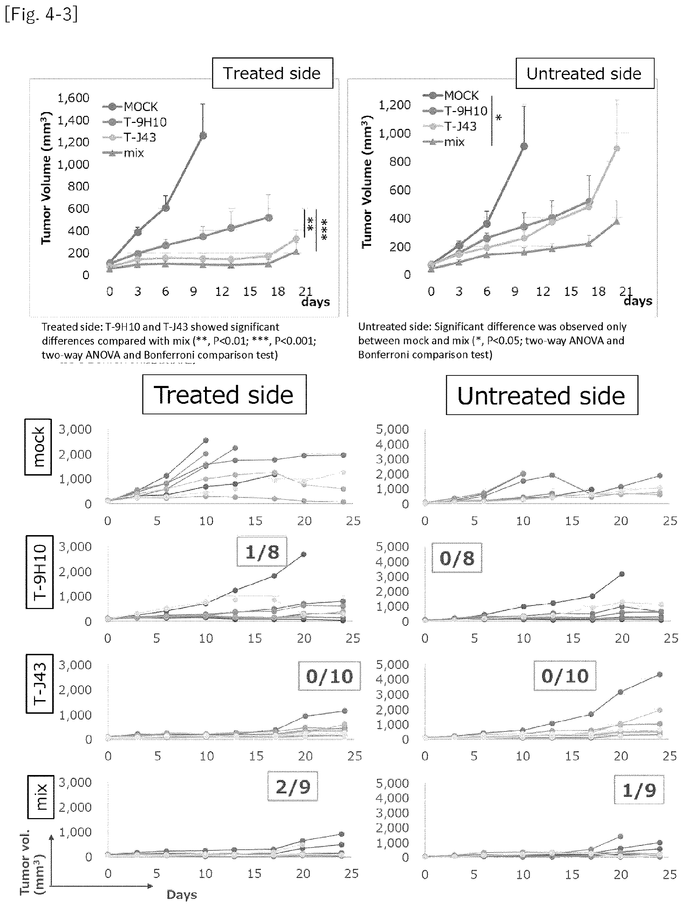

[Fig. 4-3] Effects of combination (mix) of HSV-1 with anti-CTLA-4 antibody expression (T-9H10) and HSV-1 with anti-PD-1 antibody expression (T-J43) in Clone M3/DBA/2 mouse bilateral subcutaneous tumor model.

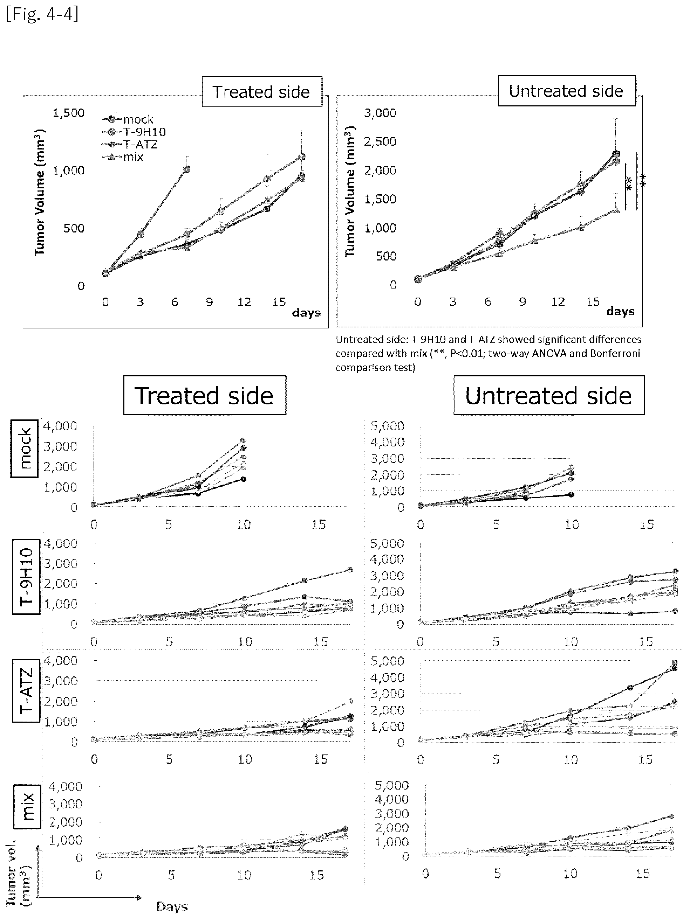

[Fig. 4-4] Effects of combination (mix) of HSV-1 with anti-CTLA-4 antibody expression (T-9H10) and HSV-1 with anti-PD-L1 antibody expression (T-ATZ) in Clone M3/DBA/2 mouse bilateral subcutaneous tumor model.

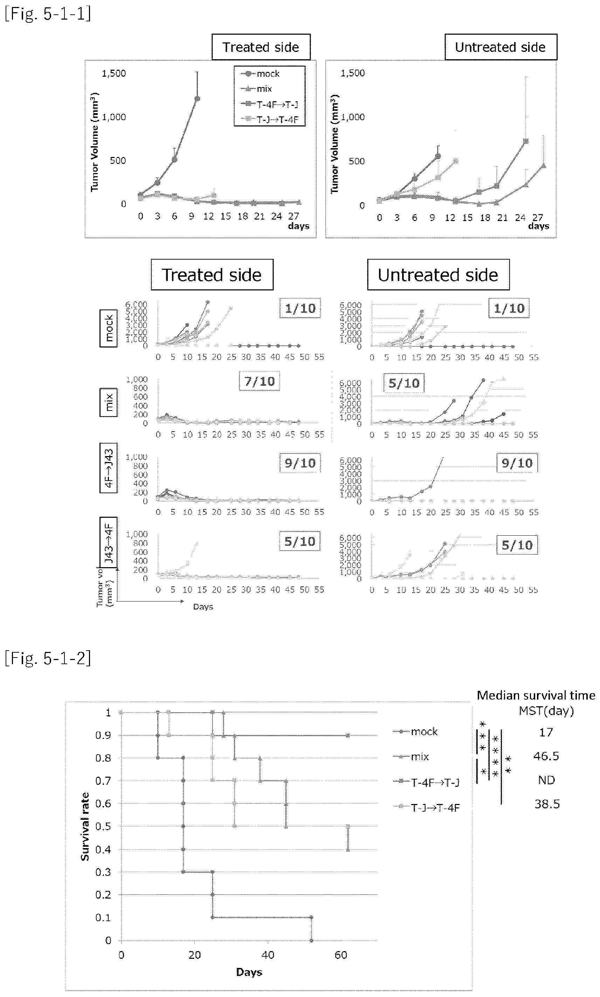

[Fig. 5-1-1] Therapeutic efficacies of administration of two-ingredient cocktail (mix) of HSV-1 with anti-CTLA-4 antibody expression (T-4F10HA) and HSV-1 with anti-PD-1 antibody expression (T-J43) and sequential administration of them in Neuro2a/A/J mouse bilateral subcutaneous tumor model. The ratios of mice that showed tumor disappearance (defined as tumor size of 50 mm³ or smaller) on day 48 are shown.
[Fig. 5-1-2] Kaplan-Meier survival curves. Survival curves obtained by observation ended upon death or when the maximum tumor diameter reached 20 mm (during observation). *, P < 0.05; **, P < 0.01; ***, P < 0.001; ****, P<0.0001; log-rank test

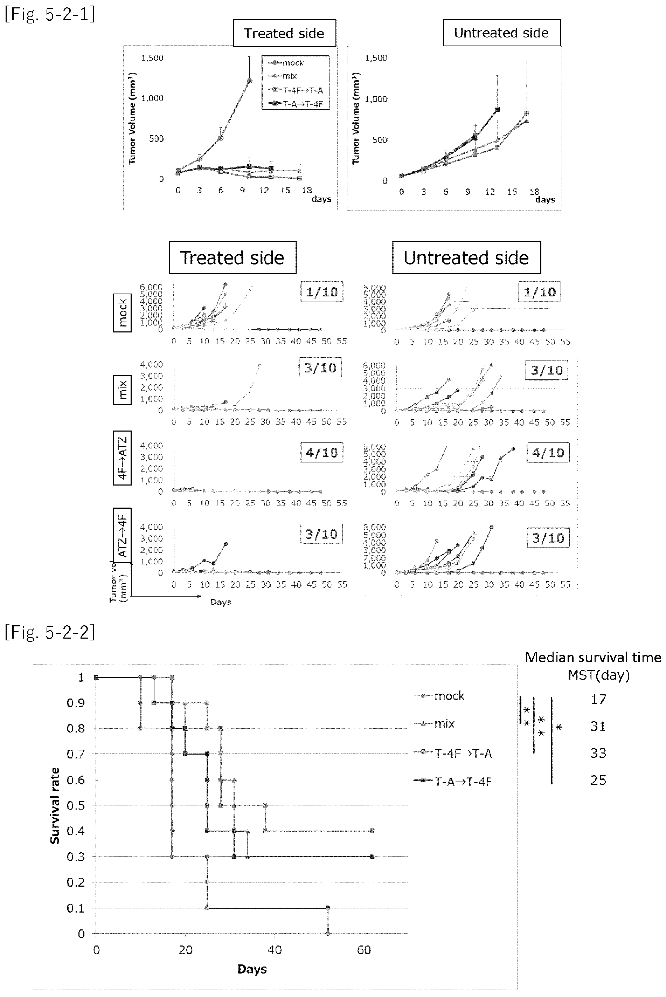

[Fig. 5-2-1] Therapeutic efficacies of administration of two-ingredient cocktail (mix) of HSV-1 with anti-CTLA-4 antibody expression (T-4F10HA) and HSV-1 with anti-PD-L1 antibody expression (T-ATZ) and sequential administration of them in Neuro2a/A/J mouse bilateral subcutaneous tumor model. The ratios of mice that showed tumor disappearance (defined as tumor size of 50 mm³ or smaller) on day 48 are shown.
[Fig. 5-2-2] Kaplan-Meier survival curves. Survival curves obtained by observation ended upon death or when the maximum tumor diameter reached 20 mm (during observation). *, P<0.05; **, P<0.01; log-rank test.

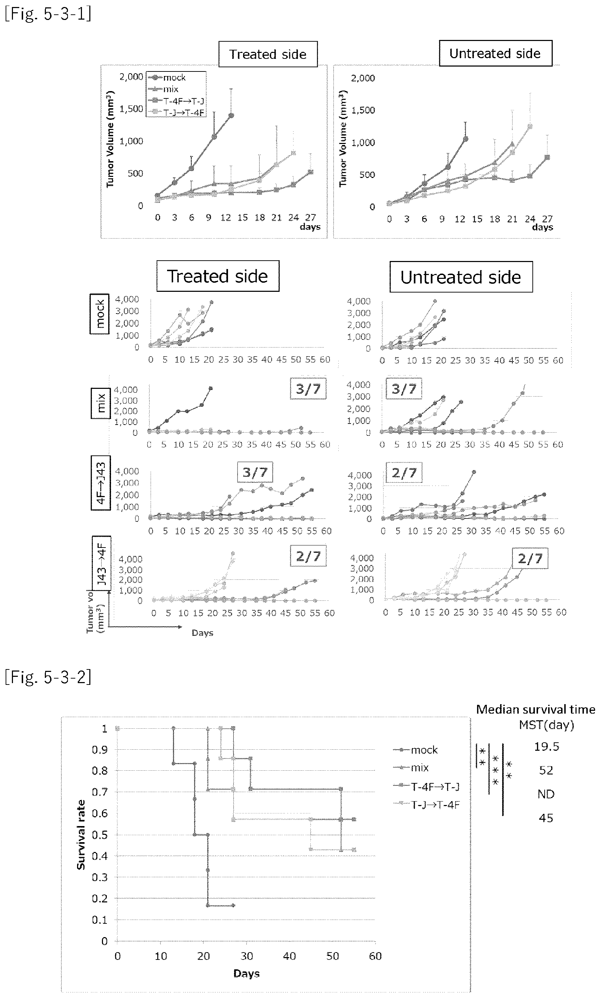

[Fig. 5-3-1] Therapeutic efficacies of administration of two-ingredient cocktail (mix) of HSV-1 with anti-CTLA-4 antibody expression (T-4F10HA) and HSV-1 with anti-PD-1 antibody expression (T-J43) and sequential administration of them in Clone M3/DBA/2 mouse bilateral subcutaneous tumor model. The ratios of mice that showed tumor disappearance (defined as tumor size of 50 mm³ or smaller) on day 50 are shown.
[Fig. 5-3-2] Kaplan-Meier survival curves. Survival curves obtained by observation ended upon death or when the maximum tumor diameter reached 20 mm (during observation). *, P<0.05; **, P<0.01; ***, P<0.001; log-rank test.

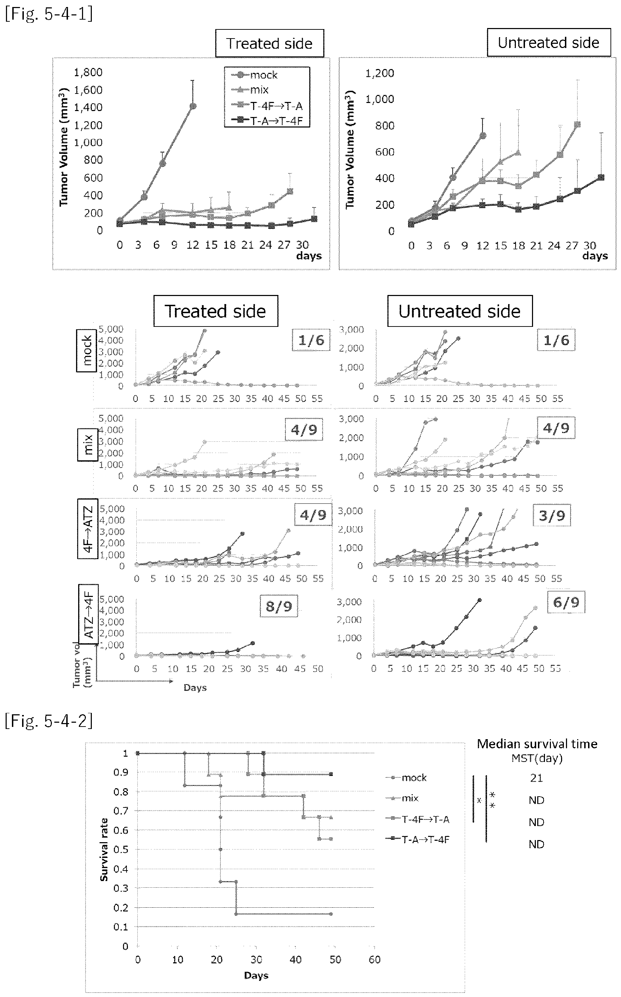

[Fig. 5-4-1] Therapeutic efficacies of administration of two-ingredient cocktail (mix) of HSV-1 with anti-CTLA-4 antibody expression (T-4F10HA) and HSV-1 with anti-PD-L1 antibody expression (T-ATZ) and sequential administration of them in Clone M3/DBA/2 mouse bilateral subcutaneous tumor model. The ratios of mice that showed tumor disappearance (defined as tumor size of 50 mm³ or smaller) on day 49 are shown.
[Fig. 5-4-2] Kaplan-Meier survival curves. Survival curves obtained by observation ended upon death or when the maximum tumor diameter reached 20 mm (under observation). *, P < 0.05; **, P < 0.01; log-rank test.
[Fig. 6-1] Amino acid sequence for expressing anti-human PD-1 antibody (derived from nivolumab). The constant region of the H chain was derived from human IgG4. The constant region of the L chain was derived from human κ chain. Underlined portions indicate hypervariable regions predicted by IMGT/DomainGapAlign.
[Fig. 6-2-1] Comparison of anti-PD-1 antibody expression levels. HEK293T cells were infected with T-Niv and T-01 at MOI=0.1. After 48 hours, supernatants were collected, concentrated by ultrafiltration (Amicon Ultra 30K), and subjected to A) ELISA for human Fc (human Fc antibody was immobilized, and detection was performed with HRP-human Fc antibody) and B) PD-1-binding ELISA (human PD-1 was immobilized, and detection was performed with HRP-human Fc antibody).
[Fig. 6-2-2] Comparison of anti-human PD-1 antibody expression levels in cells of various cell lines. PD-1-binding ELISA (human PD-1 was immobilized, and detection was performed with HRP-labeled anti-human IgG Fc antibody) was performed. Nivolumab was used as the standard.

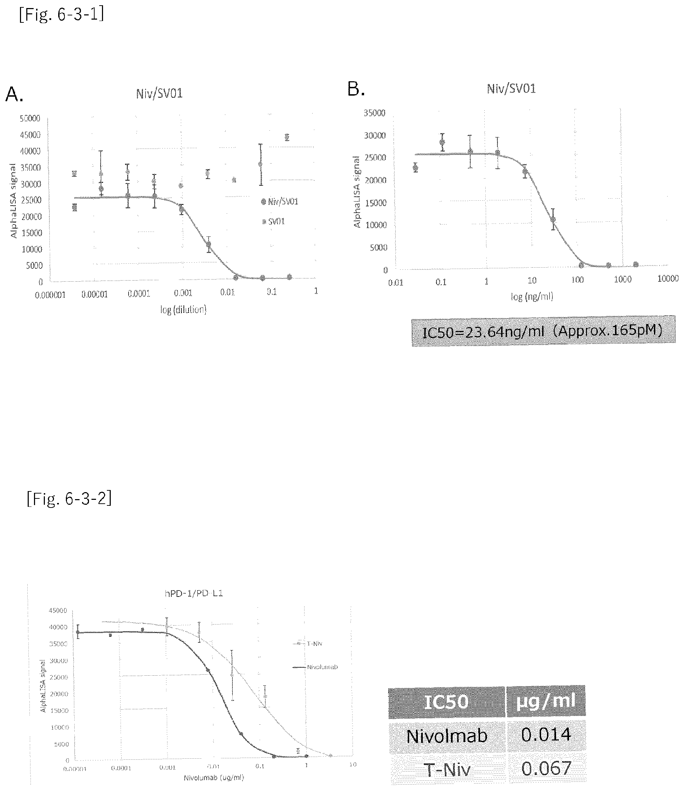

[Fig. 6-3-1] Results of PD-1/PD-L1 binding inhibitory activity assay. A) Transfection culture supernatants obtained with Niv/SV-01 and SV-01 (empty vector). B) The IC₅₀ value of Niv/SV-01 used in the experiment of A) calculated in terms of the concentration as human Fc was 23.64 ng/ml.
[Fig. 6-3-2] Results of PD-1/PD-L1 binding inhibitory activity assay.

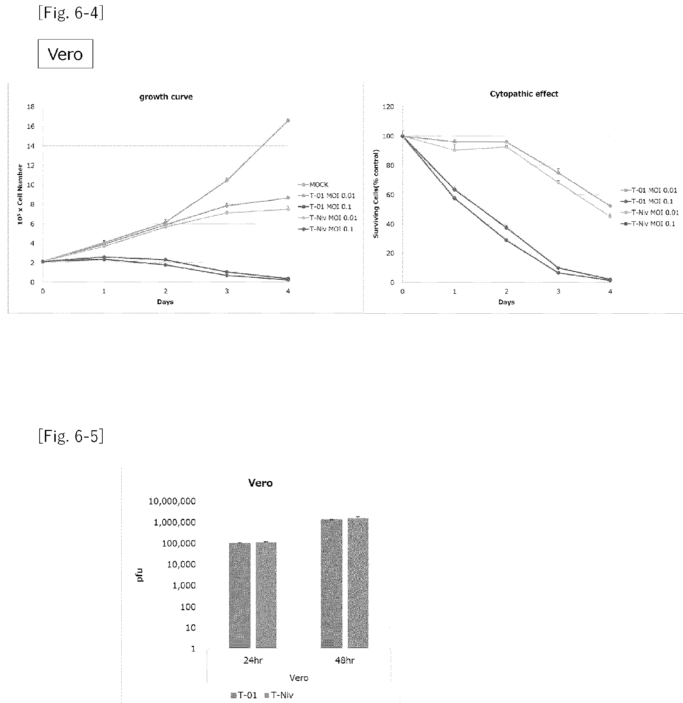

[Fig. 6-4] Cytotoxicity assay.
[Fig. 6-5] Replication assay.
[Fig. 6-6] Infection efficiencies of the prepared viruses (X-gal staining).
[Fig. 6-7] Structural confirmation of the prepared viruses by Southern blotting.
[Fig. 7-1-1] Amino acid sequence for expressing anti-human CTLA4 antibody (derived from ipilimumab). The constant region of the H chain was derived from human IgG1, and the constant region of the L chain was derived from the human κ chain. The underlined red characters indicate the hypervariable regions predicted by IMGT/DomainGapAlign.
[Fig. 7-1-2] Amino acid sequence for expressing anti-human CTLA4 antibody (derived from ticilimumab). The constant region of the H chain was derived from human IgG2, and the constant region of the L chain was derived from human κ chain. The underlined red characters indicate hypervariable regions predicted by IMGT/DomainGapAlign.
[Fig. 7-2-1] Comparison of anti-CTLA4 antibody expression levels. A) Culture supernatants of HEK293T cells transiently transfected with Ipi/SV-01 or Tic/SV-01 plasmid were concentrated and used as samples. A sample prepared in the same manner by using the SV-01 plasmid was used as a negative control. ELISA for human Fc was performed (human Fc antibody was immobilized, and detection was performed with HRP-human Fc antibody). B) HEK293T cells were infected with T-Ipi at MOI=1. After 48 hours, the supernatant was collected, concentrated by ultrafiltration (Amicon Ultra 30K), and subjected to CTLA-4-binding ELISA (human CTLA-4 was immobilized, and detection was performed with HRP-human Fc antibody; standard was ipilimumab). C) HEK293T cells were infected with T-Tic at MOI=1. After 48 hours, the supernatant was collected, concentrated by ultrafiltration (Amicon Ultra 30K), and subjected to CTLA-4 binding ELISA (human CTLA-4 was immobilized, and detection was performed with HRP-human Fc antibody; standard was ticilimumab).
[Fig. 7-2-2] Comparison of anti-human CTLA-4 antibody expression levels in cells of various cell lines. A) Expression with T-Ipi and B) Expression with T-Tic. Ipilimumab (A) and ticilimumab (B) were used as standards, respectively.

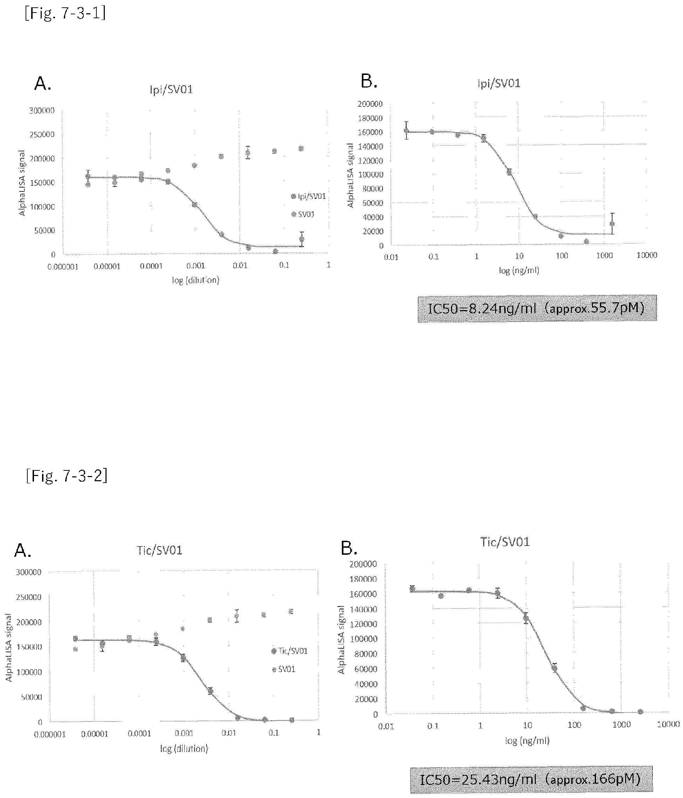

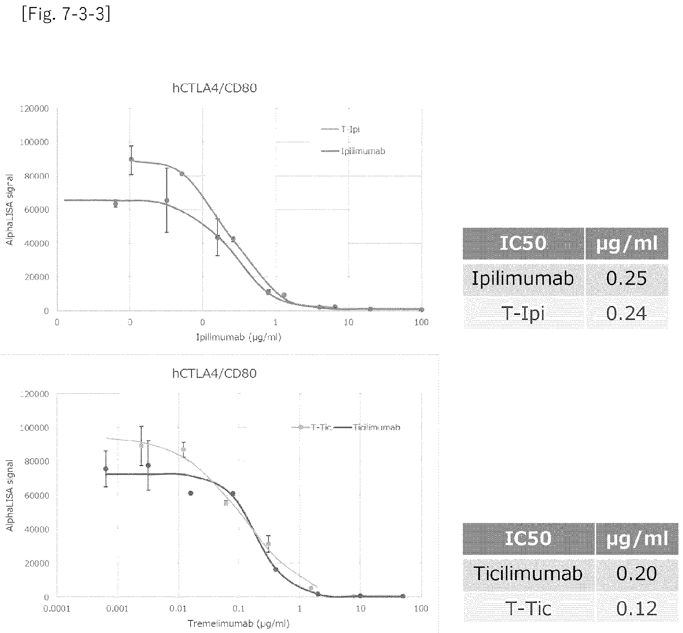

[Fig. 7-3-1] Results of CTLA-4/CD80(B7-1) binding inhibitory activity assay. A) Transfection culture supernatants obtained with Ipi/SV-01 and SV-01 (empty vector). B) The IC₅₀ value of Ipi/SV-01 used in the experiment of A) calculated in terms of the concentration as human Fc was 8.24 ng/ml.
[Fig. 7-3-2] Results of CTLA-4/CD80 (B7-1) binding inhibitory activity assay. A) Transfection culture supernatants obtained with Tic/SV-01 and SV-01 (empty vector). B) The IC₅₀ value of Tic/SV-01 used in the experiment of A) calculated in terms of the concentration as human Fc was 25.43 ng/ml.
[Fig. 7-3-3] Results of CTLA-4/CD80 binding inhibitory activity assay.

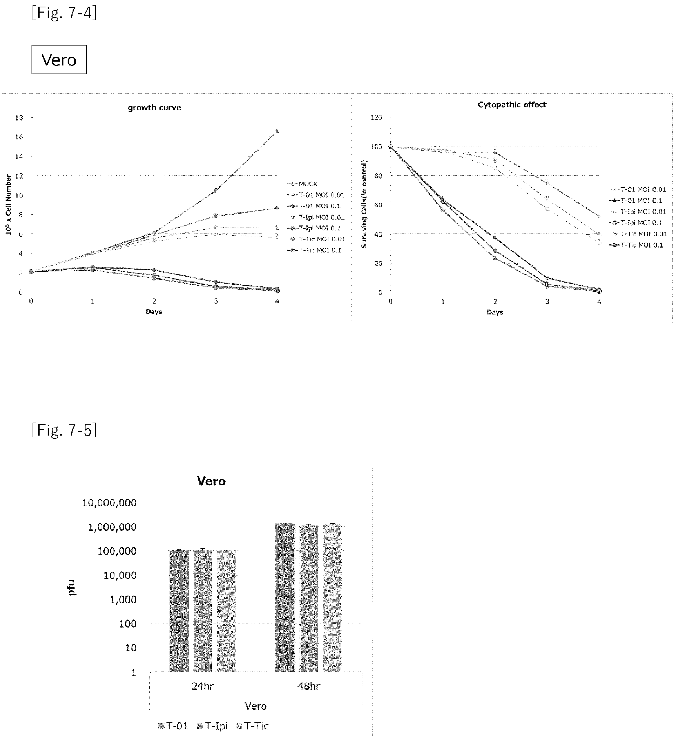

[Fig. 7-4] Cytotoxicity assay.
[Fig. 7-5] Replication assay.

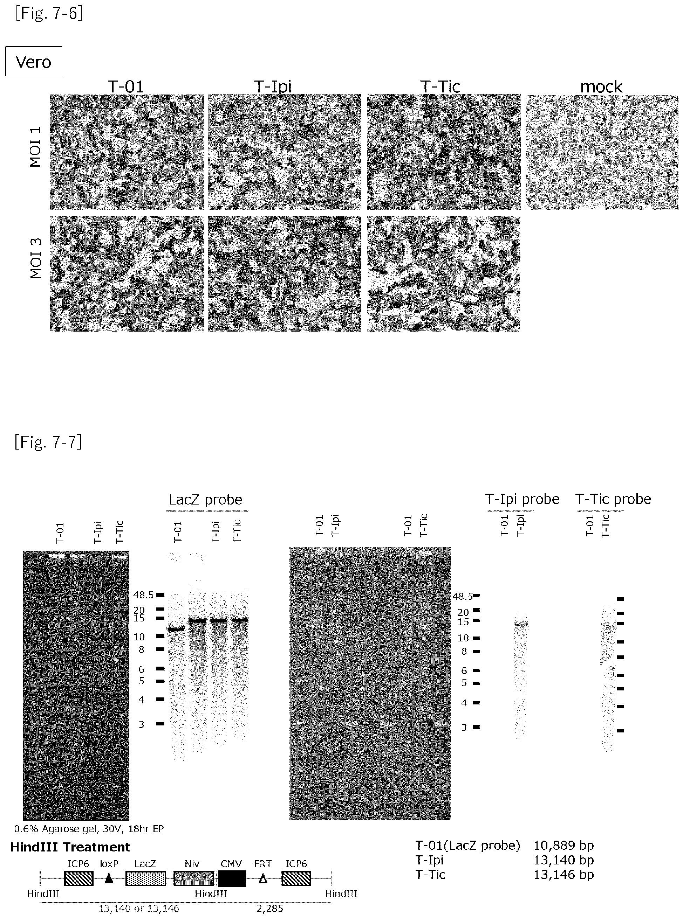

[Fig. 7-6] Infection efficiencies of the prepared viruses (X-gal staining).
[Fig. 7-7] Structural confirmation of the prepared viruses by Southern blotting.

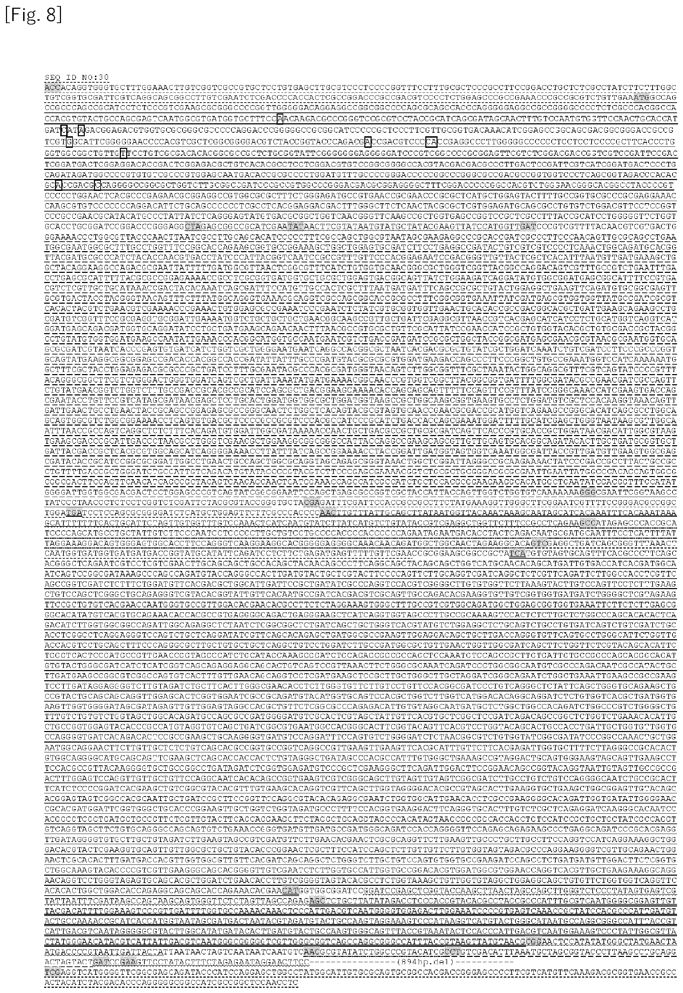

[Fig. 8] Nucleotide sequence of insertion site of gene encoding immune checkpoint inhibitor and upstream and downstream parts thereof.

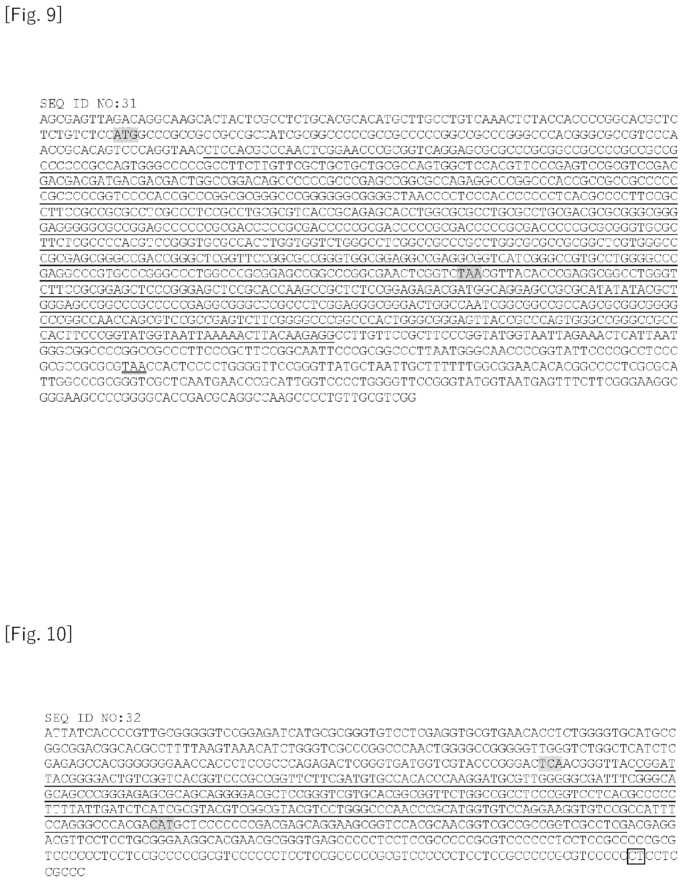

[Fig. 9] Nucleotide sequence of the γ34.5 gene and upstream and downstream parts thereof (within the TR_{L} region).
[Fig. 10] Nucleotide sequence of the α47 gene and upstream and downstream parts thereof.

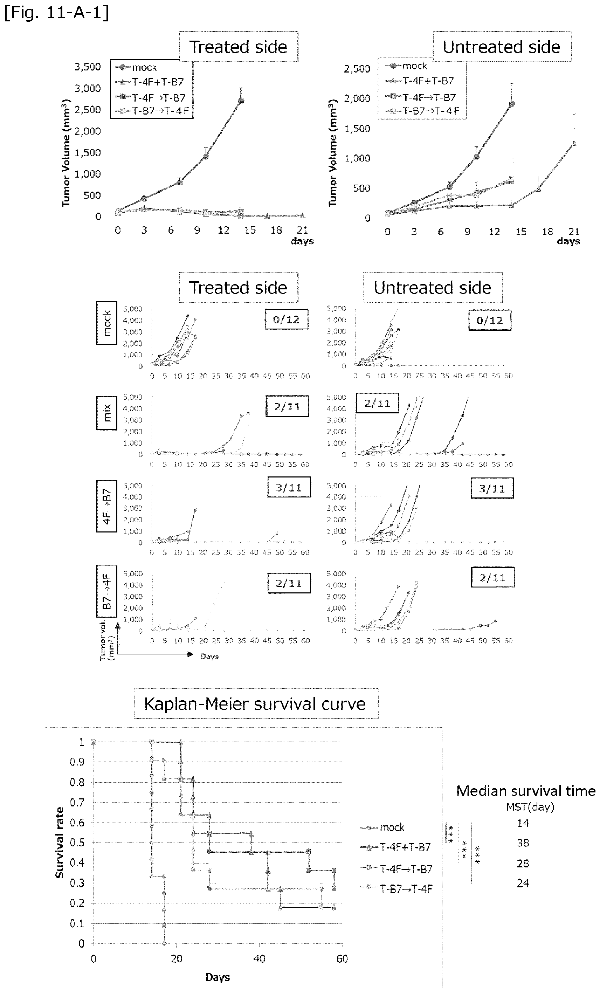

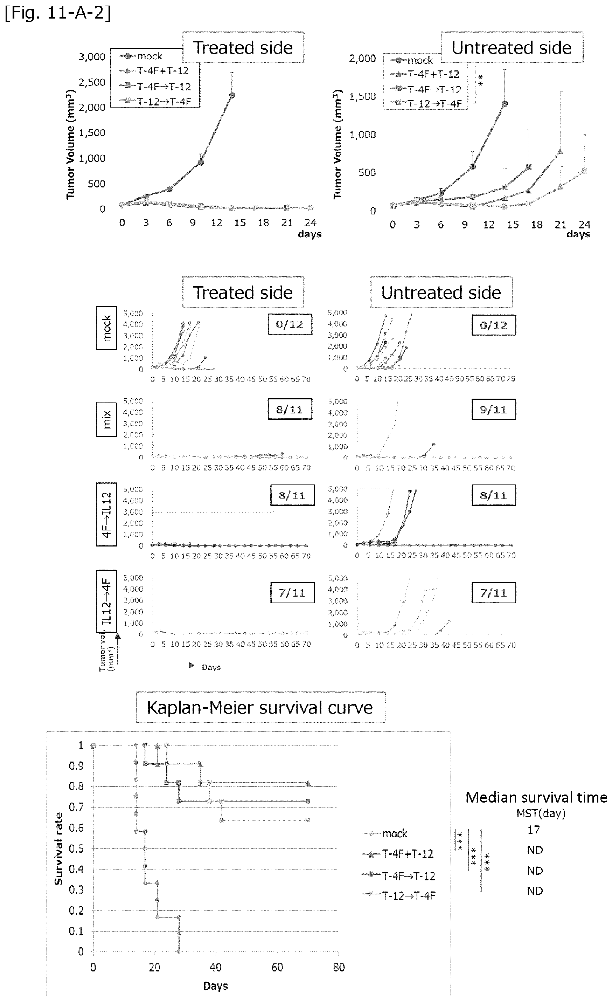

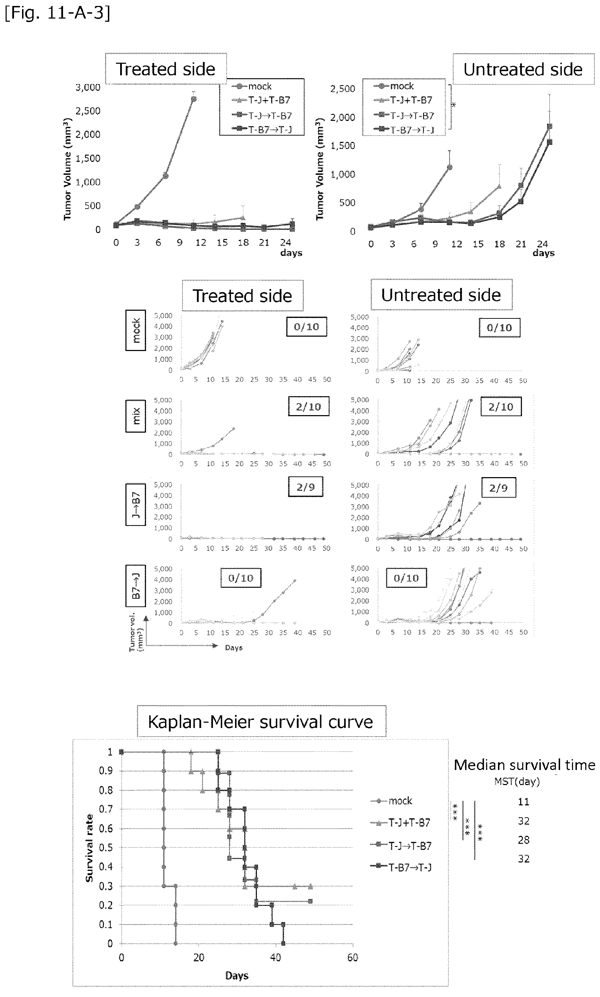

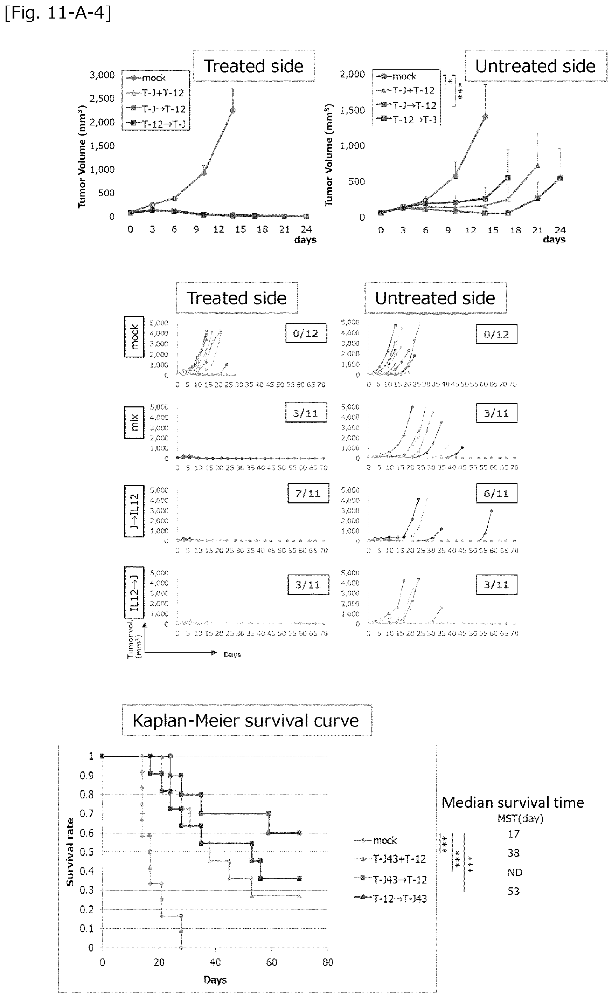

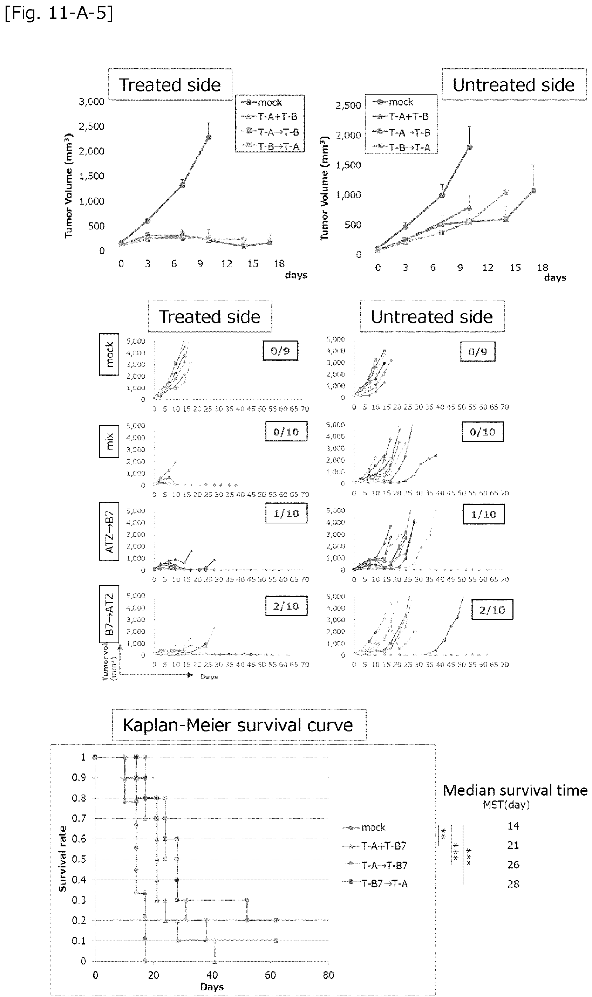

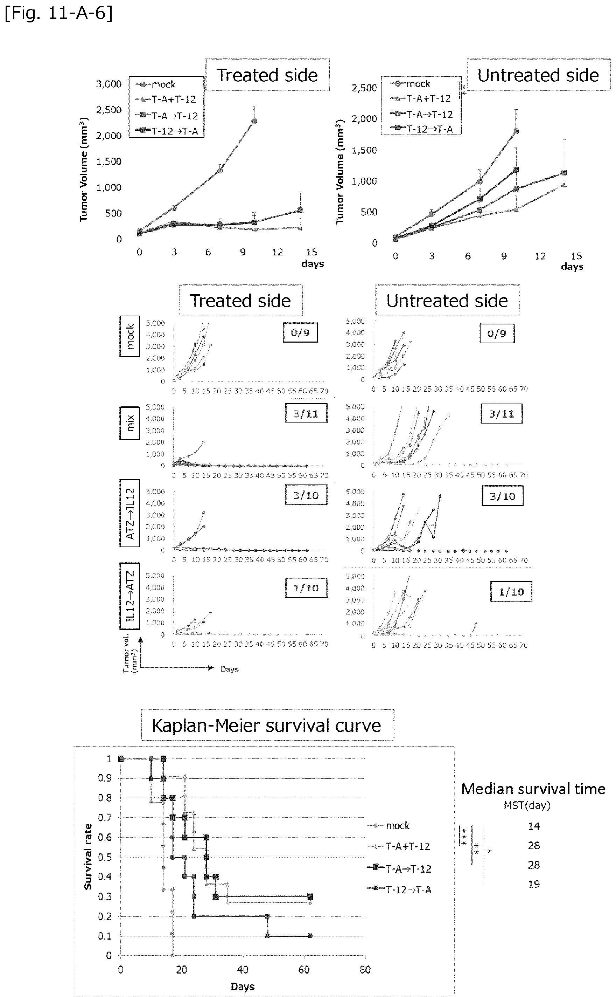

[Fig. 11-A-1] Effects of concurrent administration (mix) of HSV-1 with anti-CTLA-4 antibody expression (T-4F10HA) and HSV-1 with soluble B7-1 expression (T-mB7.1) and sequential administration of them in Neuro2a/mouse bilateral subcutaneous tumor model. Top: Treated side, all showed significant difference compared with the mock. Untreated side, all showed significant difference compared with the mock. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 58. ***, p<0.001; log-rank test
[Fig. 11-A-2] Effects of concurrent administration (mix) of HSV-1 with anti-CTLA-4 antibody expression (T-4F10HA) and HSV-1 with expression IL12 (T-mfIL12) and sequential administration of them in Neuro2a/mouse bilateral subcutaneous tumor model. Top: Treated side, all showed significant difference compared with the mock. Untreated side; **, p < 0.01; two-way ANOVA and Bonferroni comparison test. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 70. ***, p < 0.001; log-rank test.
[Fig. 11-A-3] Effects of concurrent administration (mix) of HSV-1 with anti-PD-1 antibody expression (T-J43) and HSV-1 with soluble B7-1 expression (T-mB7.1) and sequential administration of them in Neuro2a/mouse bilateral subcutaneous tumor model. Top: Treated side, all showed significant difference compared with the mock. Untreated side; *, p<0.05; two-way ANOVA and Bonferroni comparison test. Middle: The ratios of mice that showed tumor disappearance (defined as tumor size of 50 mm³ or smaller) on day 49 are shown with rectangles enclosing them. Bottom: Survival curves obtained by observation ended upon death or when the maximum tumor diameter reached 20 mm on either side. The observation ended on day 49. ***, p<0.001; log-rank test.
[Fig. 11-A-4] Effects of concurrent administration (mix) of HSV-1 with anti-PD-1 antibody expression (T-J43) and HSV-1 with IL12 expression (T-mfIL12) and sequential administration of them in Neuro2a/mouse bilateral subcutaneous tumor model. Top: Treated side, all showed significant difference compared with the mock. Untreated side; *, p<0.05; ***, p<0.001; two-way ANOVA and Bonferroni comparison test. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 70. ***, p < 0.001; log-rank test.
[Fig. 11-A-5] Effects of concurrent administration (mix) of HSV-1 with anti-PD-L1 antibody expression (T-ATZ) and HSV-1 with soluble B7-1 expression (T-mB7.1) and sequential administration of them in the Neuro2a/mouse bilateral subcutaneous tumor model. Top: Treated side, all showed significant difference compared with the mock. Untreated side, all showed significant difference compared with the mock. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 62. **, p < 0.01; ***, p < 0.001; log-rank test.
[Fig. 11-A-6] Effects of concurrent administration (mix) of HSV-1 with anti-PD-L1 antibody expression (T-ATZ) and HSV-1 with IL12 expression (T-mfIL12) and sequential administration of them in Neuro2a/mouse bilateral subcutaneous tumor model. Top: Treated side, all showed significant difference compared with the mock. Untreated side; **, p<0.01; two-way ANOVA and Bonferroni comparison test. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 58. *, p < 0.05; **, p < 0.01; ***, p < 0.001; log-rank test.

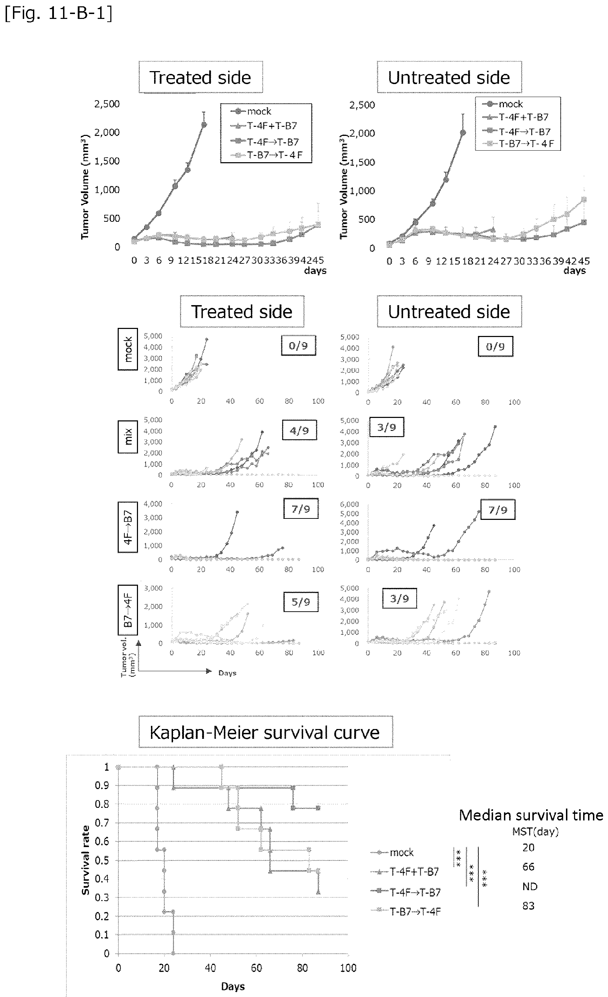

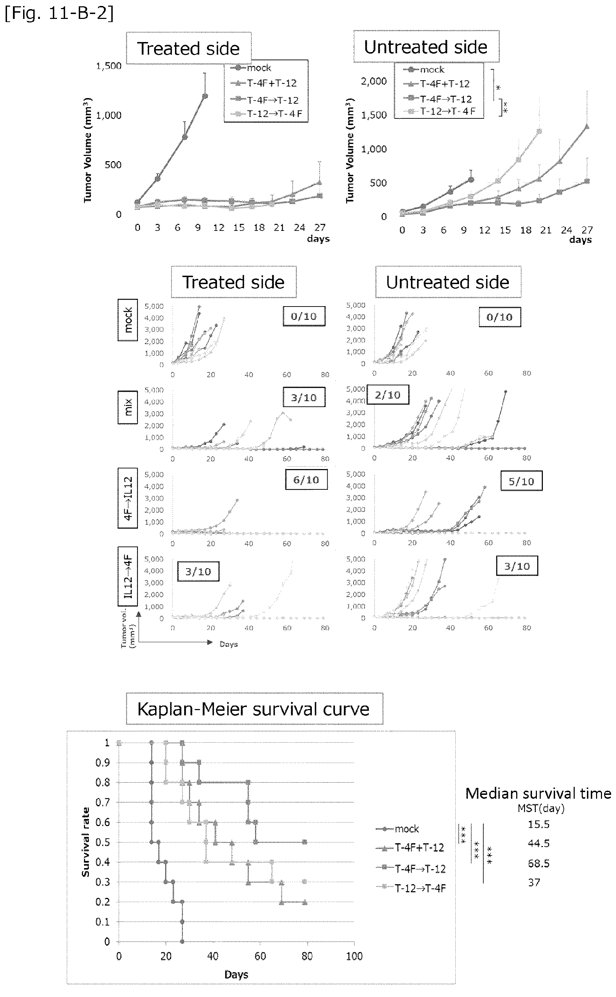

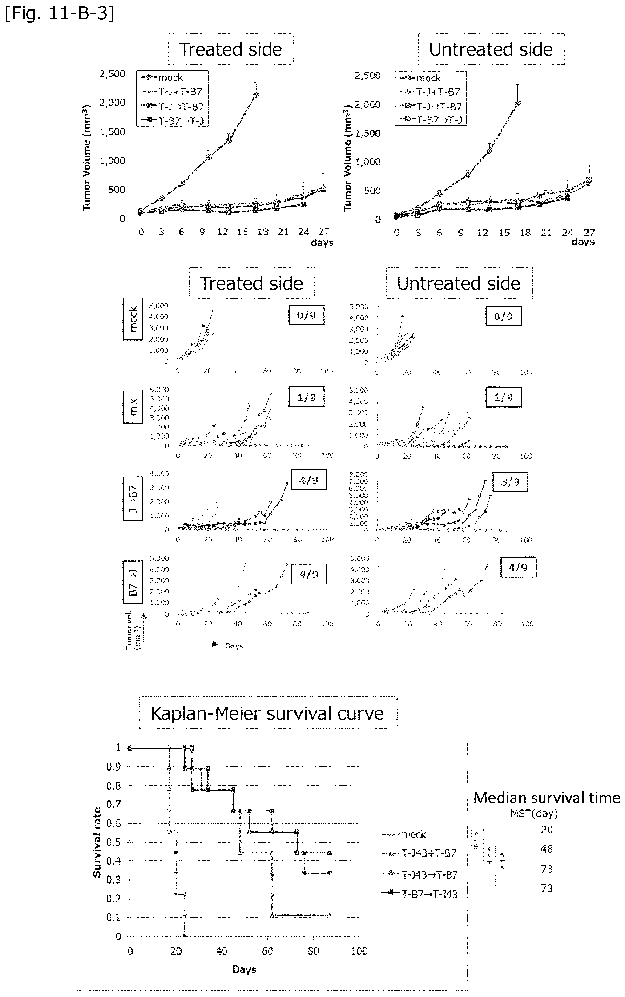

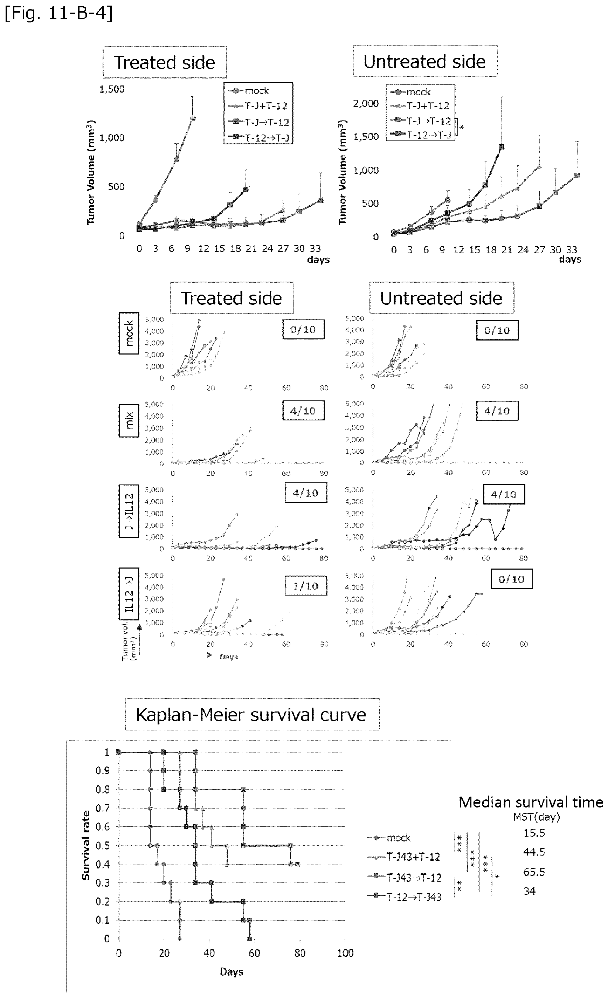

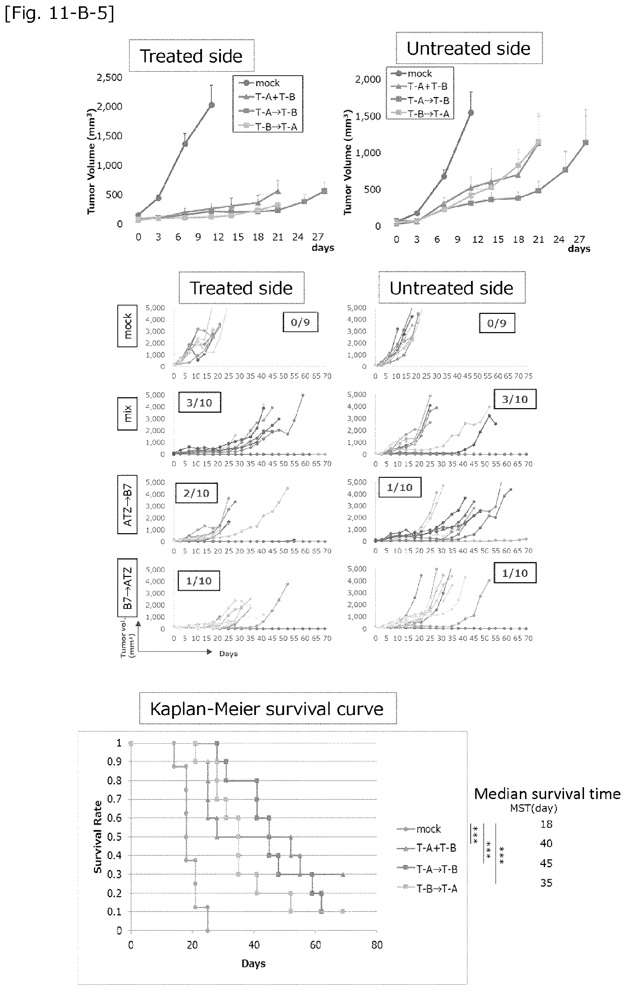

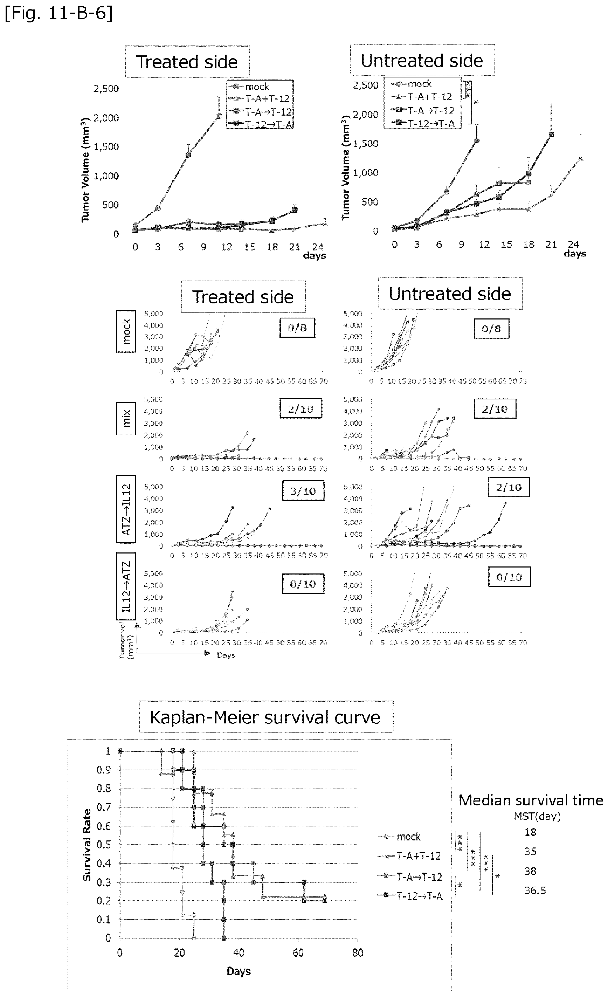

[Fig. 11-B-1] Effects of concurrent administration (mix) of HSV-1 with anti-CTLA-4 antibody expression (T-4F10HA) and HSV-1 with soluble B7-1 expression (T-mB.7) and sequential administration of them in Clone M3 mouse bilateral subcutaneous tumor model. Top: Treated side, all showed significant difference compared with the mock. Untreated side: all showed significant difference compared with the mock. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 87. ***, p<0.001; log-rank test.
[Fig. 11-B-2] Effects of concurrent administration (mix) of HSV-1 with anti-CTLA4 antibody expression (T-4F10HA) and HSV-1 with IL12 expression (T-mfIL12) and sequential administration of them in Clone M3 mouse bilateral subcutaneous tumor model. Top: Treated side, all showed significant difference compared with the mock. Untreated side; *, p<0.05; **, p<0.01; two-way ANOVA and Bonferroni comparison test. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 79. ***, p < 0.001; log-rank test.
[Fig. 11-B-3] Effects of concurrent administration (mix) of HSV-1 with anti-PD-1 antibody expression (T-J43) and HSV-1 with soluble B7-1 expression (T-mB7.1) and sequential administration of them in Clone M3 mouse bilateral subcutaneous tumor model. Top: Treated side, all showed significant difference compared with the mock. Untreated side: all showed significant difference compared with the mock. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 87. ***, p<0.001; log-rank test.
[Fig. 11-B-4] Effects of concurrent administration (mix) of HSV-1 with anti-PD-1 antibody expression (T-J43) and HSV-1 with IL12 expression (T-mfIL12) and sequential administration of them in Clone M3 mouse bilateral subcutaneous tumor model. Top: Treated side, all showed significant difference compared with the mock. Untreated side; *, p<0.05, two-way ANOVA and Bonferroni comparison test. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 79. *, p < 0.05; **, p < 0.01; ***, p < 0.001; log-rank test.
[Fig. 11-B-5] Effects of concurrent administration (mix) of HSV-1 with anti-PD-L1 antibody expression (T-ATZ) and HSV-1 with soluble B7-1 expression (T-mB7.1) and sequential administration of them in Clone M3 mouse bilateral subcutaneous tumor model. Top: Treated side, all showed significant difference compared with the mock. Untreated side, all showed significant difference compared with the mock. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 58. ***, p<0.001; log-rank test.
[Fig. 11-B-6] Effects of concurrent administration (mix) of HSV-1 with anti-PD-L1 antibody expression (T-ATZ) and HSV-1 with IL12 expression (T-mfIL12) and sequential administration of them in Clone M3 mouse bilateral subcutaneous tumor model. Top: Treated side, all showed significant difference compared with the mock. Untreated side; *, p<0.05; ***, p<0.001; two-way ANOVA and Bonferroni comparison test. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 69. *, p < 0.05; ***, p < 0.001; log-rank test.

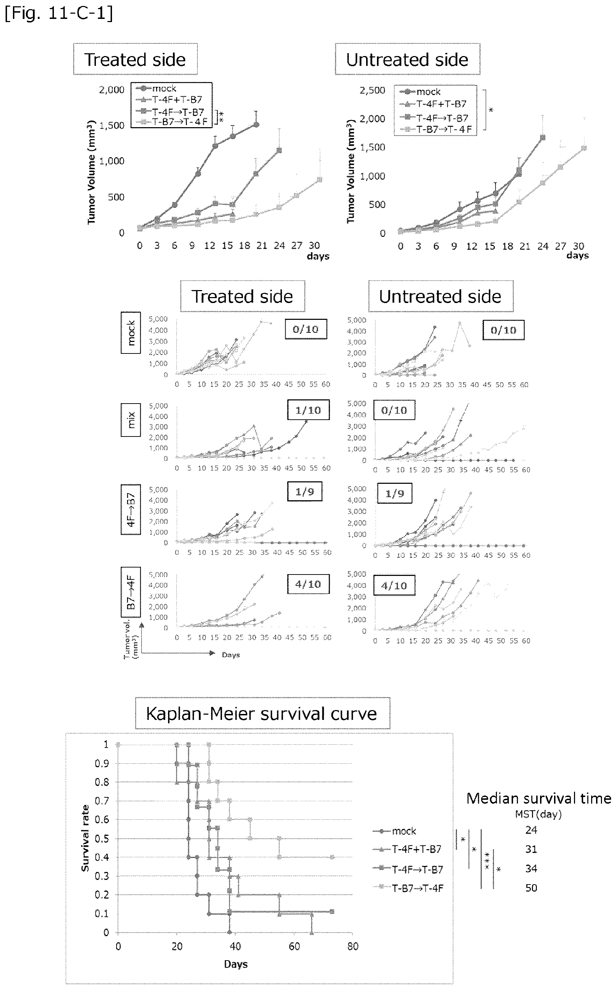

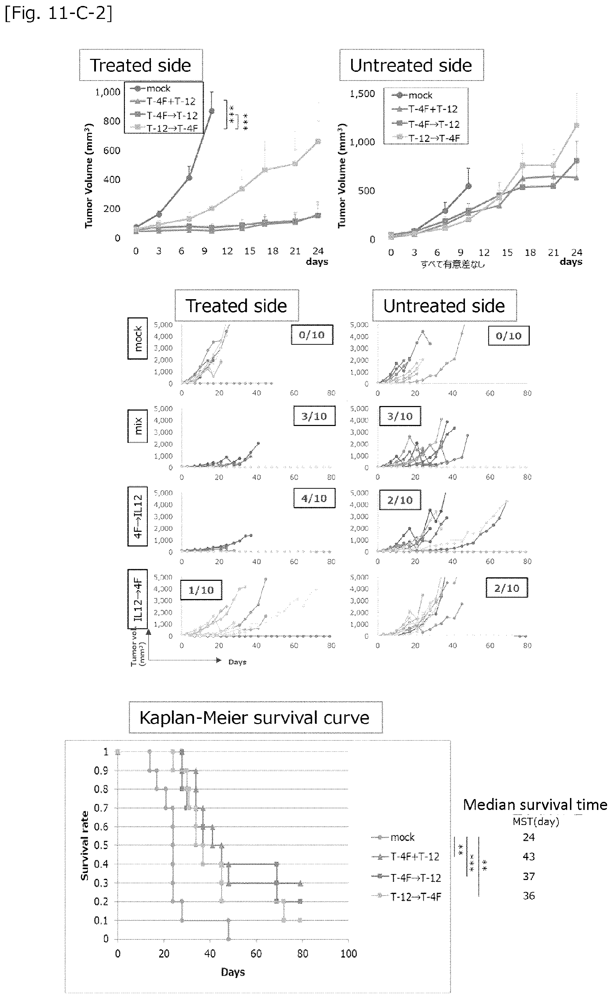

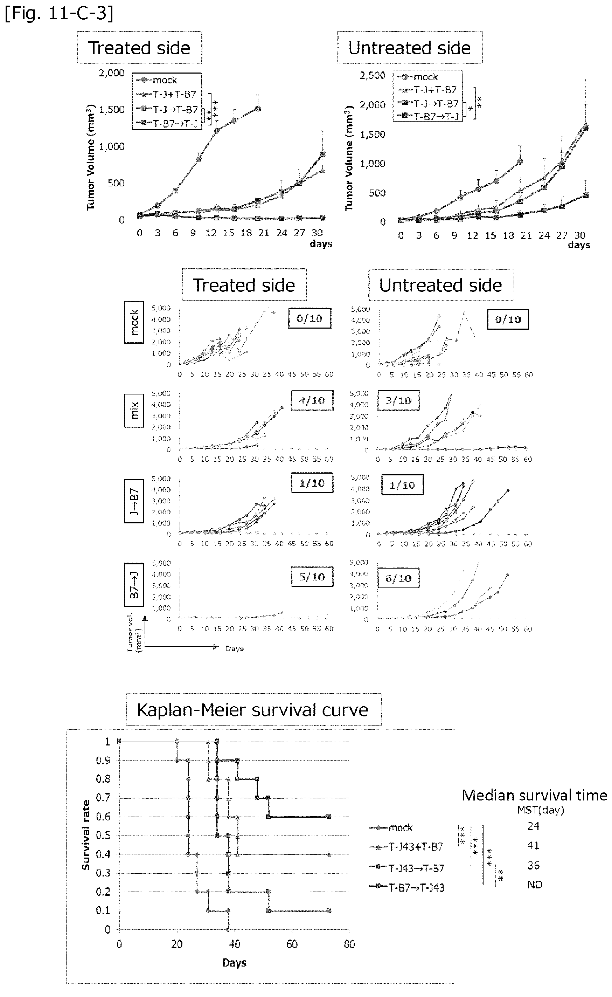

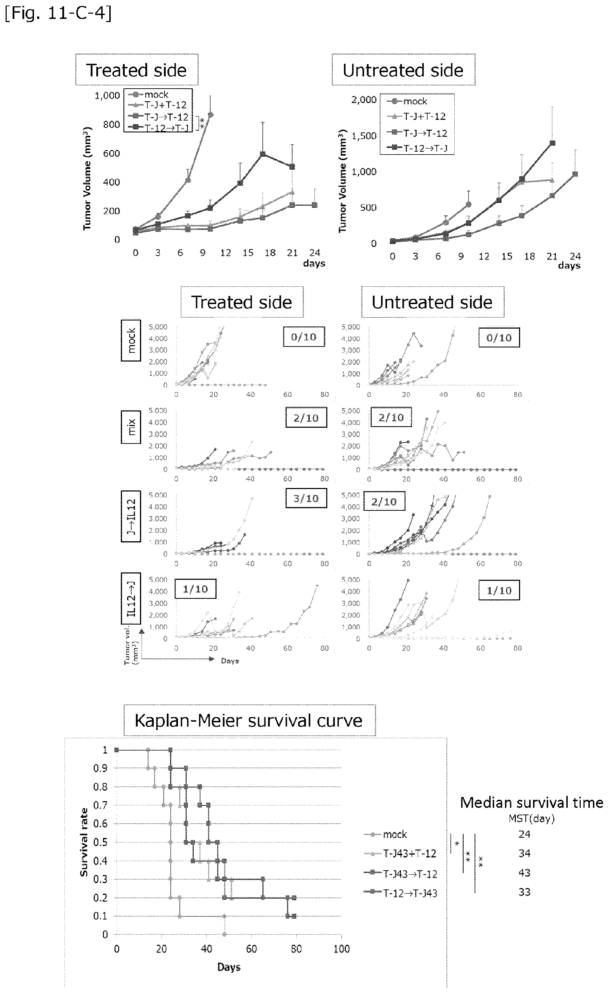

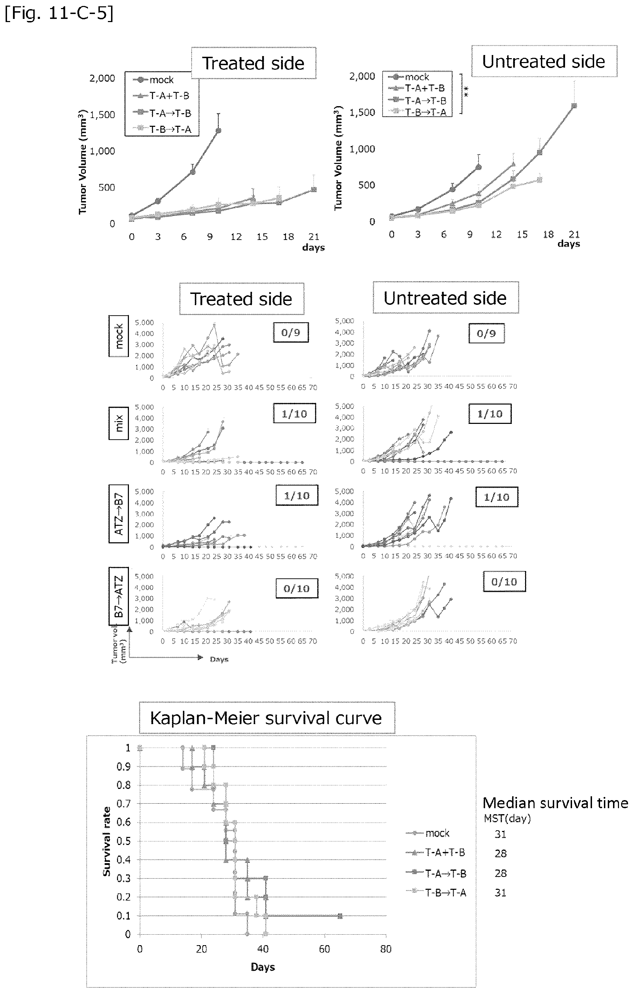

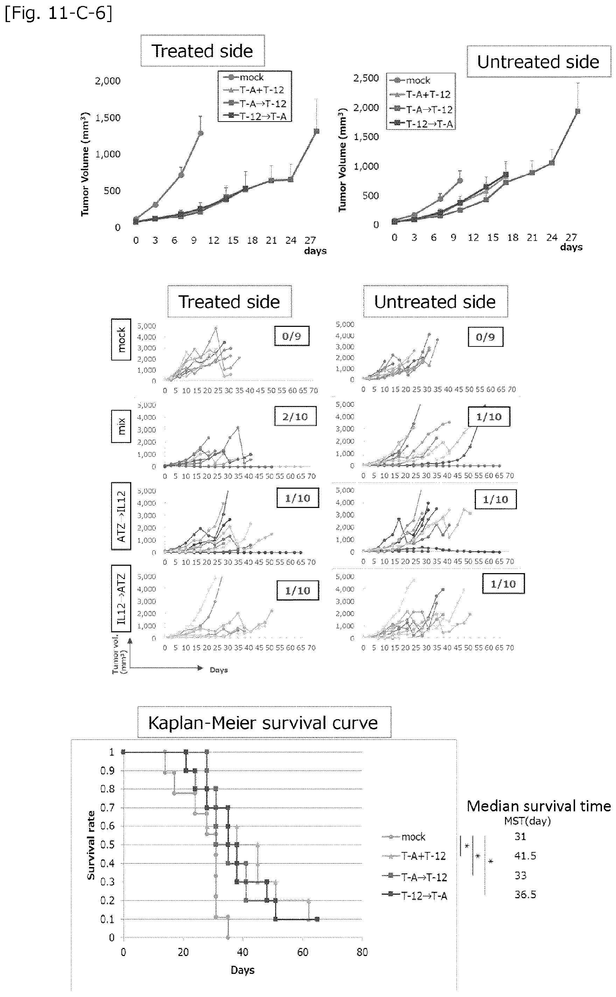

[Fig. 11-C-1] Effects of concurrent administration (mix) of HSV-1 with anti-CTLA-4 antibody expression (T-4F10HA) and HSV-1 with soluble B7-1 expression (T-mB7.1) and sequential administration of them in Renca/Balb/c mouse bilateral subcutaneous tumor model. Top: Treated side, all showed significant difference compared with the mock. Untreated side; *, p<0.05; **, p<0.01; two-way ANOVA and Bonferroni comparison test. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 73. *, p < 0.05; ***, p < 0.001; log-rank test.
[Fig. 11-C-2] Effects of concurrent administration (mix) of HSV-1 with anti-CTLA-4 antibody expression (T-4F10HA) and HSV-1 with IL12 expression (T-mfIL12) and sequential administration of them in Renca/Balb/c mouse bilateral subcutaneous tumor model. Top: Treated side, all showed significant difference compared with the mock except for 12 -> 4F. ***, p<0.001; two-way ANOVA and Bonferroni comparison test. Untreated side: all showed no significant difference. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 79. **, p < 0.01; ***, p < 0.001; log-rank test.
[Fig. 11-C-3] Effects of concurrent administration (mix) of HSV-1 with anti-PD-1 antibody expression (T-J43) and HSV-1 with soluble B7-1 expression (T-mB7.1) and sequential administration of them in Renca/Balb/c mouse bilateral subcutaneous tumor model. Top: *, p<0.05; **, p<0.01; ***, p<0.001; two-way ANOVA and Bonferroni comparison test. Middle: The ratios of mice that showed tumor disappearance (defined as tumor size of 50 mm³ or smaller) on day 59 are shown with rectangles enclosing them. Bottom: Survival curves obtained by observation ended upon death or when the maximum tumor diameter reached 20 mm on either side. The observation ended on day 73. **, p < 0.01; ***, p < 0.001; log-rank test.
[Fig. 11-C-4] Effects of concurrent administration (mix) of HSV-1 with anti-PD-1 antibody expression (T-J43) and HSV-1 with IL12 expression (T-mfIL12) and sequential administration of them in Renca/Balb/c mouse bilateral subcutaneous tumor model. Top: Treated side, all showed significant difference compared with the mock except for 12 -> J. **, p<0.01; two-way ANOVA and Bonferroni comparison test. Untreated side: all showed no significant difference. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 79. *, p < 0.05; **, p < 0.001; log-rank test.
[Fig. 11-C-5] Effects of concurrent administration (mix) of HSV-1 with anti-PD-L1 antibody expression (T-ATZ) and HSV-1 with soluble B7-1 expression (T-mB7.1) and sequential administration of them in Renca/Balb/c mouse bilateral subcutaneous tumor model. Top: Treated side, all showed significant difference compared with the mock. Untreated side; **, p<0.01; two-way ANOVA and Bonferroni comparison test. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 65. No significant difference; log-rank test.
[Fig. 11-C-6] Effects of concurrent administration (mix) of HSV-1 with anti-PD-L1 antibody expression (T-ATZ) and HSV-1 with IL12 expression (T-mfIL12) and sequential administration of them in Renca/Balb/c mouse bilateral subcutaneous tumor model. Top: Treated side, all showed significant difference compared with the mock. Untreated side, all showed no significant difference. Middle: The ratios of mice that showed tumor disappearance (defined as tumor size of 50 mm³ or smaller) on day 58 are shown with rectangles enclosing them. Bottom: Survival curves obtained by observation ended upon death or when the maximum tumor diameter reached 20 mm on either side. The observation ended on day 65. *, p<0.05; log-rank test.

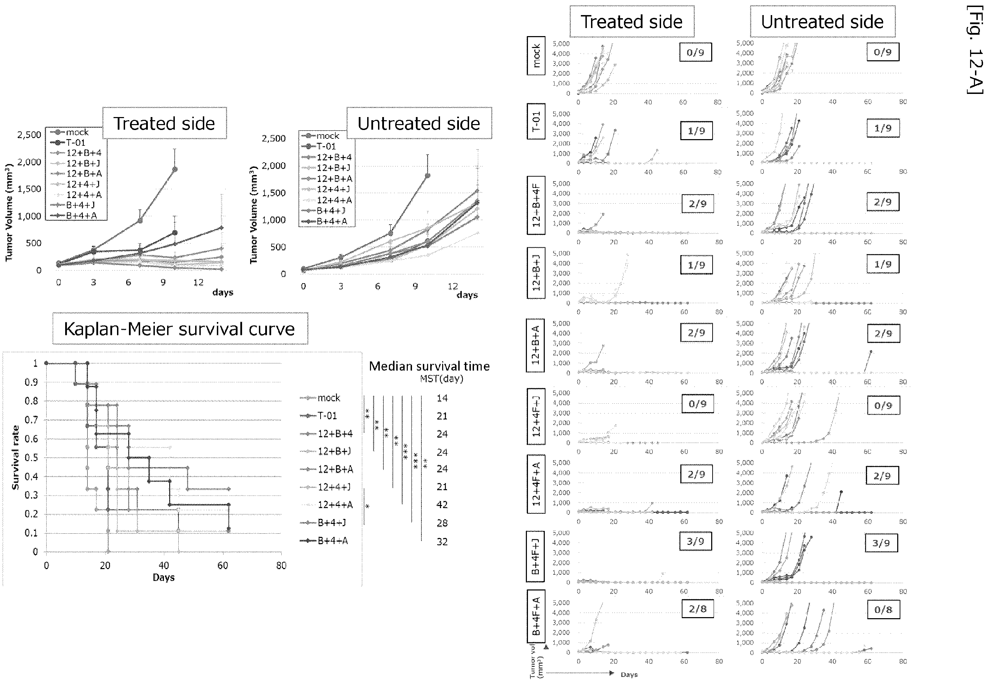

[Fig. 12-A] Effect of three-HSV-1 cocktail in Neuro2a/mouse bilateral subcutaneous tumor model. Upper left: Treated side, all showed significant difference compared with the mock. Untreated side, all showed no significant difference. Lower left: Survival curves obtained by observation ended upon death or when the maximum tumor diameter reached 20 mm on either side. The observation ended on day 62. *, p<0.05; **, p<0.01; ***, p<0.001; log-rank test. Right: The ratios of mice that showed tumor disappearance (defined as tumor size of 50 mm³ or smaller) on day 58 are shown with rectangles enclosing them.

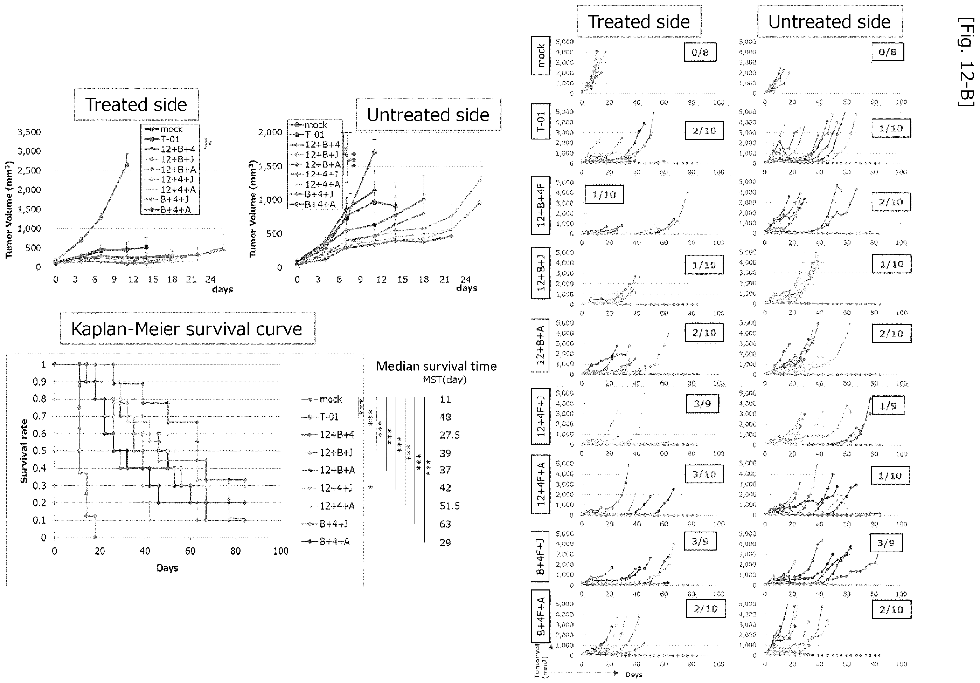

[Fig. 12-B] Effect of three-ingredient cocktail of HSV-1 in Clone M3/mouse bilateral subcutaneous tumor model. Upper left: Treated side; all showed significant difference compared with the mock. Untreated side: all showed significant difference compared with the mock except for T-01 and 12+B+4. *, p < 0.05; **, p < 0.01; ***, p < 0.001; two-way ANOVA and Bonferroni comparison test. Lower left: Survival curves obtained by observation ended upon death or when the maximum tumor diameter reached 20 mm on either side. The observation ended on day 84. *, p < 0.05; ***, p < 0.001; log-rank test. Right: The ratios of mice that showed tumor disappearance (defined as tumor size of 50 mm³ or smaller) on day 60 are shown with rectangles enclosing them. 12=T-mfIL12 (IL12), B=T-mB7.1 (soluble B7-1), 4=T-4F10HA (anti-CTLA4 antibody), J=T-J43 (anti-PD-L1 antibody), A=T-ATZ (anti-PD-1 antibody).

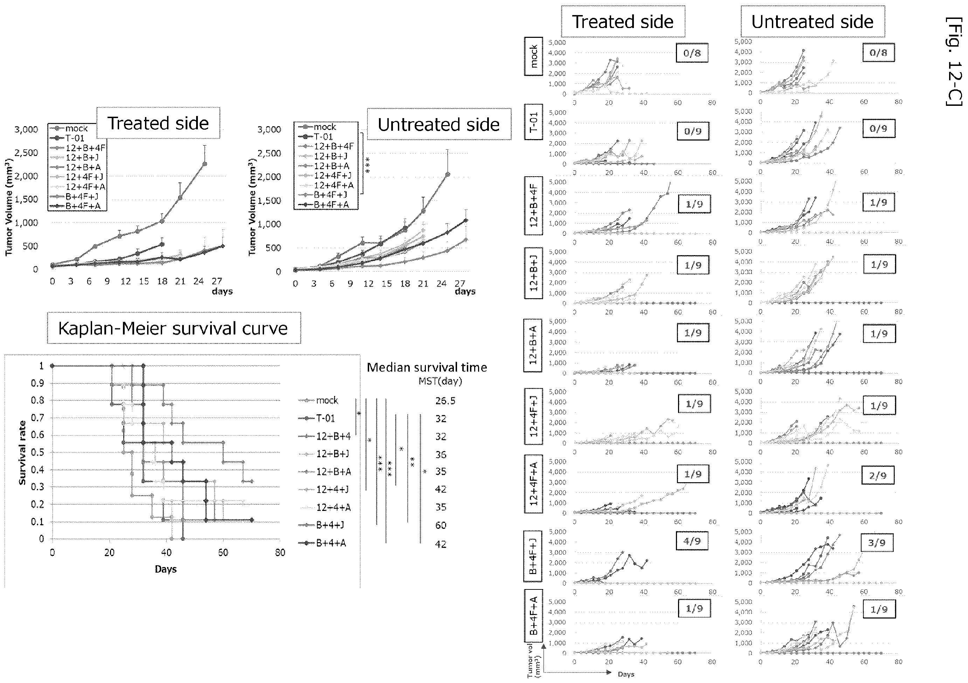

[Fig. 12-C] Effect of the three-ingredient cocktail of HSV-1 in Renca/mouse bilateral subcutaneous tumor model. Upper left: Treated side, all showed significant difference compared with the mock. Untreated side: all showed significant difference compared with the mock except for T-01. ***, p<0.001; two-way ANOVA and Bonferroni comparison test. Lower left: Survival curves obtained by observation ended upon death or when the maximum tumor diameter reached 20 mm on either side. The observation ended on day 70. *, p < 0.05; ***, p < 0.001; log-rank test. Right: The ratios of mice that showed tumor disappearance (defined as tumor size of 50 mm³ or smaller) on day 60 are shown with rectangles enclosing them. 12=T-mfIL12 (IL12), B=T-mB7.1 (soluble B7-1), 4=T-4F10HA (anti-CTLA4 antibody), J=T-J43 (anti-PD-L1 antibody), A=T-ATZ (anti-PD-1 antibody).

### Modes for Carrying out the Invention

The present invention relates to an oncolytic herpes simplex virus (HSV) having a gene encoding an immune checkpoint inhibitor. Hereafter, the present invention will be explained in detail with reference to aspects and embodiments of the present invention.

### (Gene encoding immune checkpoint inhibitor)

One aspect of the present invention relates to HSV having a gene encoding an immune checkpoint inhibitor.

In one embodiment, the immune checkpoint inhibitor can bind to an immune checkpoint protein (also referred to as an immune checkpoint regulator) or a protein that interacts therewith (e.g., receptor) with sufficient affinity and specificity, and inhibit transmission of activity suppressing signals by the immune checkpoint protein through that binding. The immune checkpoint inhibitor is preferably an antibody or fragment thereof that binds to an immune checkpoint protein or a protein that interacts therewith.

In one embodiment, the immune checkpoint inhibitor is an antibody directed to PD-1 (programmed death 1), CTLA-4 (cytotoxic T-lymphocyte-associated antigen 4), TIM-3 (T-cell immunoglobulin and mucin containing protein-3), LAG-3 (lymphocyte activation gene-3), B7-H3, B7-H4, B7-H5 (VISTA, V-domain Ig Suppressor of T cell Activation), TIGIT (T cell immunoreceptor with Ig and ITIM domain), PD-L1 (B7-H1), or PD-L2 (B7-H2), or a fragment thereof. It is preferably an antibody directed to any selected from PD-1, PD-L1, and CTLA-4, or a fragment thereof.

In one embodiment, the immune checkpoint inhibitor antibody or fragment thereof is an anti-PD-1 antibody such as nivolumab (Opdivo), pembrolizumab (Keytruda), spartalizumab, or cemiplimab; anti-PD-L1 antibody such as atezolizumab (Tecentriq), avelumab (Bavencio), or durvalumab (Imfinzi); anti-CTLA-4 antibody such as ticilimumab (tremelimumab), ipilimumab (Yervoy), or tremelimumab; anti-PD-1 antibody such as spartalizumab, or cemiplimab; or a fragment thereof.

In one embodiment, the type of the antibody is not particularly limited and may be any type, such as human antibody, humanized antibody, chimeric antibody, antibody derived from other animals (e.g., mouse antibody, rat antibody, rabbit antibody, sheep antibody, camel antibody, bird antibody), etc. However, it is preferred that the antibody be derived from the same organism as that of the tumor targeted for oncolysis by the oncolytic HSV having the gene encoding that antibody. For example, when the oncolytic HSV is intended for use in humans, a human antibody or humanized antibody directed to a human immune checkpoint protein or protein that interacts therewith is preferred.

In one embodiment, the immune checkpoint inhibitor may be an antibody fragment. The term antibody fragment means either an antibody fragment itself or an antibody fragment conjugated to any arbitrary molecule, provided it recognizes the same epitope as the original antibody. It is not particularly limited so long as it contains the complementarity-determining region (CDR) of the original antibody, and specific examples includes Fab fragment composed of V_{L}, V_{H}, C_{L} and C_{H} regions; Fab' fragment that is Fab fragment further including a part of the hinge region; F(ab')₂ fragment consisting of two Fab fragments linked by disulfide bonds at the hinge regions; Fv consisting of V_{L} and V_{H}; scFv, which is a single-chain antibody formed by linking V_{L} and V_{H} with an artificial polypeptide linker; sdFv; diabody; and sc(Fv)2. However, the antibody fragment is not limited to these, and is not particularly limited so long as it has the two variable regions constituting the antigen-binding site of antibody, the heavy chain variable region (V_{H}) and the light chain variable region (V_{L}).

In one embodiment, scFv can be prepared by linking V_{H} and V_{L} using a known linker (e.g., GS linker (SEQ ID NO: 11) etc.).

In one embodiment, the antibody or fragment thereof preferably shows a long half-life within tumor cells. For example, compared with single-chain antibodies, double-chain antibodies exhibit extended half-lives due to the presence of the Fc portion, and are therefore expected to enable antibody-dependent immune responses.

In one embodiment, HSV having a gene encoding an immune checkpoint inhibitor is a virus that has a gene encoding any of the following polypeptides (i) to (iii) as the V_{H} region:
(i) a polypeptide having the sequence of SEQ ID NO: 3, 11, 15, 18, 20, 22, 25, or 27, preferably SEQ ID NO: 11, 22, 25, or 27;
(ii) a polypeptide consisting of an amino acid sequence derived from the sequence of SEQ ID NO: 3, 11, 15, 18, 20, 22, 25, or 27, preferably SEQ ID NO: 11, 22, 25, or 27, by deletion, substitution, or addition of one or several amino acids and having the desired binding ability; and
(iii) a polypeptide having a homology of 75% or higher, 80% or higher, 85% or higher, 90% or higher, 95% or higher, 98% or higher, or 99% or higher to the polypeptide of SEQ ID NO: 3, 11, 15, 18, 20, 22, 25, or 27, preferably SEQ ID NO: 11, 22, 25, or 27, and having the desired binding ability; and
a gene encoding any of the following polypeptides (iv) to (vi) as the V_{L} region:
(iv) a polypeptide of SEQ ID NO: 7, 13, 17, 19, 21, 24, 26, or 29, preferably SEQ ID NO: 13, 24, 26, or 29;
(v) a polypeptide consisting of an amino acid sequence derived from the sequence of SEQ ID NO: 7, 13, 17, 19, 21, 24, 26, or 29, preferably SEQ ID NO: 13, 24, 26, or 29, by deletion, substitution, or addition of one or several amino acids and having the desired binding ability; and
(vi) a polypeptide having a homology of 75% or higher, 80% or higher, 85% or higher, 90% or higher, 95% or higher, 98% or higher, or 99% or higher to the polypeptide of SEQ ID NO: 7, 13, 17, 19, 21, 24, 26, or 29, preferably SEQ ID NO: 13, 24, 26, or 29, and having the desired binding ability;
and expresses these polypeptides.

In one embodiment, the recombinant HSV of this aspect may have a gene encoding one of the following polypeptides (vii) to (ix) as the C_{H} region:
(vii) a polypeptide of SEQ ID NO: 4, 9, 12, 16, 23, or 28, preferably SEQ ID NO: 4, 12, 16, 23, or 28;
(viii) a polypeptide consisting of an amino acid sequence derived from the polypeptide of SEQ ID NO: 30 by deletion, substitution, or addition of one or several amino acids, and having the desired binding ability; and
(ix) a polypeptide having a homology of 75% or higher, 80% or higher, 85% or higher, 90% or higher, 95% or higher, 98% or higher, or 99% or higher to the polypeptide of SEQ ID NO: 30, and having the desired binding ability when used together with any one of the polypeptides (i) to (iii) as the V_{H} region.

In one embodiment, the recombinant HSV of this aspect may have a gene encoding any one of the following polypeptides (x) to (xii) as the C_{L} region:
(x) the polypeptide of SEQ ID NO: 8, 10, or 14, preferably SEQ ID NO: 8 or 14;
(xi) a polypeptide consisting of an amino acid sequence derived from the sequence of SEQ ID NO: 31 by deletion, substitution, or addition of one or several amino acids, and having the desired binding ability; and
(xii) a polypeptide having a homology of 75% or higher, 80% or higher, 85% or higher, 90% or higher, 95% or higher, 98% or higher, or 99% or higher to the polypeptide of SEQ ID NO: 31, and having the desired binding ability when used together with any one of the polypeptides (iv) to (vi) as the V_{L} region.

In one embodiment, the recombinant HSV preferably further comprises genes encoding polypeptides of the furin recognition sequence (SEQ ID NO: 5) and FMDV-2A (SEQ ID NO: 6), and may comprise these genes encoding the polypeptide of SEQ ID NO: 5 and the polypeptide of SEQ ID NO: 6 between the gene encoding any of the polypeptides (i) to (iii) and any of the polypeptides (vii) to (ix), and a gene encoding any of the polypeptides (iv) to (vi) and any of the polypeptides (x) to (xii).

When an amino acid sequence derived by deletion, substitution, or addition of one or several amino acids is mentioned, the number of amino acids deleted, replaced or added is not particularly limited as long as the protein consisting of the amino acid sequence has the desired binding ability, but it may be, for example, 1 to 5, 1 to 3, or 1 or 2. The position of the deletion, substitution or addition may be at the end or in middle of the peptide, and may consist of one or more positions.

Similarly, when a nucleotide sequence derived by deletion, substitution, or addition of one or several nucleotides is mentioned, the number of nucleotides deleted, replaced or added is not particularly limited as long as the polypeptide encoded by that nucleotide sequence has the desired binding ability, but it may be, for example, 1 to 5, 1 to 3, or 1 or 2. The position of the deletion, substitution or addition may be at the end or in middle of the polynucleotide, and may consist of one or more positions.

Whether a polypeptide has the desired binding ability can be determined by using a method known to those skilled in the art. For example, it can be determined by using the ELISA described in the examples mentioned below.

The amino acids may be natural amino acids, as well as derivatives thereof, artificial amino acids, and non-natural amino acids. In one embodiment, the amino acids are preferably natural amino acids.

For introducing a gene encoding an immune checkpoint inhibitor into an oncolytic HSV, known methods can be used. For example, DNA encoding an immune checkpoint inhibitor can be inserted into an expression vector by a known method (e.g., methods using restriction enzymes etc.), and the expression vector can be transfected into an oncolytic HSV. The expression vector can further contain a promoter, which regulates the expression of the target gene, replication origin, selection marker gene, etc. The promoter and replication origin can be appropriately selected according to the types of the oncolytic HSV and vector into which the gene is introduced.

Specifically, for example, an amino acid sequence or nucleotide sequence of the antibody or fragment thereof to be expressed can be obtained, and the desired region can be synthesized by using known techniques. For example, a sequence encoding polypeptides corresponding to the heavy chain and light chain of the antibody can be designed so that the heavy chain and light chain are expressed in equal amounts from cDNA to enable efficient antibody production, preferably so that both the amino acid sequences of the self-cleaving foot-and-mouth disease virus (FMDV) 2A and the furin cleavage site are introduced, and can be used to modify a virus. Furthermore, the polypeptides corresponding to the heavy chain and light chain may each have a signal sequence at their N-terminus for secretion.

The insertion site for the above gene is not particularly limited as long as the oncolytic HSV maintains the oncolytic activity thereof. For example, the gene encoding an immune checkpoint inhibitor can be inserted into a site where a non-essential viral gene is deleted. When the virus is HSV-1, non-essential genes shown in Table 1 mentioned below can be deleted, and a gene encoding an immune checkpoint inhibitor can be introduced. Expression of the introduced gene can be confirmed by using known methods, as shown in the examples described later.

For genetic recombination in viruses having large genomes such as HSV, for example, the T-BAC system with IL-12 expression described in Non-patent document 2 or similar systems can be used.

### (Oncolytic HSV)

One aspect of the present invention relates to an oncolytic HSV. The term oncolytic means that the virus has been subjected to genetic recombination for use in tumor therapy.

In this aspect, the oncolytic HSV is a virus that can be used as an active ingredient of a pharmaceutical composition for virus therapy or cancer therapy, and is not particularly limited so long as it has an ability to infect tumor cells, selectively replicate in the tumor cells, destroy the tumor cells in the process of viral replication, infect other surrounding tumor cells, and further replicate, and known viruses can be used. Many of such viruses are mutants of naturally occurring viruses obtained by genetic modification to increase tumor selectivity. It may be one attenuated as necessary and modified to enhance antitumor activity, such as those incorporated with a gene encoding VEGF antagonist (Patent document 2) or those incorporated with a gene that expresses a fusion polypeptide consisting of the light chain and heavy chain of IL-12 linked with an elastin motif (Non-patent document 2).

Examples of the oncolytic HSV usable in this aspect of the present invention include, for example, mutants of HSV-1 or HSV-2.

In one embodiment, the oncolytic HSV is a mutant of HSV-1. HSV-1 is classified as an enveloped double-stranded DNA virus and has the following characteristics advantageous for cancer therapy: 1) it is infectious to all types of cells in human; 2) the life cycle and the genome sequence of the virus have been elucidated; 3) the functions of the majority of the viral genes have become clear and genetic manipulation can be applied; and 4) the viral genome is large (about 152 kb), and therefore a large gene or multiple genes can be incorporated. HSV-1 further has the following advantages suitable for clinical application: 5) it can kill all cells at a relatively low multiplicity of infection (MOI); 6) there are antiviral drugs to suppress proliferation thereof; 7) anti-HSV-1 antibodies in blood do not affect the spread of the virus infection from cell to cell, and therefore repeated administration thereof is possible; 8) preclinical evaluation of safety and effects can be performed in animals thanks to the existence of mice and monkeys that are sensitive to HSV-1; and 9) the viral DNA is not incorporated into the genome of the host cell and is present outside the chromosome.

The genome of HSV-1 consists of two unique sequence regions: 82% of long unique region (U_{L}) and 12% of short unique region (Us), as well as terminal repeat (TR) and inverted repeat (IR) locating at each end of U_{L} and Us (Table 1, Todo, T. (2008). Frontiers in Bioscience a Journal and Virtual Library 13, 2060-2064). Since each of the two regions L and S can take two directions independently, the HSV-1 genomic DNA has four isomers. This genome contains, in total, 84 genes encoded unidirectionally in R_{L}1 and R_{L}2 on TR_{L}, U_{L}1 to U_{L}56 on U_{L}, R_{S}1 on TR_{S}, and Us 1 to Us 12 on Us; about half of which are genes unnecessary for viral growth, and deletion of these non-essential gene moieties can reduce pathogenicity and allow gene transduction (Carson, J. et al. (2010). Drugs of the Future 35, 183-195).

**[Table 1]**

| 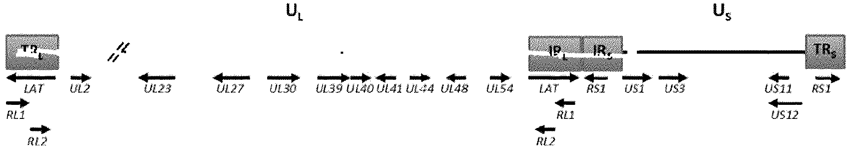 UL                                                                                    US TRL                                                    IRL  IRS                                        TRS LAT   UL2        UL23        UL27    UL30    UL39 UL40 UL41 UL44 UL48    UL54    LAT    RS1    US1    US3        US11    RS1 RL1                                                                                  RL1                                US12 RL2                                                                              RL2 | | | |
|---|---|---|---|
| Gene | Essential (E) or non-essential (N) | Protein | Function |
| *LAT* | | LAT | Only gene that is expressed during latent infection |
| *RL1* | N | ICP34.5 | Neuropathogenicity, suppressing apoptosis |
| *RL2** | N | ICP0 | Promoting transcription |
| *UL2* | E | UNG | Encoding uracil-DNA glycosylase required for DNA synthesis |
| *UL23* | N | ICP36 (Thymidine kinase) | Encoding thymidine kinase required for DNA synthesis |
| *UL27* | E | gB | Invasion into cells |
| *UL30* | E | DNA polymerase | Catalytic subunit of DNA polymerase |
| *UL39* | N | ICP6 | Encoding large subunit of ribonucleotide reductase (RR) required for DNA synthesis |
| *UL40* | | | Encoding small subunit of RR |
| *UL41* | N | vhe (virion-induced host shutoff) | Degrading host mRNA |
| *UL44* | N | gC | Adsorption to cells |
| *UL48* | E | VPI6 | Promoting most early gene expression |
| *UL54** | E | ICP27 | Regulating DNA synthesis and transcription, suppressing expression of host proteins by suppressing splicing |
| *RS1** | E | ICP4 | Regulating β and y genes, suppressing apoptosis |
| *US1** | N | ICP22 | Regulating transcription |
| *US3* | N | | Suppressing apoptosis, transferring capsid from nucleus to cytoplasm, regulating expression of gB |
| *US11* | N | | Inhibiting activation of protein kinase R under control by early promoter |
| *US12** | N | ICP47 | Inhibiting antigen presentation in host |

| | | | |
|---|---|---|---|
| * immediate early genes | | | |

If the oncolytic HSV is an HSV-1 mutant, it may have one or more of the following characteristics.
- Acquirement of tumor cell-specific replication ability due to inactivation of an enzyme involved in viral DNA synthesis, such as thymidine kinase (TK), ribonucleotide reductase (RR), and uracil-N-glycosylase (UNG or UDG).
- Acquirement of tumor cell-specific replication ability due to deletion of the gene γ34.5, which encodes ICP34.5, a protein involved in HSV-1 pathogenicity.
- Acquirement of anti-tumor effect due to deletion of α47.
- Deletion or inactivation of a gene for prevention of reversion to wild type to increase safety (e.g., deletion or inactivation of endogenous γ34.5 gene, α47 gene (ICP47 gene), α0 gene (ICP0 gene), U_{L}41 gene (vhs gene), and U_{L}56 gene).
- Enhancement of antitumor immunity and prolongation of survival by expression of an immune-stimulating gene (genes for IL-4, IL-10, GM-CSF, IL-12, soluble B7-1, etc.).
- Enhancement of antiangiogenic and antitumor effects by expression of a gene that expresses an angiogenesis inhibitory factor such as platelet factor 4, thrombospondin, endostatin, dominant-negative FGF, angiostatin, etc.
- Enhancement of antitumor effect by expression of an inhibitor for metalloproteinase involved in local invasion of tumors.
- Promotion of spread of viral infection by overexpression of metalloproteinases.
- Enhancement of tumor cell-specific viral replication ability by regulating a viral gene with a tumor or tissue-specific promoter (calponin promoter, E2F-responsive cell cycle-dependent promoter B-myb, Nestin promoter, carcinoembryonic antigen (CEA), α-fetoprotein (AFP), MUC-1, Musashi enhancer/promoter, etc.).
- Suppression of development of swelling by expression of a vascular endothelial growth factor (VEGF) antagonist.

In one embodiment, the oncolytic HSV of this aspect is preferably HSV-1 having one or more of the following characteristics (a) to (c), more preferably all of the following characteristics (a) to (c). HSV-1 mutants having these characteristics can be produced with reference to WO2011/101912, Patent document 2, etc.
(a) The ICP6 gene (UL39 gene) is deleted or inactivated, or expressed under the control of a tumor-specific or tissue-specific promoter
(b) The γ34.5 gene is deleted or inactivated.
(c) The α47 gene is deleted or inactivated.

With respect to the characteristics (a) and (b) mentioned above, deletion of ICP6, the large subunit of RR, which is the key enzyme for nucleotide metabolism and viral DNA synthesis in non-dividing cells, and/or γ34.5, which antagonizes the function of phosphorylated PKR that occurs during viral infection, results in a virus that cannot replicate in normal cells, but can replicate only in tumor cells that actively divide and show increased RR activity and suppressed phosphorylation of PKR. With respect to the characteristic (c) mentioned above, the protein encoded by the α47 gene has an ability to escape from immunosurveillance of the host by inhibiting TAP to suppress expression of MHC class I on the host cell surface, and therefore α47 gene-deleted HSV-1 is expected to give a stronger stimulation to immune cells due to maintenance of the MHC class I expression of the host cells. Further, in HSV-1 having both of the above characteristics (b) and (c), the US 11 late promoter, which overlaps with the α47 gene on the genome, is simultaneously deleted with the deletion of α47, so that the expression of the US 11 gene is placed under the control of the ICP47 immediate-early promoter, resulting in earlier expression of the gene, and therefore it comes to have an ability to restore the viral replication ability, which has been weakened by the deletion of γ34.5, only in tumor cells.

The term deletion or inactivation of a gene means deletion of all or a part of the gene, substitution or modification of some nucleotides of the gene, insertion of an unnecessary sequence into the gene, or the like to suppress expression of the gene, which can be carried out by known methods.

The term tumor-specific promoter or tissue-specific promoter means a promoter that enables expression of a gene that is under the control thereof specifically in a desired tumor cell or tissue, and known promoter can be used according to the gene. For example, the telomerase reverse transcriptase promoter (hTERT promoter) and E2F promoter can be used.

Examples of HSV of which γ34.5 gene, ICP6 gene and/or α47 gene are deleted or inactivated as described in the characteristics (a) to (c) mentioned above include G207 in which two copies of the γ34.5 gene are both deleted and the ICP6 gene is inactivated, and G47Δ in which two copies of the γ34.5 gene are deleted, the ICP6 gene is inactivated, and the α47 gene is deleted. Therefore, the recombinant HSV of this aspect can also be produced by further modifying G207 or G47Δ.

In particular, G47Δ is an oncolytic HSV-1 having markedly improved tumor cell-selective viral replication ability and anti-tumor immunity induction ability in addition to the improved safety, and can also be safely intracerebrally administered to humans at high doses because of the broad therapeutic spectrum thereof. In June 2021, it was approved in Japan as a product for regenerative medicine (generic name, teserpaturev; product name, Delytact Injection) for the treatment of malignant glioma.

With respect to the characteristic (a) mentioned above, it is preferred that a region encoding an immune checkpoint inhibitor is inserted at the site where the ICP6 gene has been deleted or inactivated. The expression that a region encodes a polypeptide means that the nucleotide sequence of the region encodes the amino acid sequence of the polypeptide or the complementary sequence of the nucleotide sequence of the region encodes the amino acid sequence of the polypeptide.

The expression that a region encoding an immune checkpoint inhibitor is inserted at the site where the ICP6 gene has been deleted or inactivated means that, for example, the virus has a sequence highly identical to a portion of the nucleotide sequence of SEQ ID NO: 30 mentioned in Sequence Listing except for Location 5349..9170, and inserted with a sequence encoding an immunogenic polypeptide or a complementary sequence thereof instead of Location 5349..9170. The term highly identical means to have an identity of 75% or higher, 80% or higher, 85% or higher, 90% or higher, 95% or higher, 98% or higher, or 99% or higher.

As the sequence of SEQ ID NO: 30 and in Fig. 8, the sequence of the virus prepared in the experiment described in the section of Examples, including the insertion site of the region encoding the immune checkpoint inhibitor and upstream and downstream nucleotide sequences thereof, are shown. The insertion site for the region encoding the immune checkpoint inhibitor is Location 5349..9170 in the sequence of SEQ ID NO: 30, and in Fig. 8, it corresponds to the sequence from the double-underlined TCA to the double-underlined CAT (the double-underlined TCA is the complementary sequence of the stop codon, and the double-underlined CAT is the complementary sequence of the start codon).

The expression that the virus has the characteristic (b) mentioned above means that, for example, the virus has a sequence derived from the nucleotide sequence of SEQ ID NO: 31 mentioned in Sequence Listing by deletion of all or a part of Location 88..834, substitution or modification of a part of the nucleotides thereof, or insertion of an unnecessary sequence into the same, which results in suppression of the expression of the γ34.5 gene, or that the virus has a sequence derived from the nucleotide sequence of SEQ ID NO: 31 by deletion of all or a part of the nucleotide sequence in IR_{L} corresponding to Location 88..834, substitution or modification of a part of the nucleotides thereof, or insertion of an unnecessary sequence into the same, which results in suppression of the expression of the γ34.5 gene. It is preferred that all or parts of the both γ34.5 gene sequences are deleted, a part of the nucleotides thereof are substituted or modified, or an unnecessary sequence is inserted into them, resulting in suppression of the expression of both genes. The expression that the virus has the characteristic (b) mentioned above also means that, for example, the virus has a sequence highly identical to the nucleotide sequence of SEQ ID NO: 31 except for Location 177..1128.

As SEQ ID NO: 31 and in Fig. 9, the nucleotide sequence of the γ34.5 gene of the strain from which G47Δ is derived and upstream and downstream nucleotide sequences thereof (within the TR_{L} region) are shown. Location 88..834 in the sequence of SEQ ID NO: 6 or the sequence from the start codon to the termination codon (these codons are shown with shading) in Fig. 9 is the coding region of the γ34.5 gene. The region deleted in the basic backbone of G47Δ (nucleotide numbers 576 to 1527 in GU734771.1) corresponds to Location 177..1128 in the sequence of SEQ ID NO: 31 and the underlined portion in Fig. 9. The aforementioned sequence of TR_{L} and the complementary sequences of the sequences around the coding region of the γ34.5 gene in IR_{L} (nucleotide numbers 124349 to 125798 in GU734771.1) are completely identical.

The expression that the virus has the characteristic (c) mentioned above means that, for example, the virus has a sequence derived from the nucleotide sequence of SEQ ID NO: 32 mentioned in Sequence Listing by deletion of all or a part of Location 218..484, substitution or modification of a part of the nucleotides thereof, or insertion of an unnecessary sequence into the same, which results in suppression of the expression of the α47 gene. The expression that the virus has the characteristic (c) mentioned above also means that, for example, the virus has a sequence highly identical to the nucleotide sequence of SEQ ID NO: 32 except for Location 230..540.

As SEQ ID NO: 32 and in Fig. 10, the nucleotide sequence of the α47 gene of the strain from which G47Δ is derived and upstream and downstream sequences thereof are shown. The α47 gene is encoded on the complementary strand side. The sequence of Location 218..484 in the sequence of SEQ ID NO: 32, or the sequence from the start codon to the termination codon (these codons are shown with shading) in Fig. 10 is the coding region of the α47 gene. The sequence of the site deleted in the basic backbone of G47Δ (nucleotide numbers 576 to 1527 in GU734771.1) corresponds to Location 177..1128 in the sequence of SEQ ID NO: 32 and the underlined portion in Fig. 10.

The oncolytic HSV can be produced by referring to, for example, Patents documents 1 and 2, Non-patent document 1, and International Publication WO2005/103237.

### (Pharmaceutical composition)

Another aspect of the present invention relates to a pharmaceutical composition containing the oncolytic HSV having a gene encoding an immune checkpoint inhibitor described above as one aspect of the present invention and having been subjected to genetic modification for use in treating tumors. If the pharmaceutical composition of this aspect is administered, the oncolytic HSV replicates specifically in tumor cells and expresses an immune checkpoint inhibitor (e.g., an immune checkpoint inhibitor antibody) locally in the tumor each time the virus replicates within the tumor.

Administration of the pharmaceutical composition of this aspect of the present invention can be expected to significantly enhance the therapeutic efficacy of viral therapy. Conventional immune checkpoint inhibitor antibodies can shift the entire immune system to an attack mode, but therapeutic efficacy is not achieved unless the immune system recognizes cancer as non-self. However, the oncolytic HSV can induce the immune system to recognize cancer cells as non-self and elicit anti-cancer immunity.

In one embodiment, the pharmaceutical composition is of an immune checkpoint inhibitor blood concentration suppression type. Conventional immune checkpoint inhibitor antibodies are known to cause severe side effects, such as autoimmune diseases, with relatively high frequency when administered systemically. In one embodiment, the immune checkpoint inhibitor antibody expressed by administration of the pharmaceutical composition remains localized to the tumor.

Examples of tumor for which application of the pharmaceutical composition of this aspect is expected include, but are not limited to, brain tumor, glioma, ocular tumor, neuroblastoma, retinoblastoma, primary malignant lymphoma of CNS, meningioma, pituitary adenoma, schwannoma, craniopharyngioma, intraocular tumor, retinoblastoma, choroidal malignant melanoma, intraocular malignant lymphoma, tumor of ocular appendage, eyelid tumor, lacrimal gland cancer, ocular appendage lymphoma, orbital sarcoma, conjunctival tumor, optic nerve tumor, glioma, oral cavity cancer, tongue cancer, pharyngeal cancer, thyroid cancer, auricular cancer, adenoid cystic carcinoma, olfactory neuroblastoma, sarcoma of head and neck, lung cancer, breast cancer, thymoma, thymic carcinoma, malignant pleural mesothelioma, sarcoma of trunk, sarcoma of heart, pulmonary neuroendocrine tumor, SMARCA4-deficient thoracic tumor, non-small cell lung cancer, small cell lung cancer, esophageal cancer, stomach cancer, colorectal cancer (colon cancer and rectum cancer), small intestine cancer (duodenum cancer, jejunum cancer, and ileum cancer), GIST (gastrointestinal stromal tumor), pancreatic and gastrointestinal neuroendocrine tumors, anal cancer, gastrointestinal neuroendocrine tumor, hepatocellular cancer, biliary tract cancer (bile duct cancer (including intrahepatic bile duct cancer), gall bladder cancer, and papillary duodenal cancer), pancreatic cancer, pancreatic and gastrointestinal neuroendocrine tumors, liver tumor, pancreatic neuroendocrine tumor, renal cell carcinoma, renal pelvis and ureter cancers, bladder cancer, urachal carcinoma, renal tumor, prostate cancer, breast cancer, cervical cancer, uterine body cancer (endometrial cancer), uterine sarcoma, ovarian and oviduct cancers, vaginal cancer, vulvar cancer, malignant melanoma (skin), basal cell carcinoma, spinous cell carcinoma, lymphoma of skin, soft tissue sarcoma (adults), desmoid tumor, osteosarcoma (pediatric), Ewing's sarcoma (pediatric), soft tissue sarcoma (pediatric), rhabdomyosarcoma (pediatric), undifferentiated/unclassifiable sarcomas, osteosarcoma, chondrosarcoma, chordoma, Ewing's sarcoma, liposarcoma, fibrosarcoma, myxofibrosarcoma, undifferentiated pleomorphic sarcoma, leiomyosarcoma, rhabdomyosarcoma, hemangiosarcoma, malignant peripheral nerve sheath tumor, synovial sarcoma, epithelioid sarcoma, alveolar soft part sarcoma, clear cell sarcoma, extraskeletal Ewing's sarcoma, acute myeloid leukemia, acute lymphocytic leukemia/lymphoblastic lymphoma, chronic myeloid leukemia, myelodysplastic syndrome, malignant lymphoma, B cell lymphoma, T/NK cell lymphoma, Hodgkin lymphoma, lymphoma of skin, chronic lymphocytic leukemia/small lymphocytic lymphoma, adult T-cell leukemia lymphoma, multiple myeloma, acute promyelocytic leukemia, follicular lymphoma, MALT lymphoma, lymphoplasmacytic lymphoma, mantle cell lymphoma, diffuse large B-cell lymphoma, peripheral T-cell lymphoma, Burkitt's lymphoma, extranodal NK/T-cell lymphoma, cancer of unknown primary origin, hereditary and familial tumors, neuroendocrine cancer, germ cell tumor, paraganglioma, germ cell tumor, testicular germ cell tumor, ovarian germ cell tumor, extragonadal germ cell tumor, and neuroendocrine tumor.

From another viewpoint, examples of tumors for which the pharmaceutical composition of this aspect is effective include tumors for which the application of immune checkpoint inhibitor is approved or being considered, such as malignant melanoma, non-small cell lung cancer, renal cell carcinoma, Hodgkin lymphoma, head and neck cancer, stomach cancer, malignant pleural mesothelioma, esophageal cancer, MSI-High colorectal cancer, gastroesophageal junction cancer/esophageal cancer, small cell lung cancer, hepatocellular cancer, glioblastoma, urothelial carcinoma, ovarian cancer, bladder cancer, prostate cancer, cervical cancer, uterine body cancer, soft tissue sarcoma, primary lymphoma of CNS, primary testicular lymphoma, pancreatic cancer, and biliary tract cancer,

Examples of tumors for which the pharmaceutical composition of this aspect is effective also include the following diseases for which application of immune checkpoint inhibitor is currently approved in Japan:
radically unresectable malignant melanoma, unresectable advanced or recurrent non-small cell lung cancer, unresectable locally advanced non-small cell lung cancer (for maintenance therapy after radical chemoradiation), radically unresectable or metastatic renal cell carcinoma, curatively unresectable or metastatic renal cell carcinoma, recurrent or refractory classic Hodgkin lymphoma, recurrent head and neck cancer or head and neck cancer with distant metastasis, curatively unresectable advanced or recurrent stomach cancer exacerbated after cancer chemotherapy, unresectable advanced or recurrent malignant pleural mesothelioma exacerbated after cancer chemotherapy, curatively unresectable advanced or recurrent colorectal cancer with high frequency of microsatellite instability (MSI-High) exacerbated after cancer chemotherapy, radically unresectable advanced or recurrent esophageal cancer exacerbated after cancer chemotherapy, radically unresectable urothelial carcinoma exacerbated after cancer chemotherapy, advanced or recurrent solid tumors with high frequency of microsatellite instability (MSI-High) exacerbated after cancer chemotherapy (only when standard treatments are not feasible), radically unresectable or metastatic renal cell carcinoma, advanced small cell lung cancer, inoperable or recurrent breast cancer that is PD-L1-positive, hormone receptor-negative and HER2-negative, and radically unresectable Merkel cell carcinoma,

From another viewpoint, examples of tumors for which the pharmaceutical composition of this aspect is effective include, for example, nerve system type tumors (e.g., astrocytoma, anaplastic astrocytoma, oligodendroglioma, anaplastic oligodendroglioma, meningioma, neurofibroma, glioblastoma, diffuse midline glioma, ependymoma, schwannoma, neurofibrosarcoma, and neuroblastoma), pituitary gland tumor (e.g., pituitary adenoma), medulloblastoma, melanoma, brain tumor, prostate cancer, head and neck cancer, esophageal cancer, renal cancer, renal cell cancer, pancreatic cancer, breast cancer, lung cancer, colon cancer, large bowel cancer, stomach cancer, skin cancer, ovarian cancer, bladder cancer, sarcoma (e.g., osteosarcoma, chondrosarcoma, rhabdomyosarcoma, leiomyosarcoma, fibrosarcoma, liposarcoma, and angiosarcoma), squamous cell carcinoma, neuroectoderm tumor, thyroid tumor, lymphoma, hepatocellular cancer, mesothelioma, epidermoid carcinoma, benign tumor (e.g., benign tumor of the thyroid gland or benign prostatic hyperplasia), etc., for which virus therapies are considered to be especially effective.

For the present invention and this aspect thereof, the term treatment referred to in relation to a disease or condition, it includes prevention, reduction of the risk of developing the disease, therapeutic treatment, inhibition of progression, and prevention of recurrence, unless especially noted.

The administration method of the pharmaceutical composition of this aspect is not particularly limited and it can be administered, for example, intravenously, intraarterially, intracerebroventricularly, intraperitoneally, intrathoracically, into the spinal cavity, subcutaneously, intradermally, intraepidermally, intramuscularly, or on mucosal surfaces (for example, eye, nasal cavity, lung, oral cavity, bowel, rectum, vagina, and urinary tract surfaces). It is preferably administered locally and directly to tumor tissues by injection, or endoscopic or surgical technique. If the pharmaceutical composition is locally administered, the immune checkpoint inhibitor is expressed locally at the tumor site, and therefore the risk of side effects is reduced compared with systemic administration of the immune checkpoint inhibitor. In a preferred embodiment, the pharmaceutical composition is for intratumoral administration or local injection.

The pharmaceutical composition of this aspect can be formulated by known formulation methods for administering viruses into the body of mammals including humans. For example, the pharmaceutical composition may contain an adjuvant or an arbitrary carrier, or it may simply be diluted in a physiologically acceptable solution such as sterile physiological saline or sterile buffered physiological saline without adding any adjuvant or carrier. It may also be made as, for example, a frozen, dried, or lyophilized formulation suitable for long-term storage.

The pharmaceutical composition of this aspect contains a therapeutically effective amount of the oncolytic HSV as the other aspect of the present invention. The term therapeutically effective amount herein used means an amount of the active substance with which one or more symptoms of the tumor to be treated are alleviated to some extent, more specifically, an amount that provides at least one of reduction in tumor size, inhibition (delay or arrest) of tumor metastasis, inhibition (delay or arrest) of tumor growth, and alleviation of one or more symptoms associated with the tumor. Specific doses may be appropriately determined by those skilled in the art according to the degree of symptoms, age, sex, weight, sensitivity difference of the target of administration, administration method, timing of administration, administration interval, nature of the preparation, strength of the promoter, etc.

The pharmaceutical composition of this aspect can be administered in an amount of, for example, about 10¹ to 10¹² plaque-forming units (pfu), preferably about 10⁷ to 10¹⁰ pfu, more preferably about 10⁸ to 5 x 10⁹ pfu, which can be administered by one or several times of injection. The number of times of the administration of the pharmaceutical composition of this aspect can be appropriately set according to the target patient and the target disease. For example, the first two doses can be administered within two weeks, and then the following doses can be administered at intervals of three to five weeks.

The pharmaceutical composition of this aspect may further contain one or more other active ingredients or may be used in combination with a pharmaceutical composition comprising one or more other active ingredients. The pharmaceutical composition of this aspect can also be used in combination with one or more other tumor treatment methods, such as surgery (tumor resection etc.), chemotherapy, radiation therapy, immunotherapy, hormone therapy, and combinations thereof. Since the viral therapy with the pharmaceutical composition of this aspect is based on a mechanism different from those of existing tumor treatment methods, the tumor treatment effect can be enhanced by combining it with other treatment methods, such as radiation therapy and chemotherapy.

The pharmaceutical composition of this aspect can be administered as a combination of a plurality of HSVs, each expressing a different immune checkpoint inhibitory antibody. It can also be administered in combination with at least one of a virus with soluble B7-11 expression and a virus with IL-12 expression. When administered in combination, they may be administered at the same time as a mixture thereof or sequentially. The order of administration is not also particularly limited.

The present embodiment also relates to a method for treating a tumor using an oncolytic HSV to which a function of expressing an immune checkpoint inhibitor has been added. The present embodiment also relates to use of a function-added oncolytic HSV in production of pharmaceuticals for treating tumors. The method and use can be implemented with reference to the above descriptions pertaining to the pharmaceutical composition.

### Examples

### A) Preparation of virus with anti-mouse PD-1 antibody expression (T-J43)

All viruses were prepared by using G47Δ as the basic backbone. The T-BAC system was used for virus preparation.

### 1) Amino acid sequence of anti-mouse PD-1 antibody

As the anti-mouse PD-1 antibody, an amino acid sequence for expressing the anti-mouse PD-1 antibody (J43) was designed on the basis of the sequence information of the J43 clone (derived from the Armenian hamster, US 7,858,746 B2) (Fig. 1-1-1). Artificial gene synthesis of a codon-optimized cDNA sequence for a mouse expression system based on this amino acid sequence was ordered.

### 1-2) Virus with anti-mouse PD-1 antibody expression (T-J43HA)

As the anti-mouse PD-1 antibody, an amino acid sequence for expressing the anti-mouse PD-1 antibody (J43) was designed on the basis of the sequence information of the J43 clone (derived from the Armenian hamster) (US 7,858,746 B2) (Fig. 1-1-2). Artificial gene synthesis of a codon-optimized cDNA sequence for a mouse expression system based on this amino acid sequence was ordered. For this synthesis, the constant regions of the heavy (H) and light (L) chains were those derived from Armenian Ham IgG, and the antibody was designated as J43HA.

### 2) Confirmation of anti-mouse PD-1 antibody expression

T-J43 and T-01 were infected into HEK293T cells at MOI=1. After 48 hours, the supernatants were collected, concentrated by ultrafiltration (Amicon Ultra 30K), and subjected to ELISA for human Fc (human Fc antibody was immobilized, and detection was performed with HRP-human Fc antibody) and PD-1-binding ELISA (mouse PD-1 was immobilized, and detection was performed with HRP-human Fc antibody). T-01 was a control virus into which no foreign gene other than LacZ was inserted at the ICP6 gene deletion site, and only an empty cassette of the cytomegalovirus (CMV) promoter and polyA was inserted. The results are shown in Fig. 1-2-1.

### 2-2) Confirmation of anti-mouse PD-1 antibody expression

Expression of the anti-mouse PD-1 antibody encoded by T-J43 was confirmed in mouse cell lines. Mouse neuroblastoma cell line Neuro2a, mouse melanoma cell line Clone M3, mouse renal carcinoma cell line Renca, and African green monkey kidney epithelial cell line Vero as a control were infected at MOI=2. After 18 hours, the culture supernatants were collected, and concentrated by ultrafiltration, and the expression of the encoded anti-PD-1 antibody was confirmed by ELISA. The results are shown in Fig. 1-2-2.

### 3) Confirmation of function of anti-mouse PD-1 antibody

To confirm whether the anti-mouse PD-1 antibody encoded by T-J43 has the PD-1/PD-L1 binding inhibitory activity as expected, an assay was performed by using AlphaLISA Mouse PD-1 and PD-L1 Binding Kit (PerkinElmer). First, J43/SV-01 plasmid was transiently expressed in HEK293T cells, and the culture supernatant of the cells was concentrated and used as a sample. Further, commercially available RMP1-14 and J43 antibody were used as positive controls, and a sample obtained by a similar treatment using SV-01 plasmid was used as a negative control. The results are shown in Fig. 1-3-1.

### 3-2) Confirmation of function of anti-mouse PD-1 antibody

To confirm whether the anti-mouse PD-1 antibody encoded by T-J43 has the PD-1/PD-L1 binding inhibitory activity as expected, an assay was performed by using AlphaLISA Mouse PD-1 and PD-L1 Binding Kit (PerkinElmer). First, J43/SV-01 plasmid was transiently expressed in HEK293T cells, and the culture supernatant of the cells was concentrated and used as a sample. Further, T-J43 was infected into HEK293T cells (MOI=1), and the culture supernatant was colleted after 48 hours, concentrated by ultrafiltration (Amicon Ultra 30K), and used as a sample. A commercially available J43 antibody was used as a positive control. The results are shown in Fig. 1-3-2.

Although the antibody expression level of T-J43 was not sufficiently high to calculate the IC₅₀, it is considered to be nearly identical to that obtained with J43/SV-01.

### 4) Cytotoxic effect (cytotoxicity assay)

Neuro2a cells were seeded on a 6-well plate (3 x 10⁵ cells/well) and infected with T-01 or T-J43 at MOI=0.1 or 1, and cultured at 34.5°C for 1 to 4 days. The cytotoxic effect was calculated as survival rate relative to cell count of the mock for each day. The results are shown in Fig. 1-4.

### 5) Replication assay

Vero, Neuro2a (3 x 10⁵ cells/well), and Clone M3 (4 x 10⁵ cells/well) cells were seeded on 6-well plates. T-01 or T-J43 was infected into the Vero and Clone M3 cells at MOI=0.1, and Neuro2a cells at MOI=0.3, and the cells were cultured at 37°C. After 24 and 48 hours, the cells and media were harvested, subjected to three times of freeze-thaw cycle, and ultrasonicated for 1 minute, and titration was performed. The results are shown in Fig. 1-5.

### 6) Infection efficiency (X-gal staining)

Vero, Neuro2a (3 x 10⁵ cells/well), and Clone M3 (5 x 10⁵ cells/well) cells were seeded on 6-well plates. T-01 or T-J43 was then infected into the cells at MOI=1 or MOI=3, and the cells were cultured at 37°C for 6 hours. Then, X-gal staining was performed. The results are shown in Fig. 1-6.

### 7) Confirmation of DNA structure

The prepared virus with anti-mouse PD-1 antibody expression (T-J43) was treated with the restriction enzyme HindIII. Southern blotting was performed by using the LacZ sequence and the insert sequence as probes. The results are shown in Fig. 1-7-1.

### 7-2) Confirmation of DNA structure

The prepared virus with anti-mouse PD-1 antibody expression (T-J43HA), virus with anti-mouse CTLA4 antibody expression (T-4F10HA), and virus with human B7-1Fc-expression (T-hB7.1) were treated with the restriction enzyme HindIII. Southern blotting was performed by using LacZ sequence and the insert sequences as probes. The results are shown in Fig. 1-7-2.

### 8) Comparison of antitumor effects in Neuro2a/A/J mouse unilateral subcutaneous tumor model

Neuro2a cells (3 x 10⁶ cells/70 µl) were unilaterally and subcutaneously injected into A/J mice. T-01 virus, T-J43 virus, or mock was intratumorally administered twice on the day the maximum tumor diameter reached approximately 5 mm (day 0) and 3 days later (day 3) at a dose of 4 x 10⁴ pfu/20 µl (n=9 to 10 per group), and tumor diameters were measured. The results are shown in Fig. 1-8. T-J43 showed a significant difference compared with T-01 (*, P<0.05).

### 9) Comparison of antitumor effects in Neuro2a/A/J mouse bilateral subcutaneous tumor model

Neuro2a cells (3 x 10⁶ cells/70 µl) were bilaterally and subcutaneously injected into A/J mice. T-01 virus, T-J43 virus, or mock was administered twice on the day the maximum tumor diameter reached approximately 5 mm (day 0) and 3 days later (day 3) at a dose of 2 x 10⁵ pfu/20 µl into the left ventral tumor (n=10 per group), and tumor diameters were measured. The results are shown in Fig. 1-9. Only T-J43 showed a significant difference compared with mock (*, P<0.05) (on day 10 for the untreated side).

### 10) Comparison of antitumor effects in Clone M3/DBA/2 mouse unilateral subcutaneous tumor model

Antitumor effects were compared in a unilateral subcutaneous tumor model by using the following method.

Unilaterally and subcutaneously inject Clone M3 cells (1 x 10⁶ cells/50 µl) into DBA/2 mice.
↓ 8 Days

Administer virus into the left tumor twice on the day the maximum tumor diameter reached approximately 5 mm (day 0) and 3 days later (day 3) (n=5 to 7 per group).

| |
|---|
| T-01 or T-J43, 6 x 10⁴ pfu/20 µl, or mock |
| ↓ |
| Measure tumor diameter. |

The results are shown in Fig. 1-10. T-J43 did not show significant difference compared with T-01, but showed a tendency of higher efficacy.

### 11) Comparison of antitumor effects in Clone M3/DBA/2 mouse bilateral subcutaneous tumor model

Antitumor effects were compared in a bilateral subcutaneous tumor model by using the following method.

Bilaterally and subcutaneously inject Clone M3 cells (1 x 10⁶ cells/50 µl) into DBA/2 mice.
↓ 8 Days

Administer virus into the left tumor twice on the day the maximum tumor diameter reached approximately 5 mm (day 0) and 3 days later (day 3) (n=5 to 8 per group).

| |
|---|
| T-01 or T-J43, 2 x 10⁵ pfu/20 µl, or mock |
| ↓ |
| Measure tumor diameter. |

The results are shown in Fig. 1-11. T-J43 showed a significant difference on the treated side compared with T-01 on day 13. On the untreated side, only T-J43 showed a significant difference compared with the mock.

### 12) Comparison of antitumor effects in Renca/Balb/c mouse unilateral subcutaneous tumor model (anti-PD-1 antibody, anti-CTLA-4 antibody, and two-ingredient cocktail thereof)

Renca cells (1 x 10⁵ cells/50 µl) were unilaterally and subcutaneously injected into Balb/c mice. T-01, T-J43, T-4F10HA virus, or mock was intratumorally administered twice on the day the maximum tumor diameter reached approximately 5 mm (day 0) and 3 days later (day 3) at a dose of 2 x 10⁵ pfu/20 µl (n=9 or 10 per group), and tumor diameters were measured. As mix, 20 µl of a mixture of equal volumes of T-J43 and T-4F10HA prepared at a concentration of 2 x 10⁵ pfu/20 µl was administered. The results are shown in Figs. 1-12 and 1-12-2.

### 13) Comparison of antitumor effects of combination of T-01 and J43 antibody and. T-J43 alone in Neuro2a/A/J mouse bilateral subcutaneous tumor model

Neuro2a cells (3 x 10⁶ cells/70 µl) were bilaterally and subcutaneously injected into A/J mice. T-01, T-J43, or mock was intratumorally administered twice on the day the maximum tumor diameter reached approximately 5 mm (day 0) and 3 days later (day 3) at a dose of 2 x 10⁵ pfu/20 µl (n=10 per group), 10 µg of J43 antibody or isotype control (Armenian hamster IgG, 2A3) was intraperitoneally administered at the same time, and tumor diameters were measured.

### 14) Comparison of blood and intratumoral antibody concentrations after intraperitoneal administration of J43 and intratumoral administration of T-J43HA* in Neuro2a/A/J mouse unilateral subcutaneous tumor model

Neuro2a cells (3 x 10⁶ cells/70 µl) were unilaterally and subcutaneously injected into A/J mice. On the day the maximum tumor diameter reached approximately 5 mm (day 0), T-J43 or mock was intratumorally administered at a dose of 2 x 10⁵ pfu (M), 1 x 10⁶ pfu (H), or 5 x 10⁶ pfu (XH)/20 µl) (n=3 per group), 10, 50, or 200 µg of anti-PD-1 antibody, J43, or isotype control (Armenian hamster IgG, 2A3) was intraperitoneally administered at the same time, and blood samples and tumors were collected 1, 4, and 7 days later. Serum and tumor proteins were prepared, and antibody concentrations in blood and tumors were measured by ELISA. For ELISA, mouse PD-1 was immobilized, and detection was performed with HRP-labeled anti-Armenian hamster IgG antibody as the secondary antibody. J43 was used as the standard.

Blood concentration: Intraperitoneal administration of the antibodies resulted in significant concentrations of the antibodies in blood, which decreased over time. Although antibodies were detectable following the intratumoral administration of T-J43HA virus, the concentration was as low as approximately 1/10,000 of that observed with the intraperitoneal antibody administration, nearly at the detection limit (Fig. 1-14, top).

Intratumoral concentration (concentration in 0.1 mg of tumor proteins; measured in 100 µl of a 1 mg/ml preparation): Following the intraperitoneal antibody administration, antibodies were detected in the tumor in a dose-dependent manner and decreased over time. Following the intratumoral administration of T-J43HA virus, antibodies were detected even with administration at low titers, but the concentrations were comparable to those detected in the mock group, that is, on day 1, antibodies were detected at concentrations of approximately 13 ng/ml and 25 ng/ml in the H (1 x 10⁶ pfu) and XH (5 x 10⁶ pfu) administration groups, respectively (Fig. 1-14, bottom).

In this experiment, T-J43HA, designed with an Armenian hamster IgG as the Fc region, was used for comparison with the commercially available antibody J43.

### B) Preparation of virus with anti-human and anti-mouse PD-L1 antibody expression (T-ATZ)

### 1) Amino acid sequences of anti-human and anti-mouse PD-L1 antibodies

For the anti-human and anti-mouse PD-L1 antibodies, the amino acid sequence for expressing anti-human and anti-mouse PD-1 antibodies (T-ATZ) was designed on the basis of the sequence information for atezolizumab (KEGG entry # D10773; TECENTRIQ (Genentech)) (Fig. 2-1). On the basis of this amino acid sequence, a cDNA sequence was designed, and artificial gene synthesis of the sequence was ordered.

### 2) Confirmation of antibody expression

### 2-2) Confirmation of anti-mouse PD-L1 antibody expression

Expression of the anti-mouse PD-L1 antibody encoded by T-ATZ was confirmed in mouse cell lines. The virus was infected into cells of the mouse neuroblastoma cell line Neuro2a, mouse melanoma cell line Clone M3, mouse renal carcinoma cell line Renca, and as the control cell line, African green monkey kidney epithelial cell line Vero at MOI=2. After 18 hours, the culture supernatants were collected, and concentrated by ultrafiltration, and expression of the encoded anti-PD-L1 antibody was confirmed by ELISA (Fig. 2-2-2).

### 2-3) Confirmation of anti-human PD-L1 antibody expression

Expression of the anti-human PD-L1 antibody encoded by T-ATZ was confirmed in human cell lines. The virus was infected into cells of the human glioma cell line U87MG, human melanoma cell line Mewo, human renal carcinoma cell line OSRC2, and as the control cell line, the African green monkey kidney epithelial cell line Vero at MOI of 2. After 18 hours, the culture supernatants were collected, and concentrated by ultrafiltration, and expression of the encoded anti-PD-L1 antibody was confirmed by ELISA (Fig. 2-2-3).

### 3) Confirmation of function of anti-mouse PD-L1 antibody

To confirm whether the anti-human/mouse PD-L1 antibodies encoded by T-ATZ have the PD-1/PD-L1 binding inhibitory activity as expected, an assay was performed by using AlphaLISA Mouse PD-1 and PD-L1 Binding Kit (PerkinElmer). First, ATZ/SV-01 plasmid was transiently expressed in HEK293T cells, and the culture supernatant of the cells was concentrated and used as a sample. Further, a sample obtained by using a similar treatment with SV-01 plasmid was used as a negative control. The results are shown in Fig. 2-3-1.

### 3-2) Confirmation of function of anti-human PD-L1 antibody

To confirm whether the anti-human/mouse PD-L1 antibodies encoded by T-ATZ have the PD-1/PD-L1 binding inhibitory activity as expected, an assay was performed by using AlphaLISA Human PD-1 and PD-L1 Binding Kit (PerkinElmer). First, ATZ/SV-01 plasmid was transiently expressed in HEK293T cells, and the culture supernatant was concentrated and used as a sample. Further, a sample obtained by using a similar treatment with SV-01 plasmid was used as a negative control. The results are shown in Fig. 2-3-2.

### 3-3) Confirmation of function of anti-mouse PD-L1 antibody

To confirm whether the anti-human/mouse PD-L1 antibodies encoded by T-ATZ have the PD-1/PD-L1 binding inhibitory activity as expected, an assay was performed by using AlphaLISA Mouse PD-1 and PD-L1 Binding Kit (PerkinElmer). T-ATZ was infected into HEK293T cells at MOI=1, and the culture supernatant was concentrated after 48 hours and used as a sample. Commercially available atezolizumab (ATZ) was used as a positive control. A culture supernatant of the cells infected with T-01 under the same conditions was used as a negative control. The results are shown in Fig. 2-3-3.

### 3-4) Confirmation of function of anti-human PD-L1 antibody

To confirm whether the anti-human/mouse PD-L1 antibodies encoded by T-ATZ have the PD-1/PD-L1 binding inhibitory activity as expected, an assay was performed by using AlphaLISA Human PD-1 and PD-L1 Binding Kit (PerkinElmer). T-ATZ was infected into HEK293T cells at MOI=1, and the culture supernatant was concentrated after 48 hours and used as a sample. Commercially available atezolizumab (ATZ) was used as a positive control. A culture supernatant of the cells infected with T-01 under the same conditions was used as a negative control. The results are shown in Fig. 2-3-4.

### 4) Cytotoxic effect (cytotoxicity assay)

Clone M3 cells (2 x 10⁵ cells/well) were seeded on a 6-well plate and infected with T-01 or T-ATZ at MOI=0.1 or 1. The cells were cultured at 34.5°C for 1 to 4 days. The results are shown in Fig. 2-4.

### 5) Replication assay

Vero cells (3 x 10⁵ cells/well) and Clone M3 cells (4 x 10⁵ cells/well) were seeded on 6-well plates. T-01 or T-ATZ was infected into Vero cells at MOI of 0.02 and Clone M3 cells at MOI of 0.1, and the cells were cultured at 37°C. After 24 and 48 hours, the cells and media were collected, subjected to three times of freeze-thaw cycle, and ultrasonicated for 1 minute, and then titration was performed. The results are shown in Fig. 2-5.

### 6) Infection efficiency (X-gal staining)

Vero and Neuro2a cells (3 x 10⁵ cells/well) were seeded on 6-well plates and infected with T-01 or T-ATZ at MOI=1 or 3, and cultured at 37°C for 6 hours. X-gal staining was then performed. The results are shown in Fig. 2-6.

### 7) Confirmation of DNA structure

The prepared virus with anti-human and anti-mouse PD-L1 antibody expression (T-ATZ) was treated with the restriction enzyme HindIII. Southern blotting was performed by using the LacZ sequence and the insert sequence as probes. The results are shown in Fig. 2-7.

### 8) Comparison of antitumor effects in Neuro2a/A/J mouse unilateral subcutaneous tumor model

The antitumor effects in a unilateral subcutaneous tumor model (first injection) were compared by using the following method.

Subcutaneously inject Neuro2a cells (5 x 10⁶ cells/50 µl) into A/J mice.
↓ 4 days

Intratumorally administer virus twice on the day the maximum tumor diameter reached approximately 5 mm (day 0) and 3 days later (day 3) (n=6 per group).

| |
|---|
| T-01, T-ATZ, 4 x 10₄ pfu/20 µl |
| Mock |
| ↓ |
| Measure tumor diameter. |

T-ATZ did not show significant difference compared with T-01, but showed a tendency of higher efficacy (Fig. 2-8).

### 9) Comparison of antitumor effects in Neuro2a/A/J mouse bilateral subcutaneous tumor model

Antitumor effects were compared in a bilateral subcutaneous tumor model by using the following method.

Bilaterally and subcutaneously inject Neuro2a cells (5 x 10⁶ cells/50 µl) into A/J mice.
↓ 4 days
Intratumorally administer virus twice on the day the maximum tumor diameter reached approximately 5 mm (day 0) and 3 days later (day 3) (n=10 per group).
T-01, T-ATZ 2 x 10⁵ pfu/20 µl
Mock
↓
Measure tumor diameter.

Only T-ATZ showed a significant difference on both the treated and untreated sides compared with the mock (Fig. 2-9).

### 10) Comparison of antitumor effects in Clone M3/DBA/2 mouse unilateral subcutaneous tumor model

Antitumor effects were compared in a unilateral subcutaneous tumor model (first injection) by using the following method.
Bilaterally and subcutaneously inject Clone M3 cells (1 x 10⁶ cells/50 µl) into DBA/2 mice.
↓ 10 Days

Administer virus into the left ventral tumor twice on the day the maximum tumor diameter reached approximately 5 mm (day 0) and 3 days later (day 3) (n=10 to 12 per group).

| | |
|---|---|
| T-01, T-ATZ, | 2x 10₅ pfu/20 µl |
| Mock | |
| ↓ | |
| Measure tumor diameter. | |

T-ATZ showed a significant difference compared with T-01 (Fig. 2-10).

### 11) Comparison of antitumor effects in Clone M3/DBA/2 mouse bilateral subcutaneous tumor model

Antitumor effects were compared in a bilateral subcutaneous tumor model by using the following method.
Bilaterally and subcutaneously inject Clone M3 cells (1 x 10 cells/50 µl) into DBA/2 mice.
↓ 8 Days

Administer virus into the left ventral tumor twice on the day the maximum tumor diameter reached approximately 5 mm (day 0) and 3 days later (day 3) (n=8 to 10 per group).

| | |
|---|---|
| T-01, T-ATZ, | 2x 10₅ pfu/20 µl |
| Mock | |
| ↓ | |
| Measure tumor diameter. | |

Only T-ATZ showed a significant difference compared with the mock on the untreated side on day 11 (Fig. 2-11).

### 12) Comparison of antitumor effects in Renca/Balb/c mouse unilateral subcutaneous tumor model (anti-PD-L1 antibody)

Renca cells (1 x 10⁵ cells/50 µl) were unilaterally and subcutaneously injected into Balb/c mice. T-01 virus, T-ATZ virus, or mock was intratumorally administered twice on the day the maximum tumor diameter reached approximately 5mm (day 0) and 3 days later (day 3) at a dose of 2 x 10⁵ pfu/20 µl (n=9 or 10 per group), and tumor diameters were measured (Fig. 2-12).

### 13) Comparison of antitumor effects of combination of T-01 and ATZ antibody and T-ATZ alone in Neuro2a/A/J mouse bilateral subcutaneous tumor model

Neuro2a cells (3 x 10⁶ cells/70 µl) were bilaterally and subcutaneously injected into A/J mice. T-01, T-ATZ, or mock was intratumorally administered twice on the day the maximum tumor diameter reached approximately 5mm (day 0) and 3 days later (day 3) at a dose of 2 x 10⁵ pfu/20 µl (n=10 per group), 10 µg of ATZ antibody or isotype control (human IgG1) was intraperitoneally administered at the same time, and tumor diameters were measured (Figs. 2-13-1, 13-2, 13-3, and 13-4).

### 14) Comparison of blood and tumor antibody concentrations following intraperitoneal ATZ administration and intratumoral T-ATZ administration in Neuro2a/A/J unilateral subcutaneous tumor model

Neuro2a cells (3 x 10⁶ cells/70 µl) were unilaterally and subcutaneously injected into A/J mice. On the day the maximum tumor diameter reached approximately 5 mm (day 0), T-ATZ or mock was intratumorally administered at a dose of 4 x 10⁴ pfu (L), 2 x 10⁵ pfu (M), or 1 x 10⁶ pfu (H)/20 µl) (n=3 per group), or 10, 50, or 200 µg of anti-PD-L1 antibody, ATZ, or isotype control (human IgG1) was intraperitoneally administered, and blood and tumors were collected 1, 4, and 7 days later. Serum and tumor proteins were prepared, and antibody concentrations in blood and tumors were measured by ELISA. For ELISA, mouse PD-L1 was immobilized, and HRP-labeled anti-human Fc antibody was used as the secondary antibody. ATZ was used as the standard.

Blood concentration: Intraperitoneal administration of the antibodies resulted in significant blood concentrations of antibodies, which decreased over time. In the 200 µg ATZ administration group, the concentration exceeded the detection range even with 1/1000 dilution, and so the result was indicated as ND. In contrast, no antibody was detected following the intratumoral administration of the T-ATZ virus (Fig. 2-14, top).

Intraperitoneal administration: Following the intraperitoneal antibody administration, antibodies were detected also in the tumors in a dose-dependent manner, and decreased over time. Following the intratumoral administration of T-ATZ virus, antibodies were detectable in the tumors, albeit at lower levels (Fig. 2-14, bottom).

### C) Production of viruses with anti-mouse CTLA4 antibody expression (T-9H10 and 4F10)

### 1) Virus with anti-mouse CTLA4 antibody expression (T-9H10)

For the anti-mouse CTLA4 antibody, the antibody-coding region was cloned from hybridoma 9H10.11G3, and sequenced, and an amino acid sequence for expressing the anti-mouse CTLA4 antibody (4F10) was designed (Fig. 3-1-1). On the basis of this amino acid sequence, a cDNA sequence was designed, and artificial gene synthesis of the sequence was ordered.

### 1-2) Virus with anti-mouse CTLA4 antibody expression (T-4F10)

For the anti-mouse CTLA4 antibody, the antibody-coding region was cloned from hybridoma UC10-4F10-11, and sequenced, and an amino acid sequence for expressing the anti-mouse CTLA4 antibody (4F10) was designed (Fig. 3-1-2). On the basis of this amino acid sequence, a cDNA sequence was designed, and artificial gene synthesis of the sequence was ordered.

### 1-3) Virus with anti-mouse CTLA4 antibody expression (T-4F10HA)

For the anti-mouse CTLA4 antibody, the antibody-coding region was cloned from hybridoma UC10-4F10-11, and sequenced, and an amino acid sequence for expressing the anti-mouse CTLA4 antibody (4F10) was designed (Fig. 3-1-3). On the basis of this amino acid sequence, a cDNA sequence was designed and artificial gene synthesis of the sequence was ordered. For this synthesis, the constant regions of the H chain and L chain derived from Armenian Ham IgG were used, and the antibody was designated as 4F10HA.

### 2) Confirmation of antibody expression

T-9H10, T-4F10, and T-01 were infected into HEK293T cells at MOI=0.1. After 48 hours, the supernatants were collected, concentrated by ultrafiltration (Amicon Ultra 30K), and subjected to ELISA for human Fc (human Fc antibody was immobilized, and detection was performed with HRP-human Fc antibody) (Fig. 3-2-1, A).

T-4F10HA was infected into HEK293T cells at MOI=1. After 48 hours, the culture supernatant of the cells was concentrated and used as a sample. Mouse CTLA-4 was immobilized, and detection was performed with an HRP-labeled anti-Armenian hamster IgG antibody as the secondary antibody. A commercially available anti-CTLA-4 antibody, 4F10, was used as the standard (Fig. 3-2-1, B).

### 2-2) Confirmation of anti-mouse CTLA4 antibody expression

Expression of the anti-mouse CTLA-4 antibody encoded by T-4F10HA was confirmed in mouse cell lines. Cells of the mouse neuroblastoma cell line Neuro2a, mouse melanoma cell line Clone M3, mouse renal carcinoma cell line Renca, and the control cell line, African green monkey kidney epithelial cell line Vero, were infected with the virus at MOI=2. After 18 hours, the culture supernatant was collected, and concentrated by ultrafiltration, and the expression of the encoded anti-CTLA-4 antibody was confirmed by ELISA (Fig. 3-2-2).

### 3) Confirmation of function of anti-mouse PD-1 antibody

To confirm whether the anti-mouse CTLA-4 antibody encoded by T-4F10HA has the CTLA-4/CD80 binding inhibitory activity as expected, an AlphaLISA assay system (system for measuring binding of mouse CTLA-4 and mouse CD80) was established by using biotinylated mouse CTLA-4 and His-tagged mouse CD80. T-4F10HA was infected into HEK293T cells (MOI = 1). After 48 hours, the culture supernatant was collected, concentrated by ultrafiltration (Amicon Ultra 30K), and used as a sample. A commercially available 4F10 antibody was used as a positive control (Fig. 3-3).

### 4) Cytotoxic effect (cytotoxicity assay)

Neuro2a cells (2 x 10⁵ cells/well) and Clone M3 cells (1.6 x 10⁵ cells/well) were seeded on 6-well plates. The cells were infected with T-01, T-4F10 or T-9H10 at MOI=0.1 or 1, and cultured at 34.5°C for 1 to 4 days (Fig. 3-4).

### 5) Replication assay

Cells of Neuro2a, Vero (3 x 10⁵ cells/well) and Clone M3 (4 x 10⁵ cells/well) were seeded on 6-well plates, infected with T-01, T-4F10 or T-9H10 at MOI=0.3 for Nuero2a, MOI=0.02 for Vero, and MOI=0.1 for Clone M3, and cultured at 37°C. After 24 and 48 hours, cells and culture media were collected, subjected to three times of freeze-thaw cycle, and ultrasonicated for 1 minute, and then titration was performed (Fig. 3-5).

### 6) Infection efficiency (X-gal staining)

Vero and Neuro2a cells (3 x 10⁵ cells/well), as well as Clone M3 cells (5 x 10⁵ cells/well) were seeded on 6-well plates. The cells were infected with T-01, or T-4F10 or T-9H10 at MOI=1 or 3, and cultured at 37°C for 6 hours. Then, X-gal staining was performed (Fig. 3-6-1).

### 6-2) Infection efficiency (X-gal staining)

Vero and Neuro2a cells (3 x 10⁵ cells/well) were seeded on 6-well plates, then infected with T-01 or T-4F10HA at MOI of 1 or 3, and cultured at 37°C for 6 hours. Subsequently, X-gal staining was performed (Fig. 3-6-2).

### 7) Confirmation of DNA structure

The prepared viruses with anti-mouse CTLA-4 antibody expression (T-9H10 and T-4F 10) were treated with the restriction enzyme HindIII. Southern blotting was performed by using the insert sequences as a probe. The DNA structure confirmation for T-4F10HA is shown in Fig. 1-7-2.

### 8) Comparison of antitumor effects in Neuro2a/A/J mouse unilateral subcutaneous tumor model

Antitumor effects were compared in a unilateral subcutaneous tumor model by using the following method.
Subcutaneously inject Neuro2a cells (5 x 10⁶ cells/50 µl) into A/J mice.
↓ 5 days
Intratumorally administer virus twice on the day the maximum tumor diameter reached approximately 5 mm (day 0) and 3 days later (day 3) (n=7 to 9 per group).

| |
|---|
| T-01, T-9H10 4 x 10⁴ pfu/20 µl |
| Mock |
| ↓ |
| Measure tumor diameter. |

Only T-9H10 showed a significant difference compared with the mock (Fig. 3-8).

### 9) Comparison of antitumor effects in Neuro2a/A/J mouse bilateral subcutaneous tumor model

Antitumor effects were compared in a bilateral subcutaneous tumor model by using the following method.
Bilaterally and subcutaneously inject Neuro2a cells (5 x 10⁶ cells/50 µl) into A/J mice.
↓ 4 days

Administer virus twice into the left ventral tumor twice on the day the maximum tumor diameter reached approximately 5 mm (day 0) and 3 days later (day 3) (n=8 per group).

| |
|---|
| T-01, T-9H10 2 x 10⁵ pfu/20 µl |
| Mock |
| ↓ |
| Measure tumor diameter. |

Only T-9H10 showed a significant difference compared with the mock (Fig. 3-9).

### 10) Comparison of antitumor effects in Clone M3/DBA/2 mouse unilateral subcutaneous tumor model

Antitumor effects were compared in a unilateral subcutaneous tumor model by using the following method.
Bilaterally and subcutaneously inject Clone M3 cells (1 x 10⁶ cells/50 µl) into DBA/2 mice.
↓ 8 Days

Administer virus into the left ventral tumor twice on the day the maximum tumor diameter reached approximately 5 mm (day 0) and 3 days later (day 3) (n=7 or 8 per group).

| |
|---|
| T-01, T-9H10 2 x 10⁵ pfu/20 µl |
| Mock |
| ↓ |
| Measure tumor diameter. |

Only T-9H10 showed a significant difference compared with the mock (Fig. 3-10).

### 11) Comparison of antitumor effects in Clone M3/DBA/2 mouse bilateral subcutaneous tumor models

Antitumor effects were compared in a bilateral subcutaneous tumor model by using the following method.
Bilaterally and subcutaneously inject Clone M3 cells (1 x 10⁶ cells/50 µl) into DBA/2 mice.
↓ 8 Days

Administer virus into the left ventral tumor twice on the day the maximum tumor diameter reached approximately 5 mm (day 0) and 3 days later (day 3) (n=7 to 9 per group).

| |
|---|
| T-01, T-9H10 2 x 10⁵ pfu/20 µl |
| Mock |
| ↓ |
| Measure tumor diameter. |

T-9H10 showed a significant difference compared with T-01 (Fig. 3-11).

### 12) Comparison of antitumor effects in Renca/Balb/c mouse unilateral subcutaneous tumor model (anti-CTLA-4 antibody)

The results are shown in Fig. 3-12s.

### 13) Comparison of antitumor effects of combination of T-01 and 4F10 antibody and. T-4F10HA alone in Neuro2a/A/J mouse bilateral subcutaneous tumor model

Neuro2a cells (3 x 10⁶ cells/70 µl) were bilaterally and subcutaneously injected into A/J mice. T-01, T-4F10HA, or mock was intratumorally administered twice on the day the maximum tumor diameter reached approximately 5 mm (day 0) and 3 days later (day 3) at a dose of 2 x 10⁵ pfu/20 µl (n=10 per group), 1 µg of 4F10 antibody or isotype control (Armenian hamster IgG, 2A3) was intraperitoneally administered at the same time, and tumor diameters were measured (Figs. 3-12-1, 12-2, 12-3, and 12-4).

### 14) Comparison of blood and tumor antibody concentrations following intraperitoneal administration of 4F10 and intratumoral administration of T-4F10HA in Neuro2a/A/J mouse unilateral subcutaneous tumor model

Neuro2a cells (3 x 10⁶ cells/70 µl) were unilaterally and subcutaneously injected into A/J mice. On the day the maximum tumor diameter reached approximately 5 mm (day 0), T-4F10HA or mock was intratumorally administered at a dose of 4 x 10⁴ pfu (L), 2 x 10⁵ pfu (M), 1 x 10⁶ pfu (H), or 5 x 10⁶ pfu (XH)/20 µl (n=3 per group), or 1, 5, or 25 µg of anti-CTLA4 antibody, 4F10, or isotype control (Armenian hamster IgG, 2A3) was intraperitoneally administered, and blood samples and tumors were collected 1, 4, and 7 days later. Serum and tumor proteins were prepared, and antibody concentrations in blood and tumors were measured by ELISA. For ELISA, mouse CTLA4 was immobilized, and HRP-labeled anti-Armenian hamster IgG antibody was used as the secondary antibody. 4F10 was used as the standard.

Blood concentration: Intraperitoneal administration of the antibodies resulted in quite high concentrations of antibodies in blood, which decreased over time. Intratumoral administration of T-4F10 virus did not yield detectable levels. Increasing the virus dose led to detection at significantly lower concentrations, which remained considerably lower than those observed with intraperitoneal administration of 1 µg (Fig. 3-13, top).

### Intratumoral concentration (concentration in 0.1 mg tumor proteins, measured for 100 µl of 1 mg/ml preparation)

With the intraperitoneal administration of the antibodies, antibodies were also detected in the tumor in a dose-dependent manner, and the concentration decreased over time. With the intratumoral administration of T-4F10HA virus, the concentration was below the detection limit. Only when measured after maximizing the amount of tumor proteins for the XH (5 x 10⁶ pfu) administration group, detection was possible (calculated as concentration per tumor, Fig. 3-13, bottom).

### I) Effects of combination with anti-cancer HSV-1 with ICI expression

### I-1) Effects of combination (mix) of HSV-1 with anti-CTLA-4 antibody expression (T-9H10) and HSV-1 with anti-PD-1 antibody expression (T-J43) in Neuro2a/A/J mouse bilateral subcutaneous tumor model

Neuro2a cells (3 x 10⁶ cells/70 µl) were bilaterally and subcutaneously injected into A/J mice. T-9H10, T-J43, or mock was intratumorally administered twice on the day the maximum tumor diameter reached approximately 5 mm (day 0) and 3 days later (day 3) at a dose of 2 x 10⁵ pfu/20 µl (n=13 per group), and the tumor diameters were measured. The mix was prepared by mixing equal volumes of T-9H10 and T-J43 prepared at a concentration of 2 x 10⁵ pfu/20 µl and administered in a volume of 20 µl (Figs. 4-1-1 and 4-1-2).

### I-2) Effects of combination (mix) of HSV-1 with anti-CTLA-4 antibody expression (T-9H10) and HSV-1 with anti-PD-L1 antibody expression (T-ATZ) in Neuro2a/A/J mouse bilateral subcutaneous tumor model

Neuro2a cells (3 x 10⁶ cells/70 µl) were bilaterally and subcutaneously injected into A/J mice. T-9H10, T-ATZ, or mock was intratumorally administered twice on the day the maximum tumor diameter reached approximately 5 mm (day 0) and 3 days later (day 3) at a dose of 2 x 10⁵ pfu/20 µl (n=10 per group), and the tumor diameters were measured. The mix was prepared by mixing equal volumes of T-9H10 and T-ATZ prepared at a concentration of 2 x 10⁵ pfu/20 µl and administered in a volume of 20 µl (Figs. 4-1 and 4-1-2).

### I-3) Effects of combination (mix) of HSV-1 with anti-CTLA-4 antibody (T-9H10) expression and HSV-1 with anti-PD-1 antibody (T-J43) expression in Clone M3/DBA/2 mouse bilateral subcutaneous tumor model

Clone M3 cells (1 x 10⁶ cells/50 µl) were bilaterally and subcutaneously injected into DBA/2 mice. T-9H10, T-J43, or mock was intratumorally administered twice on the day the maximum tumor diameter reached approximately 5 mm (day 0) and 3 days later (day 3) at a dose of 2 x 10⁵ pfu/20 µl (n=8 per group), and tumor diameters were measured. The mix was prepared by mixing equal volumes of T-9H10 and T-J43 prepared at a concentration of 2 x 10⁵ pfu/20 µl and administered in a volume of 20 µl (Fig. 4-3).

### I-4) Effects of combination (mix) of HSV-1 with anti-CTLA-4 antibody expression (T-9H10) and HSV-1 with anti-PD-L1 antibody (T-ATZ) expression in Clone M3/DBA/2 mice bilateral subcutaneous tumor model

Clone M3 cells (1 x 10⁶ cells/50 µl) were bilaterally and subcutaneously injected into DBA/2 mice. T-9H10, T-ATZ, or mock was intratumorally administered twice on the day the maximum tumor diameter reached approximately 5 mm (day 0) and 3 days later (day 3) at a dose of 2 x 10⁵ pfu/20 µl (n=8 per group), and tumor diameters were measured. The mix was prepared by mixing equal volumes of T-9H10 and T-ATZ prepared at a concentration of 2 x 10⁵ pfu/20 µl and administered in a volume of 20 µl (Fig. 4-4).

### II) Comparison of effects of concurrent and sequential administrations of anticancer HSV-1 with ICI expression

### II-1) Therapeutic efficacies of administration of two-ingredient cocktail (mix) of HSV-1 with anti-CTLA-4 antibody expression (T-4F10HA) and HSV-1 with anti-PD-1 antibody expression (T-J43) and sequential administration of them in Neuro2a/A/J mouse bilateral subcutaneous tumor model

Neuro2a cells (3 x 10⁶ cells/70 µl) were bilaterally and subcutaneously injected into A/J mice. T-4F10HA, T-J43, or mock was intratumorally administered twice on the day the maximum tumor diameter reached approximately 5 mm (day 0) and 3 days later (day 3) at a dose of 2 x 10⁵ pfu/20 µl (n=10 per group), and tumor diameters were measured. As the combination (mix), a mixture of equal volumes of T-4F10HA and T-J43 prepared at a concentration of 2 x 10⁵ pfu/20 µl was administered in a volume of 20 µl. For the sequential administration, T-4F10HA was administered on day 0 and T-J43 on day 3, or vice versa (Figs. 5-1-1 and 5-1-2).

### II-2) Therapeutic efficacies of administration of two-ingredient cocktail (mix) of HSV-1 with anti-CTLA-4 antibody expression (T-4F10HA) and HSV-1 with anti-PD-L1 antibody expression (T-ATZ) and sequential administration of them in Neuro2a/A/J mouse bilateral subcutaneous tumor model

Neuro2a cells (3 x 10⁶ cells/70 µl) were bilaterally and subcutaneously injected into A/J mice. T-4F10HA, T-ATZ, or mock was intratumorally administered twice on the day the maximum tumor diameter reached approximately 5 mm (day 0) and 3 days later (day 3) at a dose of 2 x 10⁵ pfu/20 µl (n=10 per group), and tumor diameters were measured. For the concurrent administration (mix), equal volumes of T-4F10HA and T-ATZ prepared at a concentration of 2 x 10⁵ pfu/20 µl were mixed, and the mixture was administered in a volume of 20 µl. For the sequential administration, T-4F10HA was administered on day 0 and T-ATZ on day 3, or vice versa (Figs. 5-2 and 5-2-2).

### II-3) Therapeutic efficacies of administration of two-ingredient cocktail (mix) of HSV-1 with anti-CTLA-4 antibody expression (T-4F10HA) and HSV-1 with anti-PD-1 antibody expression (T-J43) and sequential administration of them in Clone M3/DBA/2 mouse bilateral subcutaneous tumor model

Clone M3 cells (1 x 10⁶ cells/50 µl) were bilaterally and subcutaneously injected into DBA/2 mice. T-4F10HA, T-J43, or mock was intratumorally administered twice on the day the maximum tumor diameter reached approximately 5 mm (day 0) and 3 days later (day 3) at a dose of 2 x 10⁵ pfu/20 µl (n=7 per group), and tumor diameters were measured. For the concurrent administration (mix), equal volumes of T-4F 10HA and T-J43 prepared at a concentration of 2 x 10⁵ pfu/20 µl were mixed, and the mixture was administered in a volume of 20 µl. For the sequential administration, T-4F10HA was administered on day 0 and T-J43 on day 3, or vice versa (Figs. 5-3-1 and 5-3-2).

### II-4) Therapeutic efficacies of administration of two-ingredient cocktail (mix) of HSV-1 with anti-CTLA-4 antibody expression (T-4F10HA) and HSV-1 with anti-PD-L1 antibody expression (T-ATZ) and sequential administration of them in Clone M3/DBA/2 mouse bilateral subcutaneous tumor model

Clone M3 cells (1 x 10⁶ cells/50 µl) were bilaterally and subcutaneously injected into DBA/2 mice. T-4F10HA, T-ATZ, or mock was intratumorally administered twice on the day the maximum tumor diameter reached approximately 5 mm (day 0) and 3 days later (day 3) at a dose of 2 x 10⁵ pfu/20 µl (n=6 to 9 per group), and tumor diameters were measured. For the concurrent administration (mix), equal volumes of T-4F10HA and T-ATZ prepared at a concentration of 2 x 10⁵ pfu/20 µl were mixed, and the mixture was administered in a volume of 20 µl. For the sequential administration, T-4F10HA was administered on day 0 and T-ATZ on day 3, or vice versa (Figs.5-4-1 and 5-4-2).

### D) Preparation of virus with anti-human PD-1 antibody expression (T-Niv)

### 1) Amino acid sequence of anti-human PD-1 antibody

As the anti-human PD-1 antibody, an amino acid sequence for expressing the anti-human PD-1 antibody (T-Niv) was designed on the basis of the sequence information for nivolumab (KEGG entry# D10316, OPDIVO (Ono)) (Fig. 6-1-1). On the basis of this amino acid sequence, a cDNA sequence was designed and artificial gene synthesis of the sequence was ordered.

### 2) Confirmation of anti-human PD-1 antibody expression

T-Niv and T-01 were infected into HEK293T cells at MOI=0.1. After 48 hours, the supernatants were collected, concentrated by ultrafiltration (Amicon Ultra 30K), and then subjected to ELISA for human Fc (human Fc antibody was immobilized, and detection was performed with HRP-human Fc antibody) (Fig. 6-2-1, A) and PD-1-binding ELISA (human PD-1 was immobilized, and detection was performed with HRP-human Fc antibody) (Fig. 6-2-1, B).

### 2-2) Confirmation of anti-human PD-1 antibody expression

The expression of the anti-human PD-1 antibody encoded by T-Niv was confirmed in human cell lines. Cells of the human glioma cell line U87MG, human melanoma cell line Mewo, human renal carcinoma cell line OSRC2, and the control cell line, African green monkey kidney epithelial cell line Vero, were infected at MOI=2. After 18 hours, the culture supernatants were collected, and concentrated by ultrafiltration, and expression of the encoded anti-PD-L1 antibody was confirmed by ELISA (Fig. 6-2-2).

### 3) Confirmation of function of anti-human PD-1 antibody

To confirm whether the anti-human PD-1 antibody encoded by T-Niv has the PD-1/PD-L1 binding inhibitory activity as expected, an assay was performed by using AlphaLISA Human PD-1 and PD-L1 Binding Kit (PerkinElmer). First, Niv/SV-01 plasmid was transiently expressed in HEK293T cells, and the culture supernatant of the cells was concentrated and used as a sample. Further, a sample obtained by using a similar treatment with SV-01 plasmid was used as a negative control.

The results of the assay for PD-1/PD-L1-binding inhibitory activity performed by using the culture supernatants of Niv/SV-01 and SV-01 (empty vector)-transfected cells are shown in Fig. 6-3-1, A. The IC₅₀ value of Niv/SV-01 used in this experiment calculated in terms of the concentration of human Fc was 23.64 ng/ml (Fig. 6-3-1, B).

### 3-2) Confirmation of function of anti-human PD-1 antibody

To confirm whether the anti-human PD-1 antibody encoded by T-Niv has the PD-1/PD-L1 binding inhibitory activity as expected, an assay was performed by using AlphaLISA Human PD-1 and PD-L1 Binding Kit (PerkinElmer). T-Niv was infected into HEK293T cells (MOI=1). After 48 hours, the culture supernatant was collected and concentrated by ultrafiltration (Amicon Ultra 30K) to prepare a sample. Commercially available nivolumab was used as a positive control (Fig. 6-3-2).

### 4) Cytotoxic effect (cytotoxicity assay)

Vero cells (2 x 10⁵ cells/well) were seeded on a 6-well plate and infected with T-01 or T-Niv at MOI=0.01 or 0.1. The cells were cultured at 34.5°C for 1 to 4 days (Fig. 6-4).

### 5) Replication assay

Vero cells (3 x 10⁵ cells/well) were seeded on a 6-well plate and infected with T-01 or T-Niv at MOI of 0.01, and cultured at 37°C. After 24 and 48 hours, the cells and culture media were collected, subjected to three times of freeze-thaw cycle, and ultrasonicated for 1 minute, and then titration was performed (Fig. 6-5).

### 6) Infection efficiency (X-gal staining)

Vero cells were seeded on a 6-well plate, infected with T-01 or T-Niv at MOI=1 or MOI=3, and cultured at 37°C for 6 hours. Then, X-gal staining was performed (Fig. 6-6).

### 7) Confirmation of DNA structure

The prepared virus with anti-human PD-1 antibody expression (T-Niv) was treated with the restriction enzyme HindIII. Southern blotting was performed by using the LacZ sequence and the insert sequence as probes (Fig. 6-7).

### E) Production of virus with anti-human CTLA4 antibody expression

### 1) Amino acid sequence of anti-human CTLA4 antibody (T-Ipi)

As the anti-human CTLA4 antibody, an amino acid sequence for expressing anti-human CTLA4 antibody (Ipi) was designed on the basis of the sequence information of ipilimumab (KEGG entry#D04603, Bristol Myers Squibb, YERVOY) (Fig. 7-1-1). On the basis of this amino acid sequence, a cDNA sequence was designed and artificial gene synthesis of the sequence was ordered.

### 1-2) Amino acid sequence of anti-human CTLA4 antibody (T-Tic)

As the anti-human CTLA4 antibody, an amino acid sequence for expressing anti-human CTLA4 antibody (Tic) was designed on the basis of the sequence information for ticilimumab (tremelimumab) (Japanese Patent Publication No. 2012-167120) (Fig. 7-1-2). On the basis of this amino acid sequence, a cDNA sequence was designed and artificial gene synthesis of the sequence was ordered.

### 2) Confirmation of anti-human CTLA-4 antibody expression

Ipi/SV-01 and Tic/SV-01 plasmids were transiently expressed in HEK293T cells, and the culture supernatants of the cells were concentrated and used as samples (Fig. 7-2-1, A). Further, a sample obtained by a similar treatment carried out with the SV-01 plasmid was used as a negative control. ELISA for human Fc (human Fc antibody was immobilized, and detection was performed with HRP-human Fc antibody) was performed.

T-Ipi was infected into HEK293T cells at MOI=1. After 48 hours, the supernatant was collected, concentrated by ultrafiltration (Amicon Ultra 30K), and then subjected to CTLA-4 binding ELISA (human CTLA-4 was immobilized, and detection was performed with HRP-human Fc antibody using ipilimumab as the standard) (Fig. 7-2-1, B).

T-Tic was infected into HEK293T cells at MOI=1. After 48 hours, the supernatant was collected, concentrated by ultrafiltration (Amicon Ultra 30K), and then subjected to CTLA-4 binding ELISA (human CTLA-4 was immobilized, and detection was performed with HRP-human Fc antibody using ticilimumab as the standard) (Fig. 7-2-1, C).

### 2-2) Confirmation of anti-human CTLA-4 antibody expression

Expression of the anti-human CTLA-4 antibodies encoded by T-Ipi and T-Tic was confirmed in human cell lines. Cells of the human glioma cell line U87MG, human melanoma cell line Mewo, human renal carcinoma cell line OSRC2, and the control cell line, African green monkey kidney epithelial cell line Vero, were infected at MOI=2. After 18 hours, the culture supernatants were collected, and concentrated by ultrafiltration, and expression of the encoded anti-CTLA-4 antibodies was confirmed by ELISA (Fig. 7-2-2).

### 3) Confirmation of function of anti-human CTLA-4 antibody

To confirm whether the anti-human CTLA-4 antibody encoded by T-Ipi has the CTLA-4/CD80 (B7-1) binding inhibitory activity as expected, an assay was performed by using AlphaLISA Human CTLA-4/CD80 Binding Kit (PerkinElmer). First, Ipi/SV-01 plasmid was transiently expressed in HEK293T cells, and the culture supernatant of the cells was concentrated and used as a sample. Further, a sample obtained by a similar procedure performed with SV-01 plasmid was used as a negative control (Fig. 7-3-1).

### 3-2) Confirmation of function of anti-human CTLA4 antibody

To confirm whether the anti-human CTLA4 antibody encoded by T-Tic has the CTLA-4/CD80 (B7-1) binding inhibitory activity as expected, an assay was performed by using AlphaLISA Human CTLA-4/CD80 Binding Kit (PerkinElmer). First, Tic/SV-01 plasmid was transiently expressed in HEK293T cells, and the culture supernatant of the cells was concentrated and used as a sample. Further, a sample obtained by a similar procedure performed with SV-01 plasmid was used as a negative control (Fig. 7-3-2).

### 3-3) Confirmation of function of anti-human CTLA-4 antibody

To confirm whether the anti-human CTLA-4 antibodies encoded by T-Ipi and T-Tic have the CTLA-4/CD80 binding inhibitory activity as expected, an assay was performed by using AlphaLISA Human CTLA-4/CD80 Binding Kit (PerkinElmer). T-Ipi and T-Tic were infected into HEK293T cells (MOI=1). After 48 hours, the culture supernatants were collected and concentrated by ultrafiltration (Amicon Ultra 30K) to prepare samples. Commercially available ipilimumab and ticilimumab were used as positive controls.

### 4) Cytotoxic effect (cytotoxicity assay)

Vero cells were seeded on a 6-well plate (2 x 10⁵ cells/well), infected with T-01, T-Ipi, or T-Tic at MOI=0.01 or 0.1, and cultured at 34.5°C for 1 to 4 days (Fig. 7-4).

### 5) Replication assay

Vero cells were seeded on a 6-well plate (3 x 10⁵ cells/well), infected with T-01, T-Ipi or T-Tic at MOI=0.01, and cultured at 37°C. After 24 and 48 hours, the cells and culture media were collected, subjected to three times of freeze-thaw cycle, and ultrasonicated for 1 minute, and then titration was performed (Fig. 7-5).

### 6) Infection efficiency (X-gal staining)

Vero cells were seeded on a 6-well plate, infected with T-01, T-Ipi, or T-Tic at MOI=1 or 3, and cultured at 37°C for 6 hours. Then, X-gal staining was performed (Fig. 7-6).

### 7) Confirmation of DNA structure

The prepared viruses with anti-human CTLA4 antibody expression (T-Ipi and T-Tic) were treated with the restriction enzyme HindIII. Southern blotting was performed by using the LacZ sequence and the insert sequences as probes (Fig. 7-7).

### III) Effects of concurrent and sequential administration of virus with immune checkpoint inhibitor antibody expression and virus with immunostimulatory factor expression

### A) Neuro2a/A/J mouse bilateral subcutaneous tumor model

### 1) Virus with anti-CTLA4 antibody expression (T-4F10HA) and virus with soluble B7-1 expression

Neuro2a cells (3 x 10⁶ cells/70 µl) were bilaterally and subcutaneously injected into A/J mice. Therapeutic viruses or mock was intratumorally administered twice on the day the maximum tumor diameter reached approximately 5 mm (day 0) and 3 days later (day 3) at a dose of 2 x 10⁵ pfu/20 µl (n=11 or 12 per group), and tumor diameters were measured. For the concurrent administration (mix), equal volumes of T-4F10HA and T-mB7.1 (oncolytic HSV-1 that expresses soluble mouse B7-1; Fukuhara, H. et al., Cancer Res. 65, 10 663-10668 (2005)) prepared at a concentration of 2 x 10⁵ pfu/20 µl were mixed, and the mixture was administered in a volume of 20 µl. For the sequential administration, T-4F10HA was administered on day 0 and T-mB7.1 on day 3, or vice versa (Fig. 11-A-1).

### 2) Virus with anti-CTLA4 antibody expression (T-4F10HA) and virus with IL12 expression

Neuro2a cells (2.5 x 10⁶ cells/70 µl) were bilaterally and subcutaneously injected into A/J mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=11 or 12 per group), and tumor diameters were measured. For the concurrent administration (mix), equal volumes of T-4F10HA and T-mfIL12 (oncolytic HSV-1 that expresses mouse IL-12; Non-patent document 2, Fukuhara, H. et al., Commun Med (Lond) 3, 40

(2023)) prepared at a concentration of 2 x 10⁵ pfu/20 µl were mixed, and the mixture was administered in a volume of 20 µl. For the sequential administration, T-4F10HA was administered on day 0 and T-mflL12 on day 3, or vice versa (Fig. 11-A-2).

### 3) Virus with anti-PD-1 antibody expression (T-J43) and virus with soluble B7-1 expression

Neuro2a cells (3 x 10⁶ cells/70 µl) were bilaterally and subcutaneously injected into A/J mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=9 or 10 per group), and tumor diameters were measured. For the concurrent administration (mix), equal volumes of T-J43 and T-mB7.1 prepared at a concentration of 2 x 10⁵ pfu/20 µl were mixed, and the mixture was administered in a volume of 20 µl. For the sequential administration, T-J43 was administered on day 0 and T-mB7.1 on day 3, or vice versa (Fig. 11-A-3).

### 4) Virus with anti-PD-1 antibody expression (T-J43) and virus with IL-12 expression

Neuro2a cells (2.5 x 10⁶ cells/70 µl) were bilaterally and subcutaneously injected into A/J mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=11 or 12 per group), and tumor diameters were measured. For the concurrent administration (mix), equal volumes of T-J43 and T-mfIL12 prepared at a concentration of 2 x 10⁵ pfu/20 µl were mixed, and the mixture was administered in a volume of 20 µl. For the sequential administration, T-J43 was administered on day 0 and T-mflL12 on day 3, or vice versa (Fig. 11-A-4).

### 5) Virus with anti-PD-L1 antibody expression (T-ATZ) and virus with soluble B7-1 expression

Neuro2a cells (3 x 10⁶ cells/70 µl) were bilaterally and subcutaneously injected into A/J mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=9 or 10 per group), and tumor diameters were measured. For the concurrent administration (mix), equal volumes of T-ATZ and T-mB7.1 prepared at a concentration of 2 x 10⁵ pfu/20 µl were mixed, and the mixture was administered in a volume of 20 µl. For the sequential administration, T-ATZ was administered on day 0 and T-mB7.1 on day 3, or vice versa (Fig.11-A-5).

### 6) Virus with anti-PD-L1 antibody expression (T-ATZ) and virus with IL-12 expression

Neuro2a cells (3 x 10⁶ cells/70 µl) were bilaterally and subcutaneously injected into A/J mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=9 to 11 per group), and tumor diameters were measured. For the concurrent administration (mix), equal volumes of T-ATZ and T-mfIL12 prepared at a concentration of 2 x 10⁵ pfu/20 µl were mixed, and the mixture was administered in a volume of 20 µl. For the sequential administration, T-ATZ was administered on day 0 and T-mflL12 on day 3, or vice versa (Fig. 11-A-6).

### B) Clone M3/DBA/2 mouse bilateral subcutaneous tumor model

### 1) Virus with anti-CTLA4 antibody expression (T-4F10HA) and virus with soluble B7-1 expression

Clone M3 cells (1 x 10⁶ cells/50 µl) were bilaterally and subcutaneously injected into DBA/2 mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=9 per group), and tumor diameters were measured. For the concurrent administration (mix), equal volumes of T-4F10HA and T-mB7.1 prepared at a concentration of 2 x 10⁵ pfu/20 µl were mixed, and the mixture was administered in a volume of 20 µl. For the sequential administration, T-4F10HA was administered on day 0 and T-mB7.1 on day 3, or vice versa (Fig. 11-B-1).

### 2) Virus with anti-CTLA4 antibody expression (T-4F10HA) and virus with IL12 expression

Clone M3 cells (1 x 10⁶ cells/50 µl) were bilaterally and subcutaneously injected into DBA/2 mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=10 per group), and tumor diameters were measured. For the concurrent administration (mix), equal volumes of T-4F10HA and T-mfIL12 prepared at a concentration of 2 x 10⁵ pfu/20 µl were mixed, and the mixture was administered in a volume of 20 µl. For the sequential administration, T-4F10HA was administered on day 0 and T-mfIL12 on day 3, or vice versa (Fig. 11-B-2).

### 3) Virus with anti-PD-1 antibody expression (T-J43) and virus with soluble B7-1 expression

Clone M3 cells (1 x 10⁶ cells/50 µl) were bilaterally and subcutaneously injected into DBA/2 mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=9 per group), and tumor diameters were measured. For the concurrent administration (mix), equal volumes of T-J43 and T-mB7.1 prepared at a concentration of 2 x 10⁵ pfu/20 µl were mixed, and the mixture was administered in a volume of 20 µl. For the sequential administration, T-J43 was administered on day 0 and T-mB7.1 on day 3, or vice versa (Fig. 11-B-3).

### 4) Virus with anti-PD-1 antibody expression (T-J43) and virus with IL-12 expression

Clone M3 cells (1 x 10⁶ cells/50 µl) were bilaterally and subcutaneously injected into DBA/2 mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=10 per group), and tumor diameters were measured. For the concurrent administration (mix), equal volumes of T-J43 and T-mfIL12 prepared at a concentration of 2 x 10⁵ pfu/20 µl were mixed, and the mixture was administered in a volume of 20 µl. For the sequential administration, T-J43 was administered on day 0 and T-mflL12 on day 3, or vice versa (Fig. 11-B-4).

### 5) Virus with anti-PD-L1 antibody expression (T-ATZ) and virus with soluble B7-1 expression

Clone M3 cells (1 x 10⁶ cells/50 µl) were bilaterally and subcutaneously injected into DBA/2 mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=9 or 10 per group), and tumor diameters were measured. For the concurrent administration (mix), equal volumes of T-ATZ and T-mB7.1 prepared at a concentration of 2 x 10⁵ pfu/20 µl were mixed, and the mixture was administered in a volume of 20 µl. For the sequential administration, T-ATZ was administered on day 0 and T-mB7.1 on day 3, or vice versa (Fig. 11-B-5).

### 6) Virus with anti-PD-L1 antibody expression (T-ATZ) and virus with IL-12 expression

Clone M3 cells (1 x 10⁶ cells/50 µl) were bilaterally and subcutaneously injected into DBA/2 mice. The therapeutic viruses or mock was intratumorally administered twice on the day the maximum tumor diameter reached approximately 5 mm (day 0) and 3 days later (day 3) at a dose of 2 x 10⁵ pfu/20 µl (n=8 to 10 per group), and tumor diameters were measured. For the concurrent administration (mix), equal volumes of T-ATZ and T-mfIL12 prepared at a concentration of 2 x 10⁵ pfu/20 µl were mixed, and the mixture was administered in a volume of 20 µl. For the sequential administration, T-ATZ was administered on day 0 and T-mflL12 on day 3, or vice versa (Fig. 11-B-6).

### C) Renca/Balb/c mouse bilateral subcutaneous tumor model

### 1) Virus with anti-CTLA4 antibody expression (T-4F10HA) and virus with soluble B7-1 expression

Renca cells (1 x 10⁵ cells/50 µl) were bilaterally and subcutaneously injected into Balb/c mice. The therapeutic viruses or mock was intratumorally administered twice on the day the maximum tumor diameter reached approximately 5 mm (day 0) and 3 days later (day 3) at a dose of 1 x 10⁶ pfu/20 µl (n=9 or 10 per group), and tumor diameters were measured. For the concurrent administration (mix), equal volumes of T-4F10HA and T-mB7.1 prepared at a concentration of 1 x 10⁶ pfu/20 µl were mixed, and the mixture was administered in a volume of 20 µl. For the sequential administration, T-4F10HA was administered on day 0 and T-mB7.1 on day 3, or vice versa (Fig. 11-C-1).

### 2) Virus with anti-CTLA4 antibody expression (T-4F10HA) and virus with IL12 expression

Renca cells (1 x 10⁵ cells/50 µl) were bilaterally and subcutaneously injected into Balb/c mice. The therapeutic viruses or mock was intratumorally administered twice on the day the maximum tumor diameter reached approximately 5 mm (day 0) and 3 days later (day 3) at a dose of 1 x 10⁶ pfu/20 µl (n=10 per group), and tumor diameters were measured. For the concurrent administration (mix), equal volumes of T-4F10HA and T-mfIL12 prepared at a concentration of 1 x 10⁶ pfu/20 µl were mixed, and the mixture was administered in a volume of 20 µl. For the sequential administration, T-4F10HA was administered on day 0 and T-mfIL12 on day 3, or vice versa (Fig. 11-C-2).

### 3) Virus with anti-PD-1 antibody expression (T-J43) and virus with soluble B7-1 expression

Renca cells (1 x 10⁵ cells/50 µl) were bilaterally and subcutaneously injected into Balb/c mice. The therapeutic viruses or mock was intratumorally administered twice on the day the maximum tumor diameter reached approximately 5 mm (day 0) and 3 days later (day 3) at a dose of 1 x 10⁶ pfu/20 µl (n=10 per group), and tumor diameters were measured. For the concurrent administration (mix), equal volumes of T-J43 and T-mB7.1 prepared at a concentration of 1 x 10⁶ pfu/20 µl were mixed, and the mixture was administered in a volume of 20 µl. For the sequential administration, T-J43 was administered on day 0 and T-mB7.1 on day 3, or vice versa (Fig. 11-C-3).

### 4) Virus with anti-PD-1 antibody expression (T-J43) and virus with IL-12 expression

Renca cells (1 x 10⁵ cells/50 µl) were bilaterally and subcutaneously injected into Balb/c mice. The therapeutic viruses or mock was intratumorally administered twice on the day the maximum tumor diameter reached approximately 5 mm (day 0) and 3 days later (day 3) at a dose of 1 x 10⁶ pfu/20 µl (n=10 per group), and tumor diameters were measured. For the concurrent administration (mix), equal volumes of T-J43 and T-mfIL12 prepared at a concentration of 1 x 10⁶ pfu/20 µl were mixed, and the mixture was administered in a volume of 20 µl. For the sequential administration, T-J43 was administered on day 0 and T-mfIL12 on day 3, or vice versa (Fig. 11-C-4).

### 5) Virus with anti-PD-L1 antibody expression (T-ATZ) and virus with soluble B7-1 expression

Renca cells (1 x 10⁵ cells/50 µl) were bilaterally and subcutaneously injected into Balb/c mice. The therapeutic viruses or mock was intratumorally administered twice on the day the maximum tumor diameter reached approximately 5 mm (day 0) and 3 days later (day 3) at a dose of 1 x 10⁶ pfu/20 µl (n=9 or 10 per group), and tumor diameters were measured. For the concurrent administration (mix), equal volumes of T-ATZ and T-mB7.1 prepared at a concentration of 1 x 10⁶ pfu/20 µl were mixed, and the mixture was administered in a volume of 20 µl. For the sequential administration, T-ATZ was administered on day 0 and T-mB7.1 on day 3, or vice versa (Fig. 11-C-5).

### 6) Virus with anti-PD-L1 antibody expression (T-ATZ) and virus with IL-12 expression

Renca cells (1 x 10⁵ cells/50 µl) were bilaterally and subcutaneously injected into Balb/c mice. The therapeutic viruses or mock was intratumorally administered twice on the day the maximum tumor diameter reached approximately 5 mm (day 0) and 3 days later (day 3) at a dose of 1 x 10⁶ pfu/20 µl (n=9 or 10 per group), and tumor diameters were measured. For the concurrent administration (mix), equal volumes of T-ATZ and T-mfIL12 prepared at a concentration of 1 x 10⁶ pfu/20 µl were mixed, and the mixture was administered in a volume of 20 µl. For the sequential administration, T-ATZ was administered on day 0 and T-mfIL12 on day 3, or vice versa (Fig. 11-C-6).

### IV) Effect of administration of three-ingredient cocktail of virus with immune checkpoint inhibitor antibody expression and virus with immunostimulatory factor expression

### A) Neuro2a/A/J mouse bilateral subcutaneous tumor model

Neuro2a cells (2 x 10⁵ cells/70 µl) were bilaterally and subcutaneously injected into A/J mice. The therapeutic viruses, control virus thereof (non-coding T-01), or mock was intratumorally administered three times on the day the maximum tumor diameter reached approximately 5 mm (day 0), 3 days later (day 3), and 7 days later (day 7) at a dose of 8 x 10⁴ pfu/20 µl (n=9 per group), and tumor diameters were measured. For the cocktail administration, a mixture of equal volumes of three types of viruses each prepared at 8 x 10⁴ pfu/20 µl was administered in a volume of 20 µl (Fig. 12-A).

### B) Clone M3/DBA/2 mouse bilateral subcutaneous tumor model

Clone M3 cells (1 x 10⁶ cells/50 µl) were bilaterally and subcutaneously injected into DBA/2 mice. The therapeutic viruses, control virus thereof (non-coded T-01), or mock was intratumorally administered three times on the day the maximum tumor diameter reached approximately 5 mm (day 0), 4 days later (day 4), and 7 days later (day 7) at a dose of 2 x 10⁵ pfu/20 µl (n=8 to 10 per group), and tumor diameters were measured. For the cocktail administration, a mixture of equal volumes of three types of viruses each prepared at a concentration of 2 x 10⁵ pfu/20 µl was administered in a volume of 20 µl (Fig. 12-B).

### C) Renca/Balb/c mouse bilateral subcutaneous tumor model

Renca cells (1 x 10⁵ cells/50 µl) were bilaterally and subcutaneously injected into Balb/c mice. The therapeutic viruses, control virus thereof (non-coding T-01), or mock was intratumorally administered three times on the day the maximum tumor diameter reached approximately 5 mm (day 0), 4 days later (day 4), and 7 days later (day 7) at a dose of 1 x 10⁶ pfu/20 µl (n=9 per group), and tumor diameters were measured. For the cocktail administration, a mixture of equal volumes of three types of viruses each prepared at 1 x 10⁶ pfu/20 µl was administered in a volume of 20 µl (Fig. 12-C).

### [Sequences listed in Sequence Listing]

SEQ ID NO: 1 IgK Leader sequence
SEQ ID NO: 2 Signal peptidase recognition sequence
SEQ ID NO: 3 VH, anti-mouse PD-1 antibody
SEQ ID NO: 4 CH, human IgG4
SEQ ID NO: 5 Furin recognition sequence
SEQ ID NO: 6 FMDV-2A
SEQ ID NO: 7 VL, anti-mouse PD-1 antibody
SEQ ID NO: 8 CL, human λ
SEQ ID NO: 9 CH, Armenian Ham IgG
SEQ ID NO: 10 CL, Armenian Ham Ig κ
SEQ ID NO: 11 VH, derived from atezolizumab
SEQ ID NO: 12 CH, human IgG1 N298A
SEQ ID NO: 13 VL, derived from atezolizumab
SEQ ID NO: 14 CL, human κ
SEQ ID NO: 15 VH, anti-mouse CTLA4 antibody, derived from hybridoma 9H10.11G3
SEQ ID NO: 16 CH, human IgG1
SEQ ID NO: 17 VL, anti-mouse CTLA4 antibody, derived from hybridoma 9H10.11G3
SEQ ID NO: 18 VH, anti-mouse CTLA4 antibody, derived from hybridoma UC10-4F10-11
SEQ ID NO: 19 VL, anti-mouse CTLA4 antibody, derived from hybridoma UC10-4F10-11
SEQ ID NO: 20 VH, anti-mouse CTLA4 antibody, derived from hybridoma UC10-4F10-11
SEQ ID NO: 21 VL, anti-mouse CTLA4 antibody, derived from hybridoma UC10-4F10-11
SEQ ID NO: 22 VH, derived from nivolumab
SEQ ID NO: 23 CH, human IgG4 S224P
SEQ ID NO: 24 VL, derived from nivolumab
SEQ ID NO: 25 VH, derived from ipilimumab
SEQ ID NO: 26 VL, derived from ipilimumab
SEQ ID NO: 27 VH, derived from ticilimumab
SEQ ID NO: 28 CH, human IgG4 S224P
SEQ ID NO: 29 VL, derived from ticilimumab
SEQ ID NO: 30 Nucleotide sequence of insertion site of gene encoding immune checkpoint inhibitor and upstream and downstream thereof
SEQ ID NO: 31 Sequence containing γ34.5 gene deletion site (TR_{L} region)
SEQ ID NO: 32 Sequence containing α47 gene deletion site (TR_{L} region)

## Claims

1. An oncolytic herpes simplex virus having a gene encoding an immune checkpoint inhibitor.

2. The virus according to claim 1, wherein the herpes simplex virus has the following characteristics (a) to (c):
(a) the ICP6 gene is deleted or inactivated,
(b) the γ34.5 gene is deleted or inactivated, and
(c) the α47 gene is deleted or inactivated.

3. The virus according to claim 2, wherein the gene encoding an immune checkpoint inhibitor is inserted at a site where the ICP6 gene is deleted or inactivated.

4. The virus according to claim 1, wherein the immune checkpoint inhibitor is an immune checkpoint inhibitor antibody or a fragment thereof.

5. The virus according to claim 1, wherein the immune checkpoint inhibitor is an anti-PD-1 antibody, an anti-PD-L1 antibody, or an anti-CTLA-4 antibody, or a fragment thereof.

6. A pharmaceutical composition containing the virus according to any one of claims 1 to 5.

7. The pharmaceutical composition according to claim 6, which is for local injection.

8. The pharmaceutical composition according to claim 6 or 7, being a formulation designed to suppress blood concentration of the immune checkpoint inhibitor.

9. A combination of:
the virus according to any one of claims 1 to 5, and
at least one type of oncolytic herpes simplex virus having a gene encoding an immune checkpoint inhibitor different from that of the foregoing virus.

10. A combination of:
the virus according to any one of claims 1 to 5, and
at least one of an oncolytic herpes simplex virus having a gene encoding soluble B7-1 and an oncolytic herpes simplex virus having a gene encoding IL-12.
